# EUROPEAN PATENT APPLICATION

(11) **EP 4 442 827 A1**
(43) Date of publication of application: **09.10.2024**
(21) Application number: 22897977.9
(22) Date of filing: 29.11.2022
(51) Int. Cl.: C12N 15/113, A61K 31/713, A61P 1/16

(54) **COMPOSITION AND METHOD FOR INHIBITING EXPRESSION OF HEPATITIS B VIRUS (HBV) PROTEIN**

(30) Priority: 29.11.2021 WO PCT/CN2021/133837
(71) Applicant: Shanghai Argo Biopharmaceutical Co., Ltd., Shanghai 201803 (CN)
(72) Inventor: SHU, Dongxu, Ningbo, Zhejiang 315101 (CN); SHAO, Pengcheng Patrick, Warrington Township, Pennsylvania 18976 (US)
(74) Representative: Simmons & Simmons LLP (Munich)
(86) International application number: PCT/CN2022/134879
(87) International publication number: WO 2023/093896

(57) **Abstract**

Provided are a composition and a method that can be used for reducing the expression of one or more hepatitis B virus (HBV) genes and for treating HBV-related diseases and disorders. Provided are an HBV dsRNA agent that can be used for reducing the expression of HBV in a cell, and an object and a composition comprising same.

## Description

### Field of the Invention

The invention relates, in part, to compositions and methods that can be used to inhibit hepatitis B virus (HBV) protein expression.

### Background

It is estimated chronic hepatitis B infection (CHB) affects over 350 million people worldwide and is one of the leading causes of deaths from liver-related conditions and hepatocellular carcinoma (HCC) (www.cdc.gov/hepatitis/hbv/pdfs/hepbatrisk.pdf). Although hepatitis B virus (HBV) vaccine provides an effective way of preventing new cases of HBV infection, there exists a significant unmet medical need for therapeutics and methods for treating existing HBV infections and HBV-associated diseases and conditions.

The HBV virion contains a compact 3.2 kilobase (kb) genome that exists as a partially double-stranded, relaxed circular DNA (rcDNA) with a 7-9 base terminal redundancy that is converted into covalently closed circular DNA (cccDNA) within the nucleus of an hepatocyte, thereby functioning as a mini-chromosome for HBV transcription (Bock CT, et al., J Mol Biol. 2001; 307:183-196). Host RNA polymerase II transcribes HBV genes on the cccDNA to produce five viral RNAs, (1) transcripts that encode precore (HBeAg), (2) the pregenomic RNA (pgRNA) that encodes the structural capsid protein (core) and polymerase and is reverse transcribed to produce rcDNA, (3) the pre-S1 transcript that encodes the large S surface protein, (4) another transcript that encodes pre-S2 and S surface proteins (middle and small S), and (5) the X gene mRNA. The three surface proteins collectively comprise HBsAg. All HBV transcripts are encoded in overlapping reading frames, have a common 3' end and utilize the same polyadenylation signal (PAS). HBsAg, in a lipid bilayer, forms an envelope around each core-encapsidated, partially double-stranded HBV genome.

The natural evolution of chronic HBV infection includes four consecutive phases: (1) early 'immunotolerant' phase comprising high levels of virus replication and minimal liver inflammation; (2) immune reactive phase comprising significant hepatic inflammation and elevated serum aminotransferases; (3) some patients progress to 'non-replicative' phase comprising seroconversion to anti-HBeAg, undetectable or low level of viremia (below 2000 lU/ml by PCR-based assays), and resolution of hepatic inflammation; and (4) HBeAg-negative chronic hepatitis B, due to the emergence of specific viral mutations, which prevent the production of HBeAg but do not hamper virus replication. Phase four is a form of chronic hepatitis B (CHB) may present as HBeAg-positive or HBeAg negative CHB and is characterized by fluctuating serum HBV DNA and serum aminotransferases (ALT and AST) levels and progressive liver disease.

Although cccDNA is considered the driver of HBV transcription and replication, HBV DNA may integrate into the host genome. Levels of cccDNA are higher in HBeAg positive than HBeAg negative patients, whereas levels of integrated HBV are higher in HBeAg negative patients. In addition to cccDNA, studies have shown integrated HBV DNA could be a source for HBsAg (Wooddell et al., Sci Transl. Med. 2017 Sep 27;9(409):eaan0241. doi: 10.1126/scitranslmed.aan0241). HBeAg and HBsAg play important roles in chronic infection and are believed to cause T cell tolerance and exhaustion (Kakimi et el., J Virol. 2002; 76:8609-8620). HBsAg loss is considered a hallmark of effective immune control of HBV. For CHB patients, seroclearance of HBsAg is the desired treatment endpoint ("functional cure"), with improved long-term prognosis.

The rates of functional cure achievable with currently available therapies are relatively low. Nucleot(s)ide analogues (NUC) and pegylated interferon (PEG-IFN) are two currently available treatment options for CHB. The administration of NUCs can be effective in controlling viral replication and are associated with reduced adverse outcomes, however, they do not interfere with viral antigen production or secretion. It is rare to achieve "functional cure" of CHB with NUC therapy. PEG-IFN treatment may induce long-term immunological control, but has very low efficacy with respect to achieving a "functional cure". Lau et al. have shown that after 48 weeks of PEG-IFN monotherapy, only 3% of treated individuals achieved HBsAg seroclearance (Lau et al., N Engl J Med. 2005;352(26):2682-95.). Chronic hepatitis B and other HBV-associated diseases and conditions remain significant worldwide health problems and effective therapeutic approaches are needed.

### Summary of the Disclosure

According to an aspect of the invention, a double-stranded ribonucleic acid (dsRNA) agent for inhibiting expression of Hepatitis B virus (HBV) in a cell is provided, wherein the dsRNA agent comprises a sense strand and an antisense strand, nucleotide positions 2 to 18 in the antisense strand comprising a region of complementarity to an HBV RNA transcript, wherein the region of complementarity comprises at least 15 contiguous nucleotides that differ by 0, 1, 2, or 3 nucleotides from one of the antisense sequences listed in one of Tables 1-4, and optionally comprising a targeting ligand. In some embodiments, the region of complementarity to the HBV RNA transcript comprises at least 15, 16, 17, 18, or 19 contiguous nucleotides that differ by no more than 3 nucleotides from one of the antisense sequences listed in one of Tables 1-4. In certain embodiments, the antisense strand of dsRNA is at least substantially complementary to any one of a target region of SEQ ID NO: 673 and is provided in any one of Tables 1-4. In some embodiments, the antisense strand of dsRNA is fully complementary to any one of a target region of SEQ ID NO: 673 and is provided in any one of Tables 1-4. In some embodiments, the dsRNA agent comprises a sense strand sequence set forth in any one of Tables 1-4, wherein the sense strand sequence is at least substantially complementary to the antisense strand sequence in the dsRNA agent. In certain embodiments, the dsRNA agent comprises a sense strand sequence set forth in any one of Tables 1-4., wherein the sense strand sequence is fully complementary to the antisense strand sequence in the dsRNA agent. In some embodiments, the dsRNA agent comprises an antisense strand sequence set forth in any one of Tables 1-4. In some embodiments, the dsRNA agent comprises the sequences set forth as a duplex sequence in any of Tables 1-4. In some embodiments, the antisense strand of dsRNA consists of a nucleotide sequence II: 5'-z₁uugucaacaagaaaaaz₂-3', wherein z₁ is selected from c, g, a or u, z₂ is a nucleotide sequence IV. In certain embodiments, z₁ is u. In certain embodiments, the nucleotide sequence IV is 0-15 nucleotides in length. In certain embodiments, the nucleotide sequence IV is selected from c, cu, ca, cc, cg, ccu,cca, ccc, ccg, cccc, cccu, ccca, cccg, ccccg, ccccgc, ccccgcc, ccccgccu, ccccgccug, ccccgccuguaacac, cccguu or cccgga. In certain embodiments, the nucleotide sequence IV is 1, 2, 3 or 4 nucleotides in length. In certain embodiments, the nucleotide sequence IV is selected from c, cu, ca, cc, cg, ccu,cca, ccc, ccg, cccc, cccu, ccca or cccg. In some embodiments, the sense strand of dsRNA consists of a nucleotide sequence III: 5'-z₃ uuuuucuuguugacaaz₄-3', wherein z₃ is a nucleotide sequence V, z₄ is selected from c, g, a or u. In certain embodiments, z₄ is a. In certain embodiments, the nucleotide sequence V is 0-15 nucleotides in length. In certain embodiments, the nucleotide sequence V is selected from g, ag, ug, gg, cg, agg, ugg, ggg, egg, gggg, aggg, uggg, cggg, cgggg, gcgggg, ggcgggg, aggcgggg, caggcgggg or guguuacaggcgggg. In certain embodiments, the nucleotide sequence V is 1, 2, 3 or 4 nucleotides in length. In certain embodiments, the nucleotide sequence V is selected from g, ag, ug, gg, cg, agg, ugg, ggg, egg, gggg, aggg, uggg or cggg. In some embodiments, the antisense strand of dsRNA consists of a nucleotide sequence VI: 5'-z₅ugucaacaagaaaaacz₆-3', wherein z₅ is selected from c, g, a or u, z₆ is a nucleotide sequence VIII. In certain embodiments, z₅ is a. In certain embodiments, the nucleotide sequence VIII is 0-15 nucleotides in length. In certain embodiments, the nucleotide sequence VIII is selected from c, cc, ecu, cca, ccc, ccg, cccu, ccca, cccc, cccg, ccug, cccuc, cccuu, cccua, cccgc, cccgcc, ccuguu, ccugga, cccgccu, cccgccug, cccgccugu, cccgccuguaacacg. In certain embodiments, the nucleotide sequence VIII is 1, 2, 3 or 4 nucleotides in length. In certain embodiments, the nucleotide sequence VIII is selected from c, cc, ecu, cca, ccc, ccg, cccu, ccca, cccc, cccg or ccug. In some embodiments, the sense strand of dsRNA consists of a nucleotide sequence VII: 5'-z₇guuuuucuuguugacaz₈-3', wherein z₇ is a nucleotide sequence IX, z₈ is selected from c, g, a or u. In certain embodiments, z₈ is u. In certain embodiments, the nucleotide sequence IX is 0-15 nucleotides in length. In certain embodiments, the nucleotide sequence IX is selected from g, ag, ug, gg, cg, agg, ugg, ggg, egg, ggag, aagg, uagg, cagg, gcggg, ggcggg, aggcggg, caggcggg, acaggcggg or cguguuacaggcggg. In certain embodiments, the nucleotide sequence IX is 1, 2, 3 or 4 nucleotides in length. In certain embodiments, the nucleotide sequence IX is selected from g, ag, ug, gg, cg, agg, ugg, ggg, egg, ggag, aagg, uagg or cagg. In some embodiments, z₁ is a nucleotide complementary to z₄. In some embodiments, z₂ is a nucleotide sequence complementary to z₃. In some embodiments, z₅ is a nucleotide sequence complementary to z₈. In some embodiments, z₆ is a nucleotide sequence complementary to z₇. In some embodiments, the dsRNA agent includes a sense strand and an antisense strand, wherein the antisense strand of dsRNA consists of the nucleotide sequence II or VI as described above, wherein the sense strand is no more than 30 nucleotides in length comprising a region of complementarity to the antisense strand including at least 15, 16, 17, 18, or 19 nucleotides. In some embodiments, the dsRNA agent includes a sense strand and an antisense strand, wherein the sense strand of dsRNA consists of the nucleotide sequence III and the antisense strand of dsRNA consists of the nucleotide sequence II, wherein the nucleotide sequence II and III are as described above. In some embodiments, the dsRNA agent includes a sense strand and an antisense strand, wherein the sense strand of dsRNA consists of the nucleotide sequence VII and the antisense strand of dsRNA consists of the nucleotide sequence VI, wherein the nucleotide sequence VI and VII are as described above. In some embodiments, the nucleotide sequence II includes a 3' overhang of at least 1 nucleotide. In some embodiments, the nucleotide sequence II includes a 3' overhang of at least 2 nucleotide. In certain embodiments, said 3' overhang in the nucleotide sequence II is selected from uu or ga. In some embodiments, the nucleotide sequence VI includes a 3' overhang of at least 1 nucleotide. In some embodiments, the nucleotide sequence VI includes a 3' overhang of at least 2 nucleotide. In certain embodiments, said 3' overhang in the nucleotide sequence VI is selected from uu or ga.

In some embodiments, the dsRNAs include a sense strand and an antisense strand, the antisense strand comprising a region of complementarity which includes at least 15 contiguous nucleotides that differ by 0, 1, 2, or 3 nucleotides from any one of the nucleotide sequences selected from the group consisting of
5'-uuugucaacaagaaaaacccc -3' (SEQ ID NO: 143)
5'- uacaaaagaaaauugguaaca -3' (SEQ ID NO: 152)
5'- uugucaacaagaaaaaccccg -3' (SEQ ID NO: 162)
5'- ugaacaaauggcacuaguaaa -3' (SEQ ID NO: 166)

In certain embodiments, the sense and antisense strands comprise nucleotide sequences including at least 15 contiguous nucleotides that differ by 0, 1, 2, or 3 nucleotides from any one of the nucleotide sequences selected from the group consisting of
5'-gggguuuuucuuguugacaaa-3' (SEQ ID NO: 5)
5'-uuugucaacaagaaaaacccc-3' (SEQ ID NO: 143)
5'-uguuaccaauuuucuuuugua-3' (SEQ ID NO: 14)
5'-uacaaaagaaaauugguaaca-3' (SEQ ID NO: 152)
5'-cgggguuuuucuuguugacaa-3' (SEQ ID NO: 24)
5'-uugucaacaagaaaaaccccg-3' (SEQ ID NO: 162)
5'-uuuacuagugccauuuguuca-3' (SEQ ID NO: 28)
5'-ugaacaaauggcacuaguaaa-3' (SEQ ID NO: 166)

In certain embodiments, the dsRNA agent comprises at least one modified nucleotide. In some embodiments, all or substantially all of the nucleotides of the antisense strand are modified nucleotides. In certain embodiments, the at least one modified nucleotide comprises: a 2'-O-methyl nucleotide,2'-Fluoro nucleotide, 2'-deoxy nucleotide, 2'3'-seco nucleotide mimic, locked nucleotide, unlocked nucleic acid nucleotide (UNA), glycol nucleic acid nucleotide (GNA), 2'-F-Arabino nucleotide, 2'-methoyxyethyl nucleotide, abasic nucleotide, ribitol, inverted nucleotide, inverted abasic nucleotide, inverted 2'-OMe nucleotide, inverted 2'-deoxy nucleotide, 2'-amino-modified nucleotide, 2'-alkyl-modified nucleotide, mopholino nucleotide, and 3'-OMe nucleotide, a nucleotide comprising a 5'-phosphorothioate group, or a terminal nucleotide linked to a cholesteryl derivative or dodecanoic acid bisdecylamide group, a 2'-amino-modified nucleotide, a phosphoramidate, or a non-natural base comprising nucleotide. In some embodiments, the dsRNA agent comprises an E-vinylphosphonate nucleotide at the 5' end of the antisense strand. In some embodiments, the dsRNA agent comprises at least one phosphorothioate internucleoside linkage. In some embodiments, the sense strand comprises at least one phosphorothioate internucleoside linkage. In certain embodiments, the antisense strand comprises at least one phosphorothioate internucleoside linkage. In some embodiments, the sense strand comprises 1, 2, 3, 4, 5, or 6, phosphorothioate internucleoside linkages. In some embodiments, the antisense strand comprises 1, 2, 3, 4, 5, or 6, phosphorothioate internucleoside linkages. In certain embodiments, all or substantially all of the nucleotides of the sense strand and the antisense strand are modified nucleotides. In some embodiments, the antisense strand comprises 15 or more modified nucleotides independently selected from a 2'-O-methyl nucleotide and a 2'-fluoro nucleotide, wherein less than 6 modified nucleotides are 2'-fluoro nucleotides. In certain embodiments, the antisense strand comprises 3 or 5 2'-fluoro nucleotides. In some embodiments, the antisense strand comprises 15 or more modified nucleotides independently selected from a 2'-O-methyl nucleotide and a 2'-fluoro nucleotide, wherein at least 14, 15 or 16 modified nucleotides are 2'-O-methyl nucleotide and the nucleotides at position 2, 7, 12, 14 and/or 16 from the 5' end of the antisense strand are a 2'-fluoro nucleotide. In some embodiments, the dsRNAs include a sense strand and an antisense strand, the antisense strand is complementary to at least a portion of an mRNA corresponding to the target gene, wherein the antisense strand of dsRNA comprising a nucleotide sequence shown as formula (C) and the sense strand of dsRNA comprising a nucleotide sequence shown as formula (D),
3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' Formula (C)
5'-(N'_{L})_{n'} N'_{L}N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L}-3' Formula (D)
wherein, each N_{F} or N'_{F} represents a 2'-fluoro-modified nucleotide; each of N_{M1}, N_{M2}, N_{M3}, N_{M4}, N_{M5}, N_{M6}, N_{M7}, N_{M5}, N'_{N1}, N'_{N2}, N'_{N3}, N'_{N4}, N'_{N5} and N'_{N6} independently represents a modified or unmodified nucleotide and N_{M4}, N_{M5}, N_{M7} and N_{M8} are not 2'-fluoro-modified nucleotides; each N_{L} or N'_{L} independently represents a modified or unmodified nucleotide but not a 2'-fluoro-modified nucleotide, and n and n' may be an integer of 0 to 7.

In some embodiments, the dsRNAs include a sense strand and an antisense strand, the antisense strand comprising a region of complementarity which includes at least 15 contiguous nucleotides that differ by 0, 1, 2, or 3 nucleotides from any one of the nucleotide sequences selected from the group consisting of
5'- u*U*ugucAacaaGaAaAacc*c*c -3' (SEQ ID NO: 582)
5'- u*A*cuuuCcaauCaAuAggc*c*u -3' (SEQ ID NO: 585)
5'- u*G*uaaaGagagGuGcGccc*c*g -3' (SEQ ID NO: 588)
5'- u*A*caaaAgaaaAuUgGuaa*c*a -3' (SEQ ID NO: 592)
5'- u*C*aaaaGaaaaUuGgUaac*a*g -3' (SEQ ID NO: 594)
5'- u*U*gucaAcaagAaAaAccc*c*g -3' (SEQ ID NO: 596)
5'- u*G*aacaAauggCaCuAgua*a*a -3' (SEQ ID NO: 597)
5'- u*A*gacaAaagaAaAuUggu*a*a -3' (SEQ ID NO: 598)
5'- a*U*gucaAcaagAaAaAccc*u*g -3' (SEQ ID NO: 666)

In some embodiments, the modified sense strand is a modified sense strand sequence set forth in one of Tables 2-4. In certain embodiments, the modified antisense strand is a modified antisense strand sequence set forth in one of Tables 2-4. In certain embodiments, the sense strand is complementary or substantially complementary to the antisense strand, and the region of complementarity is between 16 and 23 nucleotides in length. In some embodiments, the region of complementarity is 19-21 nucleotides in length. In certain embodiments, the region of complementarity is 14, 15, 16, 17, 18, 19, 20, 21, 22, 23, 24, 25, 26, 27, 28, 29, or 30 nucleotides in length. In some embodiments, each strand is no more than 40 nucleotides in length. In some embodiments, each strand is no more than 30 nucleotides in length. In certain embodiments, each strand is no more than 27 nucleotides in length. In certain embodiments, each strand is no more than 25 nucleotides in length. In certain embodiments, each strand is no more than 23 nucleotides in length. In some embodiments, each strand is no more than 21 nucleotides in length. In certain embodiments, each strand is 19-32 nucleotides in length. In certain embodiments, each strand is 19, 20, 21, 22, 23, 24, 25, 26, 32 nucleotides in length. In some embodiments, the dsRNA agent comprises at least one modified nucleotide and further comprises one or more targeting groups or linking groups. In some embodiments, the one or more targeting groups or linking groups are conjugated to the sense strand. In some embodiments, the targeting group or linking group comprises N-acetyl-galactosamine (GalNAc). In certain embodiments, the targeting group has a structure: 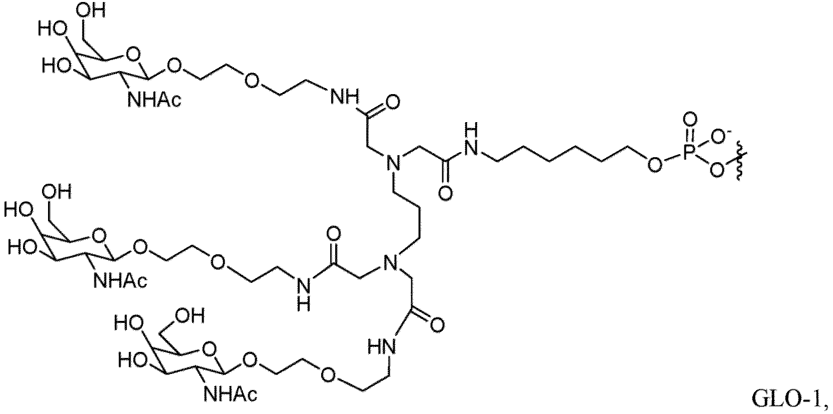 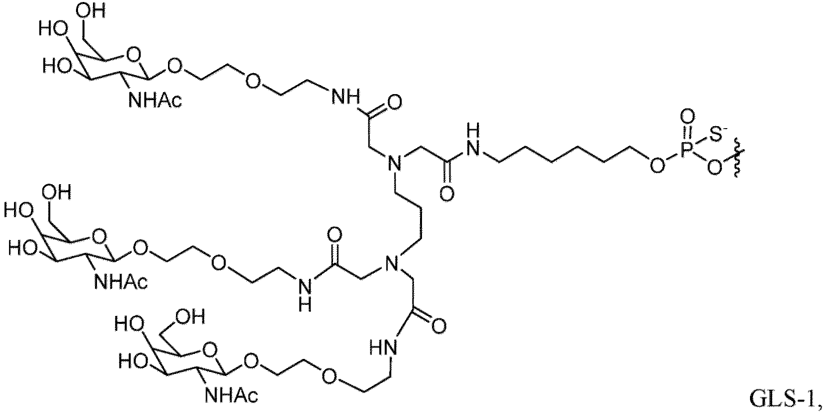 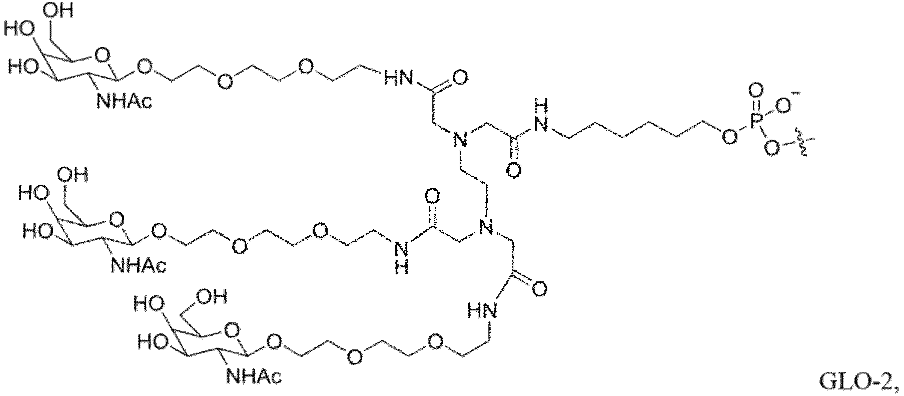 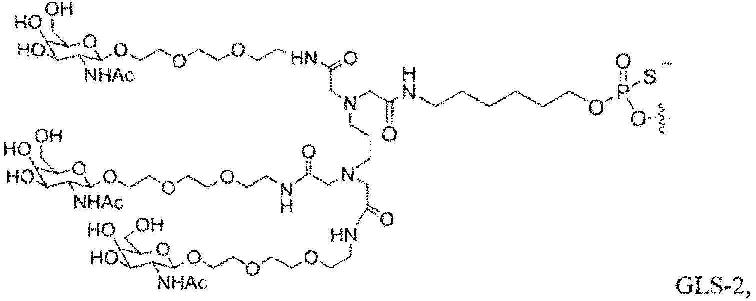 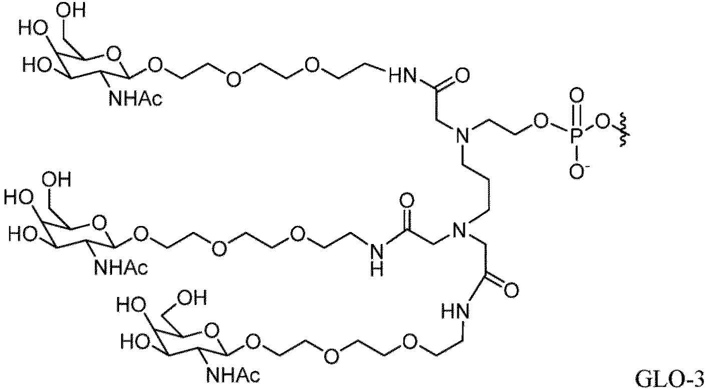 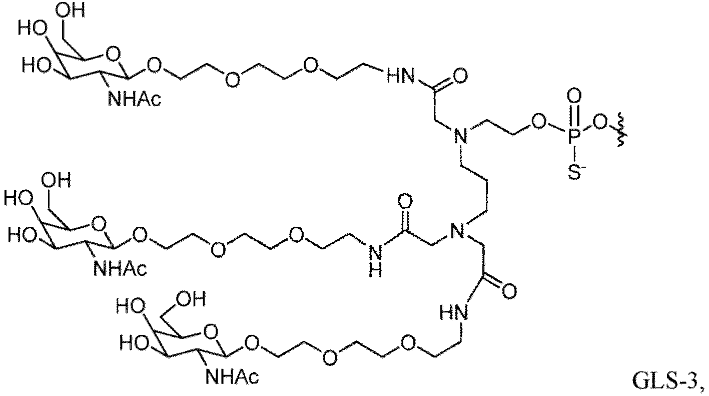 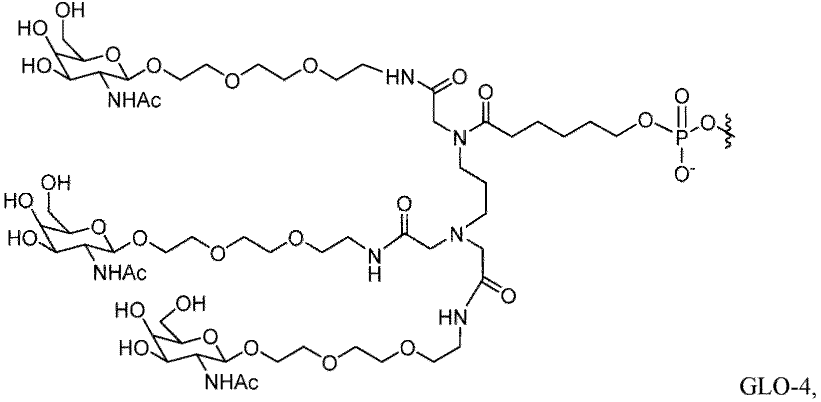 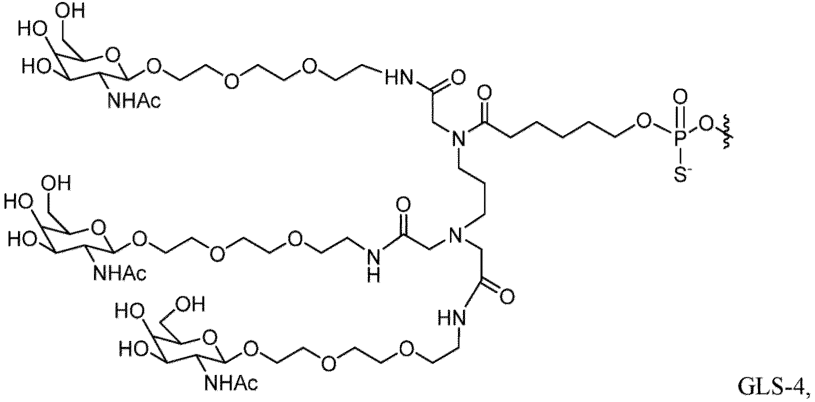 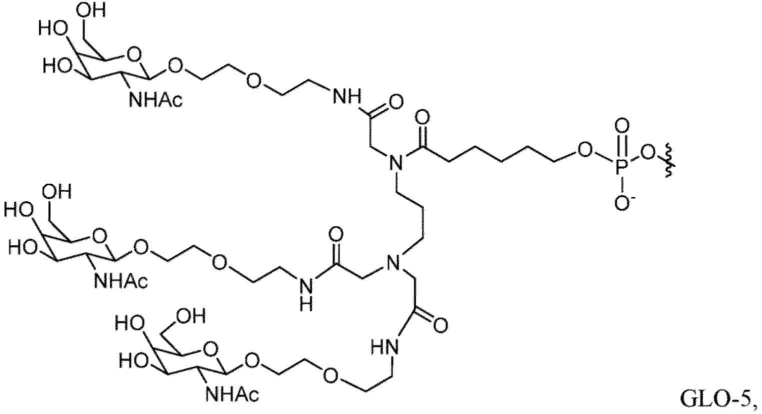 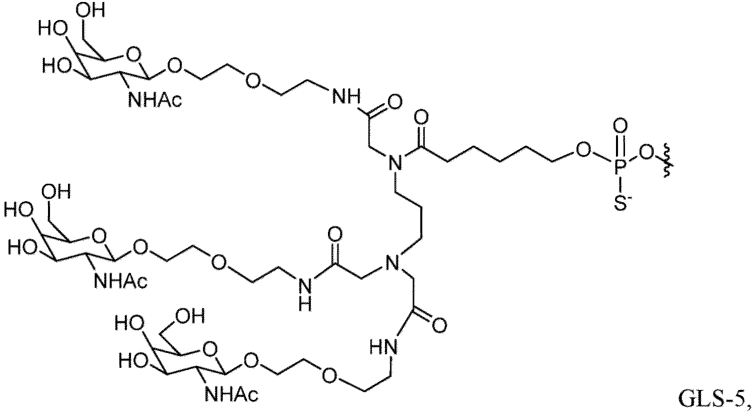 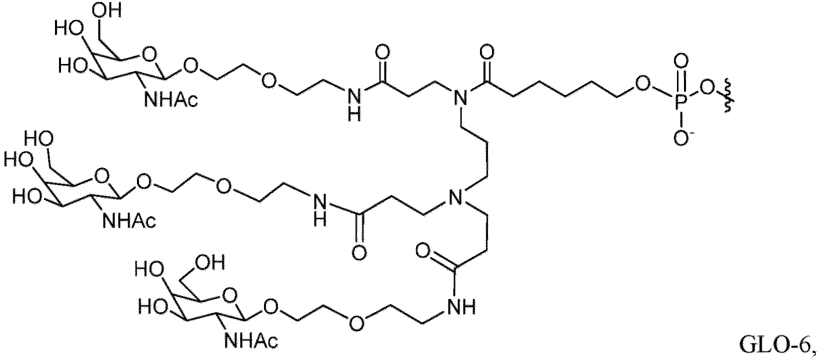 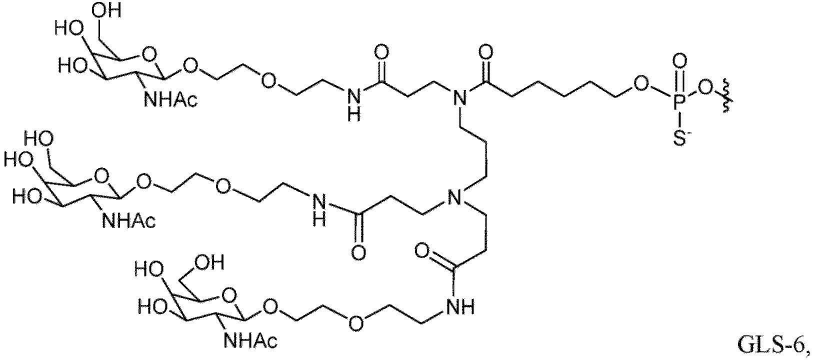 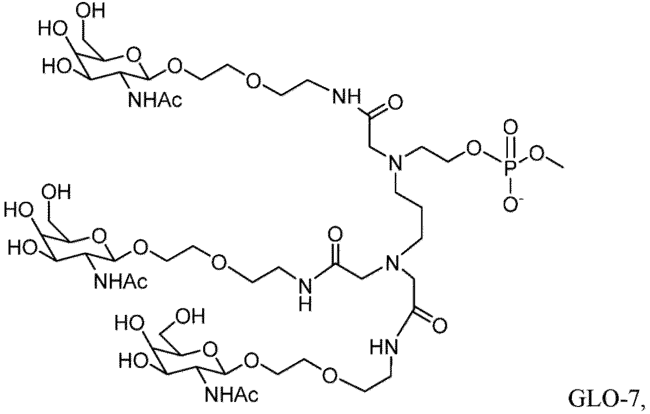 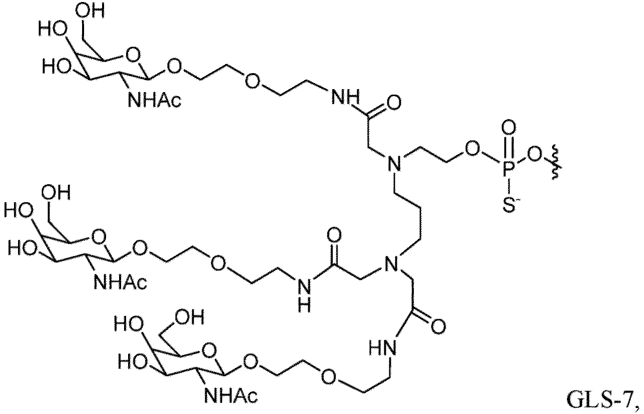 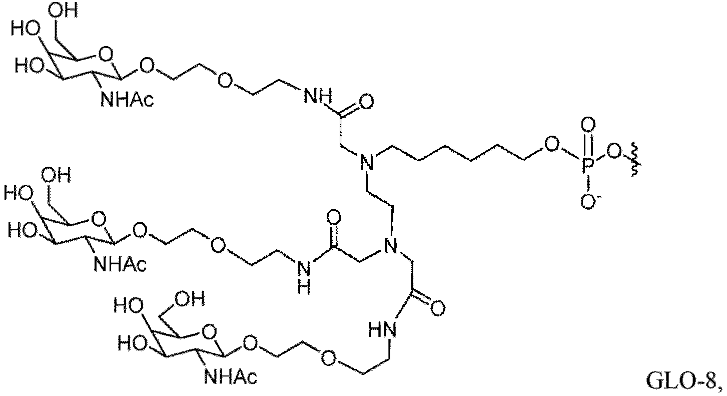 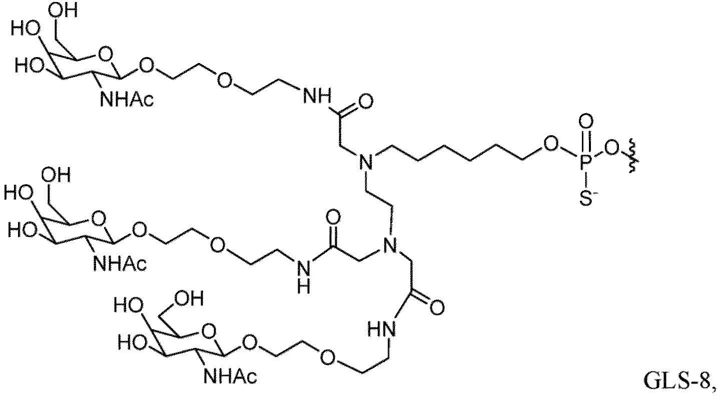 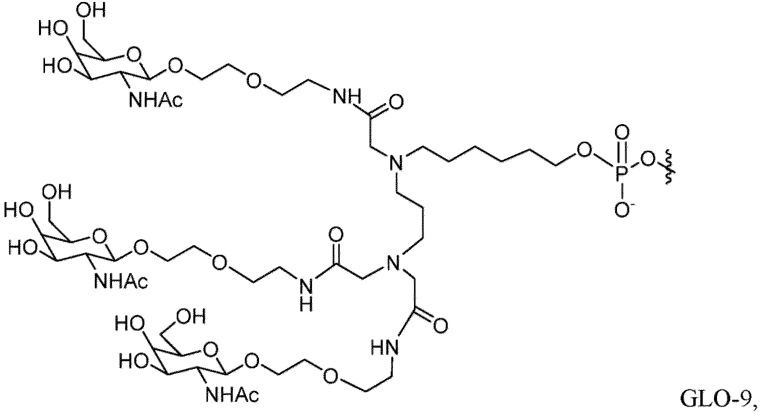 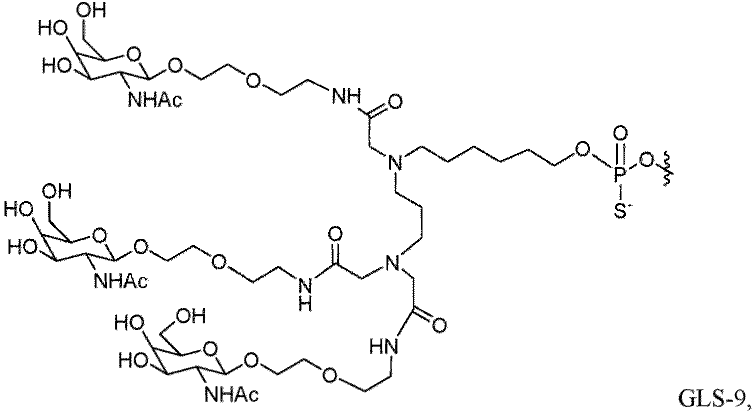 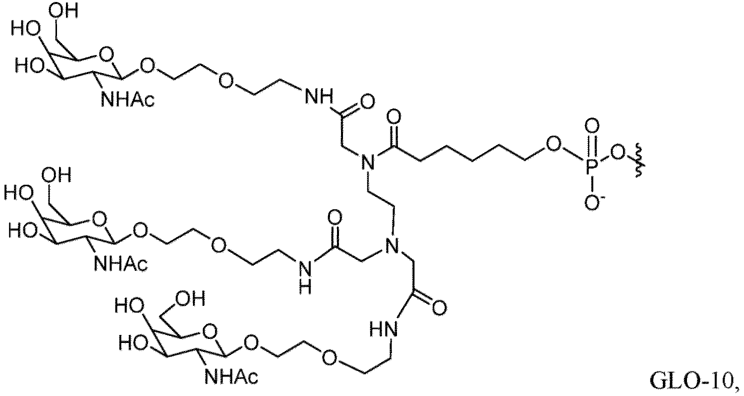 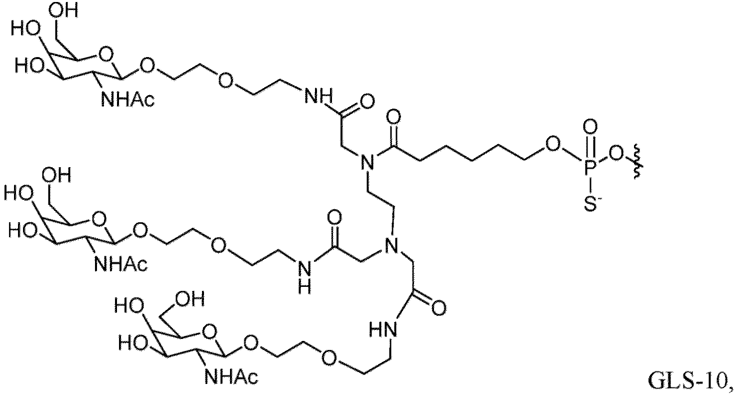 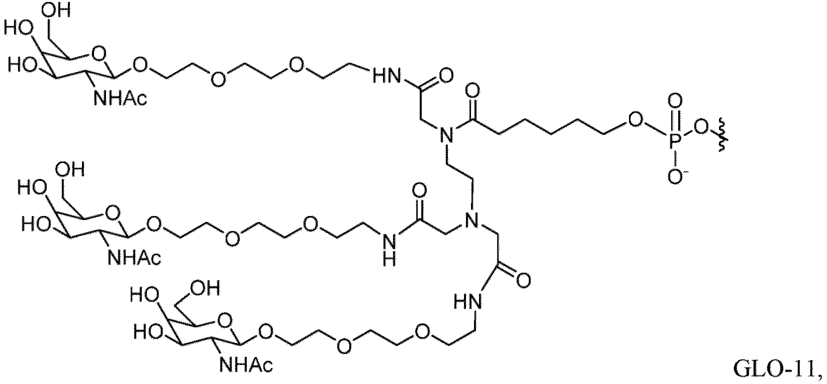 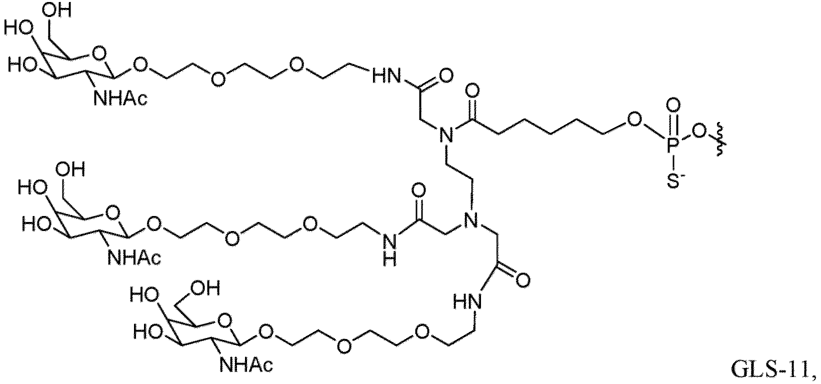 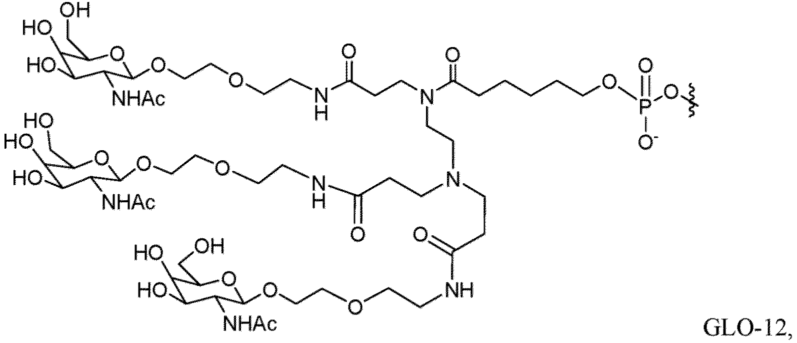 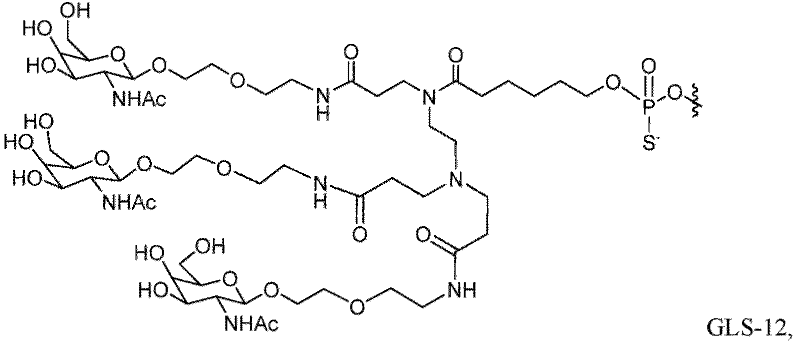 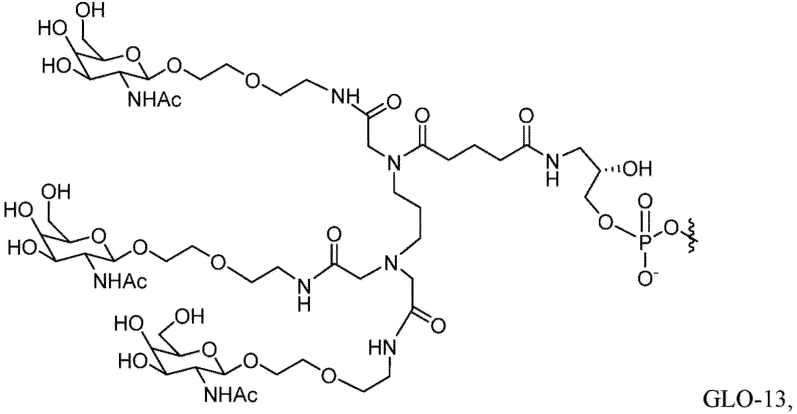 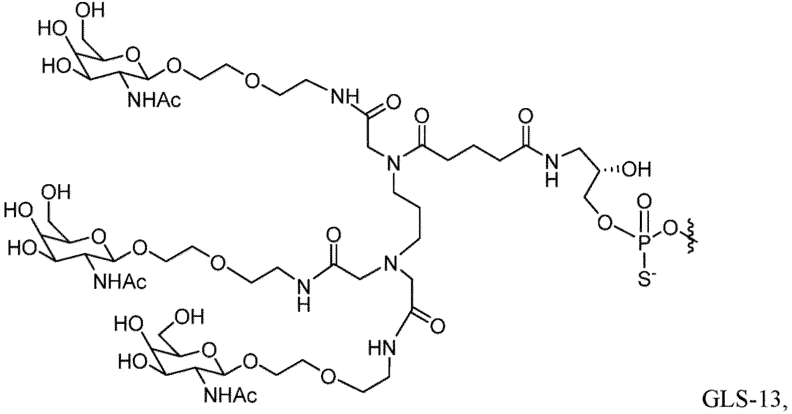 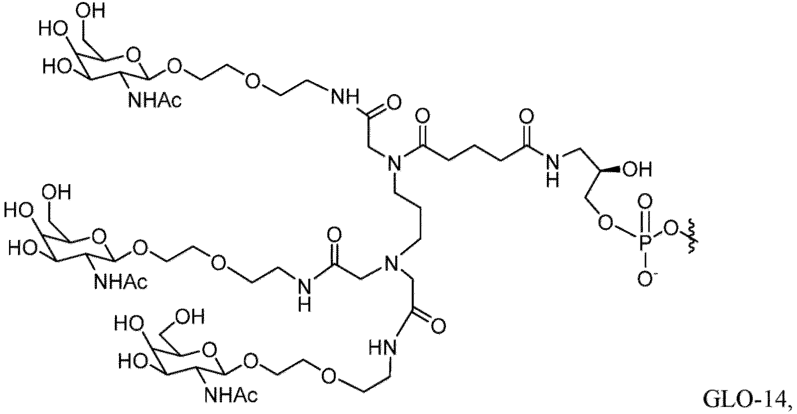 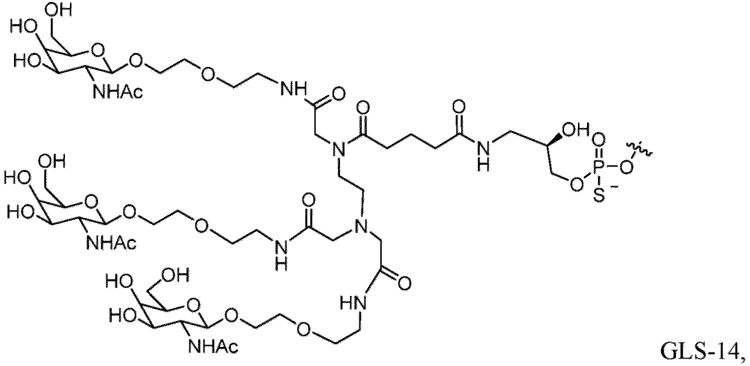 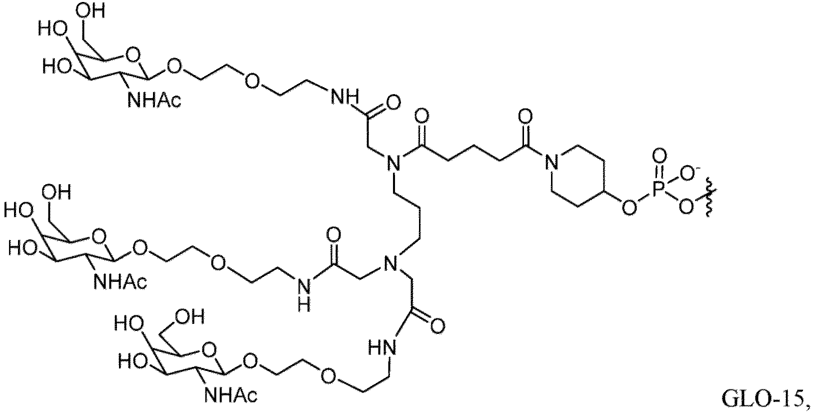 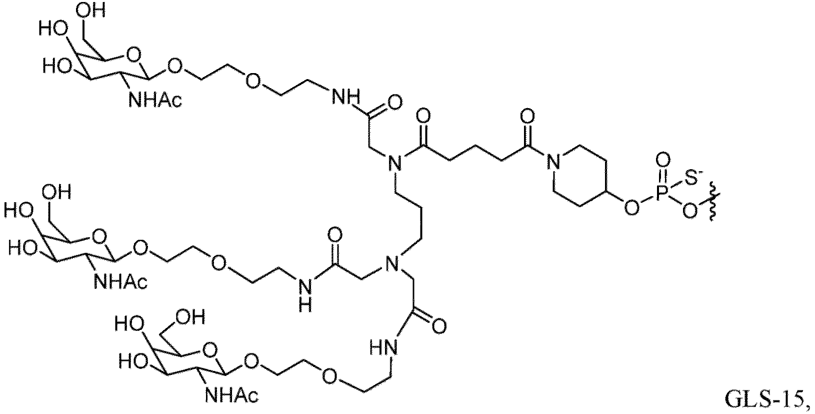 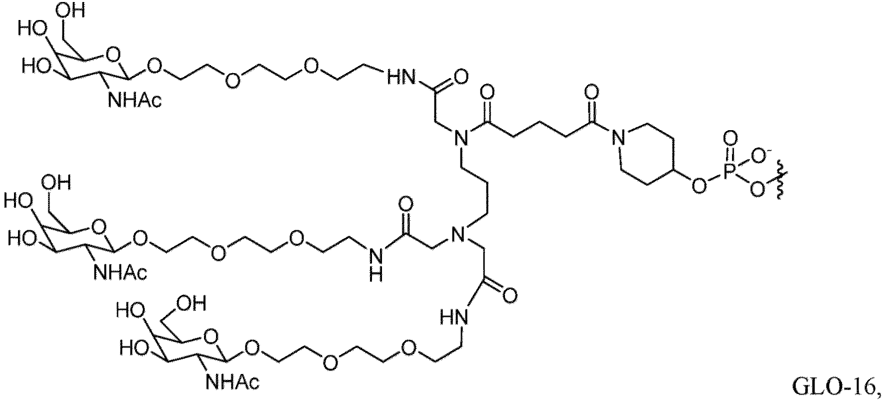 or

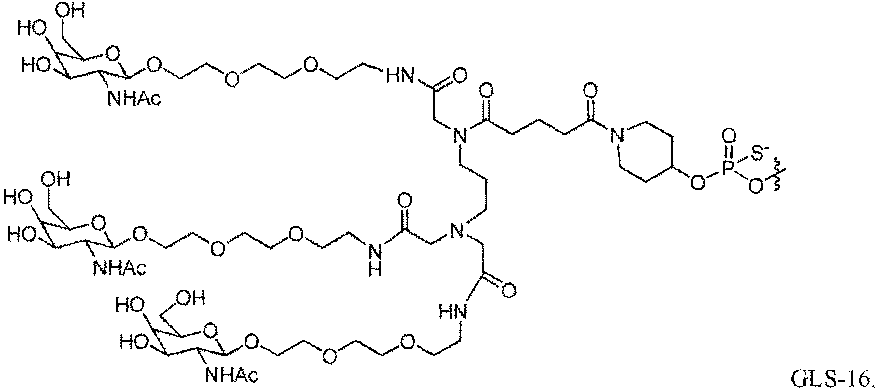

In some embodiments, the dsRNA agent comprises a targeting group that is conjugated to the 5'-terminal end of the sense strand. In some embodiments, the dsRNA agent comprises a targeting group that is conjugated to the 3'-terminal end of the sense strand. In certain embodiments, the antisense strand comprises one inverted abasic residue at 3'-terminal end. In certain embodiments, the sense strand comprises one or two inverted abasic residues at the 3' and/or the 5' terminal end. In some embodiments, the dsRNA agent has two blunt ends. In certain embodiments, at least one strand comprises a 3' overhang of at least 1 nucleotide. In some embodiments, at least one strand comprises a 3' overhang of at least 2 nucleotides. In some embodiments, the sense strand comprises one of: SEQ ID NO: 281, 290, 295, 300, 304, 306, 307, 331, 557, 567, 569, 571, 572, 573, 560, 563, 607, 628-637 or 918-921. In some embodiments, the antisense strand comprises one of: SEQ ID NO: 419, 428, 433, 438, 442, 444, 445, 469, 582, 592, 594, 596, 597, 598, 585, 588, 642 , 663-672 or 922-925 In certain embodiments, the dsRNA agent comprises: SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; SEQ ID NO: 637 and SEQ ID NO: 672; SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925. In certain embodiments, the dsRNA comprises a duplex selected from the group consisting of AD00170, AD00378, AD00170-1, AD00263, AD00265, AD00267, AD00268, AD00269, AD00173, AD00176, AD00383, AD00384, AD00378-1, AD00170-2, AD00268-2 and AD00263-2.

According to another aspect of the invention, a composition in provided that includes one, two, three, or more dsRNA agents of any embodiment of any of the aforementioned dsRNA agents. In some embodiments, the composition also includes a pharmaceutically acceptable carrier. In certain embodiments, the composition also includes one or more additional therapeutic agents. In certain embodiments, the composition is packaged in a kit, container, pack, dispenser, pre-filled syringe, or vial. In some embodiments, the composition is formulated for subcutaneous administration or is formulated for intravenous (IV) administration. In some embodiments, the sense strand of at least one of the dsRNA agents comprises one of: SEQ ID NO: 281, 290, 295, 300, 304, 306, 307, 331, 557, 567, 569, 571, 572, 573, 560, 563, 607, 628-637 or 918-921. In some embodiments, the antisense strand of at least one of the dsRNA agents comprises one of: SEQ ID NO: 419, 428, 433, 438, 442, 444, 445, 469, 582, 592, 594, 596, 597, 598, 585, 588, 642 , 663-672 or 922-925. In certain embodiments, the dsRNA agent comprises: SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; SEQ ID NO: 637 and SEQ ID NO: 672; SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925. In certain embodiments, the dsRNA comprises a duplex selected from the group consisting of AD00170, AD00378, AD00170-1, AD00263, AD00265, AD00267, AD00268, AD00269, AD00173, AD00383, AD00384, AD00378-1, AD00170-2, AD00268-2, AD00263-2 and AD00176.

According to another aspect of the invention, a cell is provided that includes a dsRNA agent of any embodiment of an aforementioned aspect of the invention. In some embodiments, the cell is a mammalian cell, optionally a human cell.

According to another aspect of the invention, a method of inhibiting the expression of a hepatitis B virus (HBV) gene in a cell is provided, the method including (i) preparing a cell that includes an effective amount of one or more double-stranded ribonucleic acid (dsRNA) agents of any embodiment of an aforementioned dsRNA agent aspect of the invention or any embodiment of a composition of any aforementioned aspect of the invention. In some embodiments, the method also includes (ii) maintaining the cell prepared for a time sufficient to obtain degradation of the mRNA transcript of an HBV gene, thereby inhibiting expression of the HBV gene in the cell; inhibiting replication of the HBV in the cell; and reducing a level of the HBV antigens in the cell. In some embodiments, the one or more HBV antigens are HBcAg, HBsAg, and HBeAg. In certain embodiments, the cell is in a subject and the dsRNA agent is administered to the subject subcutaneously. In some embodiments, the cell is in a subject and the dsRNA agent is administered to the subject by IV administration. In some embodiments, the cell is in a subject. In some embodiments, 2, 3, 4, or more dsRNA agents are administered to the subject. In certain embodiments, the method also includes assessing inhibition of the HBV gene, following the administration of the dsRNA agent(s) to the subject, wherein a means for the assessing includes: (i) determining one or more physiological characteristics of an HBV-associated disease or condition in the subject and (ii) comparing the determined physiological characteristic(s) to a baseline pre-treatment physiological characteristic of the HBV-associated disease or condition and/or to a control physiological characteristic of the HBV-associated disease or condition, wherein the comparison indicates one or more of a presence or absence of inhibition of expression of the HBV gene in the subject. In some embodiments, the control physiological characteristic is the physiological characteristic in a subject infected with HBV and not administered the dsRNA agent(s). In some embodiments, the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject. In some embodiments, the physiological characteristic is determined in a biological sample obtained from the subject. In certain embodiments, the biological sample comprises one or more of: a serum sample, a tissue sample, a cell sample, and a liver sample. In some embodiments, the determined physiological characteristic in the subject is abnormal compared to a control level of the physiological characteristic. In some embodiments, a level of one or more of ALT; AST; HBV viral load, HBV covalently closed circular DNA (cccDNA); one or more HBV antigens; one or more of: HBcAg, HBsAg and HBeAg; and one or more anti-Hepatitis B virus antibodies in the subject determined to be statistically significantly higher compared to a control physiological characteristic of the HBV-associated disease or condition and/or the baseline pre-treatment physiological characteristic of the HBV-associated disease or condition indicates a status of HBV gene expression in the subject. In certain embodiments, the control physiological characteristic is the physiological characteristic in a subject infected with HBV and not administered the dsRNA agent(s). In certain embodiments, a reduction in one or more of the ALT; AST; HBV viral load, HBV covalently closed circular DNA (cccDNA); one or more HBV antigens; HBcAg; HBsAg; and HBeAg in the subject indicates a reduction of HBV gene expression in the subject. In some embodiments, an increase in anti-Hepatitis B virus antibodies in the subject compared to a control physiological characteristic of the HBV-associated disease or condition and/or the baseline pre-treatment physiological characteristic of the HBV-associated disease or condition, indicates a reduction in HBV gene expression in the subject. In some embodiments, the HBV-associated disease or condition is one or more of: wherein the disease or condition is one or more of: hepatitis B, chronic hepatitis B, hepatitis D virus infection (delta hepatitis or HDV), liver injury, cirrhosis, acute hepatitis B, acute fulminant hepatitis, and liver fibrosis, liver inflammation, and hepatocellular carcinoma. In certain embodiments, the HBV-associated disease or condition is one or more of: hepatitis B, chronic hepatitis B, hepatitis D virus infection (delta hepatitis or HDV) and acute hepatitis B.

According to another aspect of the invention a method of inhibiting expression of an HBV gene in a subject is provided, the method including administering to the subject an effective amount of one or more double-stranded ribonucleic acid (dsRNA) agents of any embodiment of an aforementioned dsRNA agent aspect of the invention, or any embodiment of an aforementioned composition of the invention. In some embodiments, two, three, four, or more of the dsRNA agents are administered to the subject. In some embodiments, the dsRNA agent is administered to the subject subcutaneously. In certain embodiments, the dsRNA agent is administered to the subject by IV administration. In some embodiments, the method also includes assessing inhibition of the HBV gene, following the administration of the one or more dsRNA agents, wherein a means for the assessing comprises: (i) determining one or more physiological characteristics of an HBV-associated disease or condition in the subject and (ii) comparing the determined physiological characteristic(s) to a baseline pre-treatment physiological characteristic of the HBV-associated disease or condition and/or to a control physiological characteristic of the HBV-associated disease or condition, wherein the comparison indicates one or more of a presence or absence of inhibition of expression of the HBV gene in the subject. In some embodiments, the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject. In some embodiments, the physiological characteristic is determined in a biological sample obtained from the subject. In certain embodiments, the biological sample includes one or more of: a serum sample, a cell sample, a tissue sample, and a liver sample. In some embodiments, the determined physiological characteristic in the subject is more abnormal compared to a baseline pre-treatment physiological characteristic of the HBV-associated disease or condition and/or to a control physiological characteristic of the HBV-associated disease or condition. In some embodiments, a level of one or more of ALT; AST; HBV viral load, HBV covalently closed circular DNA (cccDNA); one or more HBV antigens; one or more of: HBcAg, HBsAg and HBeAg; and one or more anti-Hepatitis B virus antibodies in the subject determined to be statistically significantly higher, lower, or unchanged compared to a baseline pre-treatment physiological characteristic of the HBV-associated disease or condition and/or to a control physiological characteristic of the HBV-associated disease or condition indicates a status of HBV gene expression in the subject. In certain embodiments, a reduction in one or more of the ALT; AST; HBV viral load, HBV covalently closed circular DNA (cccDNA); one or more HBV antigens; HBcAg; HBsAg; and HBeAg in the subject indicates a reduction of HBV gene expression in the subject. In some embodiments, an increase in anti-Hepatitis B virus antibodies in the subject compared to a baseline pre-treatment physiological characteristic of the HBV-associated disease or condition and/or to a control physiological characteristic of the HBV-associated disease or condition, indicates a reduction in HBV gene expression in the subject. In certain embodiments, the HBV-associated disease or condition is one or more of: hepatitis B, chronic hepatitis B, acute hepatitis B, hepatocellular carcinoma, end-stage liver disease, liver fibrosis, liver inflammation, liver injury, cirrhosis, and a hepatitis D virus infection (delta hepatitis or HDV). In certain embodiments, the HBV-associated disease or condition is one or more of: wherein the disease or condition is one or more of: hepatitis B, chronic hepatitis B, hepatitis D virus infection (delta hepatitis or HDV) and acute hepatitis B.

According to another aspect of the invention, a method of treating a disease or condition associated with the presence of a hepatitis B virus (HBV) protein is provided, the method including administering to a subject an effective amount of one or more double-stranded ribonucleic acid (dsRNA) agents of any embodiment of an aforementioned aspect of a dsRNA agent of the invention, or any embodiment of an aforementioned composition of the invention to inhibit expression of the HBV gene encoding the HBV protein. In some embodiments, the method includes administering two, three, four, or more dsRNA agents to the subject. In some embodiments, the disease or condition is one or more of: hepatitis B, chronic hepatitis B, hepatitis D virus infection (delta hepatitis or HDV), liver injury, cirrhosis, acute hepatitis B, acute fulminant hepatitis, liver inflammation, liver fibrosis, and hepatocellular carcinoma. In certain embodiments, the method also includes administering an additional therapeutic regimen to the subject. In certain embodiments, the additional therapeutic regimen comprises: administering to the subject one or more: HBV antisense polynucleotides, additional HBV dsRNA therapeutic agent, a non-HBV dsRNA therapeutic agent, a HBV non-dsRNA therapeutic agent, and a behavioral modification. In some embodiments, the non-HBV dsRNA therapeutic agent is one of more of: an antiviral agent, an antiviral nucleoside or nucleotide analogue (NUC), a viral polymerase inhibitor, a reverse transcriptase inhibitor, an immune stimulator, a therapeutic vaccine, a viral entry inhibitor, an oligonucleotide that inhibits the secretion or release of HBsAg, a capsid inhibitor, a covalently closed circular (ccc) HBV DNA inhibitor. In some embodiments, the NUC is: Tenofovir disoproxil fumarate (TDF), Tenofovir alafenamide, Lamivudine, Adefovir dipivoxil, Entecavir (ETV), or Telbivudine. In some embodiments, the immune stimulator is pegylated interferon alfa 2a (PEG-IFN-a2a), Interferon alfa-2b, a recombinant human interleukin-7, a Toll-like receptor 7 (TLR7) agonist, or a checkpoint inhibitor (e.g., PD1 inhibitor). In certain embodiments, the one or more dsRNA agent(s) is administered to the subject subcutaneously. In certain embodiments, the one or more dsRNA agent(s) is administered to the subject by IV administration. In some embodiments, the method also includes determining an efficacy of the administered one or more double-stranded ribonucleic acid (dsRNA) agent(s) in the subject. In certain embodiments, a means of determining an efficacy of the treatment in the subject comprises: (i) determining one or more physiological characteristics of the HBV-associated disease or condition in the subject and (ii) comparing the determined physiological characteristic(s) to a baseline pre-treatment physiological characteristic of the HBV-associated disease or condition, wherein the comparison indicates one or more of a presence, absence, and level of efficacy of the administration of the double-stranded ribonucleic acid (dsRNA) agent to the subject. In some embodiments, the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject. In certain embodiments, the physiological characteristic is determined in a biological sample obtained from the subject. In some embodiments, the biological sample comprises one or more of: a serum sample, a cell sample, a tissue sample, and a liver sample. In some embodiments, the determined physiological characteristic in the subject is different compared to the baseline pre-treatment physiological characteristic of the HBV-associated disease or condition or to a control level of the physiological characteristic. In certain embodiments, a level of one or more of ALT; AST; HBV viral load, HBV covalently closed circular DNA (cccDNA); one or more HBV antigens; one or more of: HBcAg, HBsAg and HBeAg in the subject determined to be statistically significantly lower compared to the baseline pre-treatment physiological characteristic of the HBV-associated disease or condition indicates a status of reduced HBV gene expression in the subject. In some embodiments, an increase in anti-Hepatitis B virus antibodies in the subject compared to the baseline pre-treatment anti-Hepatitis B virus antibodies or a control level of anti-Hepatitis B virus antibodies indicates a reduction in HBV gene expression in the subject. In some embodiments, the HBV-associated disease or condition is one or more of: hepatitis B, chronic hepatitis B, acute hepatitis B, hepatocellular carcinoma, end-stage liver disease, acute fulminant hepatitis, liver inflammation, liver injury, cirrhosis, liver fibrosis, and hepatitis D virus infection (delta hepatitis or HDV).

According to another aspect of the invention, a method of decreasing a level of HBV protein in a subject compared to a baseline pre-treatment level of HBV protein in the subject is provided, the method including administering to the subject an effective amount of one or more double-stranded ribonucleic acid (dsRNA) agents of any embodiment of an aforementioned aspect of a dsRNA agent of the invention, or any embodiment of an aforementioned composition of the invention, to decrease the level of HBV gene expression. In certain embodiments, the dsRNA agent is administered to the subject subcutaneously or is administered to the subject by IV administration.

According to another aspect of the invention, a method of altering a physiological characteristic of a hepatitis B virus (HBV)-associated disease or condition in a subject compared to a baseline pre-treatment physiological characteristic of the HBV-associated disease or condition in the subject is provided, the method including administering to the subject an effective amount of one or more double-stranded ribonucleic acid (dsRNA) agents of any embodiment of an aforementioned aspect of a dsRNA agent of the invention, or any embodiment of an aforementioned composition of the invention, to alter the physiological characteristic of the HBV-associated disease or condition in the subject. In some embodiments, the one or more dsRNA agent(s) is administered to the subject subcutaneously or is administered to the subject by IV administration. In certain embodiments, the physiological characteristic is one or more of determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject.

According to another aspect of the invention, the aforementioned dsRNA agent for use in a method of treating a disease or condition associated with HBV gene expression is provided. In some embodiments, the disease or condition is one or more of: hepatitis B, chronic hepatitis B, hepatitis D virus infection (delta hepatitis or HDV), liver injury, cirrhosis, acute hepatitis B, acute fulminant hepatitis, and liver fibrosis, liver inflammation, and hepatocellular carcinoma.

In certain embodiments of any aforementioned method of the invention, the sense strand of the dsRNA agent includes one of: SEQ ID NO: 281, 290, 295, 300, 304, 306, 307, 331, 557, 567, 569, 571, 572, 573, 560, 563, 607, 628-637 or 918-921. In some embodiments of any aforementioned method of the invention, the antisense strand of the dsRNA agent comprises one of: SEQ ID NO: 419, 428, 433, 438, 442, 444, 445, 469, 582, 592, 594, 596, 597, 598, 585, 588, 642, 663-672 or 922-925. In certain embodiments of any aforementioned method of the invention, the dsRNA agent comprises: SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; SEQ ID NO: 637 and SEQ ID NO: 672 ; SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925. In certain embodiments, the dsRNA comprises a duplex selected from the group consisting of AD00170, AD00378, AD00170-1, AD00263, AD00265, AD00267, AD00268, AD00269, AD00173, AD00383, AD00384, AD00378-1, AD00170-2, AD00268-2, AD00263-2 and AD00176.

### Brief Description of the Sequences

SEQ ID NOs: 1-138, 674-734 are shown in Table 1 and are sense strand sequences.
SEQ ID NOs: 139-276, 735-795 are shown in Table 1 and are antisense strand sequences.
SEQ ID NOs: 277-414, 796-856 are sense strand sequences and SEQ ID NOs: 415-552, 857-917 are antisense strand sequences and are shown in Table 2 with chemical modifications indicated by upper case: 2'-Fluoro; lower case: 2'-OMe; and thiophosphate: *.
SEQ ID NOs: 553-577 are sense strand sequences and SEQ ID NO: 578-602 are antisense strand sequences and are shown in Table 3. A delivery molecule is indicated as "GLO-0" at the 3' end of each sense strand. Chemical modifications are indicated as: upper case: 2'-Fluoro; lower case: 2'-OMe; and thiophosphate: *
SEQ ID NOs: 603-637, 918-921 are sense strand sequences and 638-672, 922-925 are antisense strand sequences and are shown in Table 4. Chemical modifications are indicated as: upper case: 2'-Fluoro; lower case: 2'-OMe; thiophosphate: *; and Invab = inverted abasic.
SEQ ID NO: 673 is Homo sapiens Hepatitis B virus (HBV) mRNA sequence [NCBI Reference Sequence: AM282986]:

ttccactgccttccaccaagctctgcaggatcccaaagtcaggggtctgtattttcctgctggtggctccagttcaggaacagtaaaccctg
ctccgaatattgcctctcacatctcgtcaatctccgcgaggactggggaccctgtgacgaatatggagaacatcacatcaggattcctagg

acccctgctcgtgttacaggcggggtttttcttgttgacaagaatcctcacaataccgcagagtctagactcgtggtggacttctctcaatttt
ctagggggatcacccgtgtgtcttggccaaaattcgcagtccccaacctccaatcactcaccaacctcctgtcctccaatttgtcctggttat
cgctggatgtgtctgcggcgttttatcatattcctcttcatcctgctgctatgcctcatcttcttgttggttcttctggattatcaaggtatgttgcc
cgtttgtcctctaattccaggaacaacaacaaccagtacgggaccatgcaaaacctgcacgactcctgctcaaggcaactctatgtttccct
catgttgctgtacaaaaccttcggatggaaattgcacctgtattcccatcccatcgtcttgggctttcgcaaaatacctatgggagtgggcct
cagtccgtttctcttggctcagtttactagtgccatttgttcagtggttcgtagggctttcccccactgtttggctttcagctatatggatgatgtg
gtattgggggccaagtctgtacagcatcgtgagtccctttataccgctgttaccaattttcttttgtctctgggtatacatttaaaccctaacaaa
acaaaaagatggggttattccctaaacttcatgggttacataattggaagttggggaacgttgccacaggatcatattgtacaaaagatcaa
acactgttttagaaaacttcctgttaacaggcctattgattggaaagtatgtcaaagaattgtgggtcttttgggctttgctgctccatttacaca
atgtggatatcctgccttaatgcctttgtatgcctgtatacaagctaaacaggctttcactttctcgccaacttacaaggcctttctaagtaaac
agtacatgaacctttaccccgttgctcggcaacggcctggtctgtgccaagtgtttgctgacgcaacccccactggctggggcttggccat
aggccatcagcgcatgcgtggaacctttgtggctcctctgccgatccatactgcggaactcctagccgcttgttttgctcgcagccggtct
ggggcaaagctcatcggaactgacaattctgtcgtcctctcgcggaaatatacatcgtttccatggctgctaggttgtactgccaactggat
ccttcgcgggacgtcctttgtttacgtcccgtcggcgctgaatcccgcggacgacccctctcggggccgcttgggactctctcgtcccctt
ctccgtctgccgttccagccgaccacggggcgcacctctctttacgcggtctccccgtctgtgccttctcatctgccggtccgtgtgcactt
cgcttcacctctgcacgttgcatggagaccaccgtgaacgcccatcagatcctgcccaaggtcttacataagaggactcttggactccca
gcaatgtcaacgaccgaccttgaggcctacttcaaagactgtgtgtttaaggactgggaggagctgggggaggagattaggttaaaggt
ctttgtattaggaggctgtaggcataaattggtctgcgcaccagcaccatgcaactttttcacctctgcctaatcatctcttgtacatgtcccac
tgttcaagcctccaagctgtgccttgggtggctttggggcatggacattgacccttataaagaatttggagctactgtggagttactctcgttt
ttgccttctgacttttttccttccgtcagagatctcctagacaccgcctcagctctgtatcgggaagccttagagtctcctgagcattgctcacc
tcaccatactgcactcaggcaagcaattctctgctggggggaattgatgactctagctacctgggtgggtaataatttggaagatccagcat
ccagggatctagtagtcaattatgttaatactaacatgggtttaaagatcaggcaactattgtggtttcatatatcttgccttacttttggaagag
agactgtacttgaatatttggtctctttcggagtgtggattcgcactcctccagcctatagaccaccaaatgcccctatcttatcaacacttcc
ggaaactactgttgttagacgacgggaccgaggcaggtcccctagaagaagaactccctcgcctcgcagacgcagatctcaatcgccg
cgtcgcagaagatctcaatctcgggaatctcaatgttagtattccttggactcataaggtgggaaactttactgggctttattcctctacagta
cctatctttaatcctgaatggcaaactccttcctttcctaagattcatttacaagaggacattattaataggtgtcaacaatttgtgggccctctc
actgtaaatgaaaagagaagattgaaattaattatgcctgctagattctatcctacccacactaaatatttgcccttagacaaaggaattaaac
cttattatccagatcaggtagttaatcattacttcaaaaccagacattatttacatactctttggaaggctggtattctatataagagggaaacc
acacgtagcgcatcattttgcgggtcaccatattcttgggaacaagagctacagcatgggaggttggtcatcgaaacctcgcaaaggcat
ggggacgaatctttctgttcccaaccctctgggattctttcccgatcatcagttggaccctgcattcggagccaactcaaacaatccagattg
ggacttcaaccccatcaaggaccactggccagcagccaaccaggtaggagtgggagcattcgggccagggttcacccctccacacgg
cggtgttttggggtggagccctcaggctcagggcatattgaccacagtgtcaacaattcctcctcctgcctccaccaatcggcagtcagg
aaggcagcctactcccatctctccacctctaagagacagtcatcctcaggccatgcagtggaa

### Brief Description of the Drawings

Figure 1 is a graph showing HBsAg level in AAV-HBV mouse serum after a single subcutaneous dose of siRNA compounds at 3 mg/kg.
Figure 2 is a graph showing HBV DNA level in AAV-HBV mouse serum after a single subcutaneous dose of siRNA compounds at 3 mg/kg.

### Detailed Description

The invention in part, includes RNAi agents, for example, though not limited to double stranded (ds) RNAi agents capable of inhibiting hepatitis B virus (HBV) gene expression. Anti-HBV dsRNA agents of the invention can target all five HBV transcripts, leading to suppression of all HBV viral proteins. Compounds, compositions, and methods of the invention provide anti-HBV RNAi therapeutic agents and treatments for chronic hepatitis B (CHB) and/or other HBV-associated diseases and conditions. Anti-HBV RNAi agents and compounds delivered to cells using methods of the invention are capable of inhibiting HBV gene expression, thereby reducing activity in the cell of the HBV protein product of the gene. Anti-HBV dsRNAi agents of the invention can be administered to a subject to treat an HBV-associated disease and/or condition in the subject.

In certain embodiments of the invention, agents, compounds, compositions and methods of the invention are used to achieve a "functional cure" of an HBV-associated disease and/or condition. A functional cure of an HBV infection and/or an HBV-associated disease or condition may be indicated by seroclearance of hepatitis B surface antigen (HBsAg). The term "seroclearance" as used herein means a reduction in a subject's level of detectable serum HBsAg in a subject and/or complete elimination of detectable HBsAg in a subject's serum. It will be understood that seroclearance may occur in a subject with or without seroconversion to hepatitis B surface antibody (anti-HBs).

An HBV RNAi as described herein is capable of inhibiting expression of HBV protein. In some embodiments of the invention reducing HBV expression in a cell or subject treats a disease or condition associated with HBV expression in the cell or subject, respectively. Non-limiting examples of diseases and conditions that may be treated by reducing HBV activity are: hepatitis B, chronic hepatitis B, acute hepatitis B, hepatocellular carcinoma, end-stage liver disease, liver inflammation, liver fibrosis, liver injury, cirrhosis, acute fulminant hepatitis, hepatitis D virus infection (delta hepatitis or HDV), or other diseases for which reducing a level and activity of HBV protein is medically beneficial to a subject.

The invention, in part, includes compositions comprising anti-HBV RNAi agents and methods of use of such compositions. Some aspects of the invention include pharmaceutical compositions comprising one or more HBV dsRNA agents and a pharmaceutically acceptable carrier. In certain embodiments, an anti-HBV RNAi agent of the invention is attached to a delivery compound capable of delivering the RNAi agent to a cell, including but not limited to a hepatocyte. In some embodiments, a delivery compound is a GalNAc-containing delivery compound. Certain pharmaceutical compositions of the invention include one, two, three, or more independently selected anti-HBV dsRNA agents, and may also include one or more independently selected delivery compounds. In some embodiments, two, three, four, or more anti-HBV dsRNAs capable of targeting one, two, three, four, or more different positions/regions of HBV mRNA, respectively, are administered to a cell and/or a subject.

The following describes ways to make and use compositions comprising anti-HBV dsRNA agents (duplexes) capable of inhibiting HBV gene expression, as well as compositions and methods for treating diseases and conditions caused and/or modulated by HBV gene expression. As used herein diseases and/or conditions caused or modulated by a presence and/or level of HBV gene expression are referred to as "HBV-associated diseases and/or conditions."

As used herein, the term "RNAi" refers to an agent comprising RNA that is capable of mediating targeted cleavage of an RNA transcript via an RNA-induced silencing complex (RISC) pathway. The terms "RNAi" and RNAi agent" may be used interchangeably herein with the terms "anti-HBV RNAi" and "anti-HBV RNAi agent"; "HBV RNAi" and "HBV RNAi agent"; "dsRNA" and "dsRNA agent"; "HBV dsRNA" and "HBV dsRNA agent"; and "anti-HBV dsRNA" and "anti-HBV dsRNA agent", respectively. As is known in the art, an RNAi target region refers to a contiguous portion of the nucleotide sequence of an mRNA molecule formed during the transcription of a gene, including messenger RNA (mRNA) that is a product of RNA processing of a primary transcription product. The target portion of the mRNA molecule (also referred herein to as the "target sequence") will be at least long enough to serve as a substrate for RNAi-directed cleavage at or near that portion. A target sequence may be from 8-30 nucleotides long (inclusive), from 10 - 30 nucleotides long (inclusive), from 12 - 25 nucleotides long (inclusive), from 15 - 23 nucleotides long (inclusive), from 16 -23 nucleotides long (inclusive), or from 18 - 23 nucleotides long (inclusive), including all shorter lengths within each stated range. In certain embodiment a target sequence is between 9 and 26 nucleotides long (inclusive), including all sub-ranges and integers there between. For example, though not intended to be limiting, in certain embodiments of the invention a target sequence is one of 8, 9, 10, 11, 12, 13, 14, 15, 16, 17, 18, 19, 20, 21, 22, 23, 24, 25, 26, 27, 28, 29, or 30 nucleotides long, with the sequence fully or at least substantially complementary to at least part of an RNA transcript of an HBV gene.

As used herein, a "dsRNA agent" means a composition that contains an RNA or RNA-like (e.g., chemically modified RNA) oligonucleotide molecule that is capable of degrading or inhibiting translation of messenger RNA (mRNA) transcripts of a target mRNA in a sequence specific manner. Although not wishing to be limited to a particular theory, dsRNA agents of the invention may operate through the RNA interference mechanism [i.e., inducing RNA interference through interaction with the RNA interference pathway machinery (RNA-induced silencing complex or RISC) of mammalian cells], or by any alternative mechanism(s) or pathway(s). Methods for silencing genes in plant, invertebrate, and vertebrate cells are well known in the art [see, for example, (Sharp et al., Genes Dev. 2001, 15:485; Bernstein, et al., (2001) Nature 409:363; Nykanen, et al., (2001) Cell 107:309; and Elbashir, et al., (2001) Genes Dev. 15:188)], the disclosure of each of which is incorporated herein by reference in its entirety.]. Art-known gene silencing procedures can be used in conjunction with the disclosure provided herein to inhibit expression of HBV.

dsRNA agents disclosed herein are comprised of a sense strand and an antisense strand, and include, but are not limited to: short interfering RNAs (siRNAs), RNAi agents, micro RNAs (miRNAs), short hairpin RNAs (shRNA), and dicer substrates. An antisense strand of a dsRNA agents described herein is at least partially complementary to the mRNA being targeted. It is understood in the art that different lengths of dsRNA duplex structure can be used to inhibit target gene expression. For example, dsRNAs having a duplex structure of 19, 20, 21, 22, and 23 base pairs are known to be effective to induce RNA interference (Elbashir et al., EMBO 2001, 20:6877-6888) and shorter or longer RNA duplex structures are also known in the art capable of inducing RNA interference. HBV dsRNAs in certain embodiments of the invention can include at least one strand of a length of minimally 21 nt or may have shorter duplexes. In certain embodiments a dsRNA agent of the invention is a duplex disclosed in any of Tables 1-4. In some embodiments, a dsRNA agent of the invention is a duplex disclosed in Tables 1-4, but minus 1, 2, 3, or 4 nucleotides on one or both ends of the duplex, and is also capable of reducing HBV expression. In some embodiments of the invention, HBV dsRNA agents may have a partial sequence of at least 15, 16, 17, 18, 19, 20, or more contiguous nucleotides from one or more sequences of Tables 1-4, and differ in their ability to inhibit the expression of an HBV gene by not more than 5%, 10%, 15%, 20%, 25%, or 30% from the level of inhibition resulting from a dsRNA comprising the full sequence. A sense sequence, an antisense sequence and a duplex disclosed in Tables 1-4 may be referred to herein as a "parent" sequence, meaning that the sequences disclosed in Tables 1-4 may be modified, shorten, lengthened, include substitutions, etc. as set forth herein, with the resulting sequences retaining all or at least a portion of the efficacy of their parent sequences in methods and compositions of the invention. Sense and antisense strands included in a dsRNA of the invention are independently selected. As used herein the term "independently selected" means each of two or more like elements can be selected independent of the selection of the other elements. For example, though not intended to be limiting, in preparing a dsRNA of the invention, one may select the "elements" of the two strands to include in the duplex. One selected element, the sense sequence may be SEQ ID NO: 553 (shown in Table 3) and the other selected element, the antisense sequence, may be SEQ ID NO: 578, or may be SEQ ID NO: 578 that is modified, shortened, lengthened, and/or includes 1, 2, or 3 substitutions as compared to its parent sequence SEQ ID NO: 578. It will be understood that a duplex of the invention need not include both sense and antisense sequences shown as paired in duplexes in Tables 1-4.

Tables 1-4 show certain HBV dsRNA agent antisense strand and sense strand core stretch base sequences. The term "base sequence" is used herein in reference to a polynucleotide sequence without chemical modifications or delivery compounds. For example, the sense strand acuucucucaauuuucuagga (SEQ ID NO: 2) shown in Table 1 is the base sequence for SEQ ID NO: 278 in Table 2 with SEQ ID NO: 278 shown with their chemical modifications. Sequences disclosed herein may be assigned identifiers. For example, a single-stranded sense sequence may be identified with a "Sense strand SS#"; a single stranded antisense sequence may be identified with an "Antisense strand AS#" and a duplex that includes a sense strand and an antisense strand may be identified with a "Duplex AV#" or a "Duplex AD#".

Table 1 discloses sense and antisense strand sequences and provides the identification number of duplexes formed from the sense and antisense strand on the same line in Table 1. As used herein, the term "matching position" in a sense and an antisense strands are the positions in each strand that "pair" when the two strands are duplexed strands. For example, in a 21 nucleobase sense strand and a 21 nucleobase antisense strand, nucleobase in position 1 of the sense strand and the nucleobase in position 21 in the antisense strand are in "matching positions". In yet another non-limiting example in a 23 nucleobase sense strand and a 23 nucleobase antisense strand, nucleobase 2 of the sense strand and the nucleobase in position 22 of the antisense strand are in matching positions. In another non-limiting example, in an 18 nucleobase sense strand and an 18 nucleobase antisense strand, the nucleobase in position 1 of the sense strand and the nucleobase in position 18 in the antisense strand are in matching positions, and the nucleobase in position 4 in the sense strand and the nucleobase in position 15 in the antisense strand are in matching positions. A skilled artisan will understand how to identify matching positions in sense and antisense strands that are or will be duplexed strands and paired strands.

The first column in Table 1 indicates the Duplex AV# for a duplex that includes the sense and antisense sequences in the same table row. For example, Table 1 discloses the duplex assigned Duplex AV# AV00053, which includes sense strand SEQ ID NO: 1 and antisense strand SEQ ID NO: 139. Thus, each row in Table 1 discloses a duplex of the invention, each comprising the sense and antisense sequences shown in the same row.

In some embodiments of methods of the invention, an RNAi agent comprising a double stranded sequence disclosed in Table 1 is administered to a subject. In some embodiments of the invention an RNAi agent administered to a subject comprises a duplex comprising at least one of the strand sequences set forth in Table 1, that comprises 0, 1, 2, 3, 4, 5, 6, 7, 8, 9, 10, 11, 12, 13, 14, 15, 16, 17, 18, 19, 20, 21, 22, 23, or 24 sequence modifications. In some embodiments of methods of the invention an RNAi agent comprising a dsRNA polynucleotide sequence shown in Table 1 is attached to a delivery molecule, also referred to herein as a delivery compound. A non-limiting example of a delivery compound is a delivery compound comprising a GalNAc compound.

**Table 1: Unmodified HBV RNAi agent sense strand and antisense strand sequences. All sequences shown 5' to 3' direction. Duplex AV#s are the number assigned to the duplex of the two strands in the same row in the table.**

| Duplex ID | Sense Strand | SEQ ID NO | Antisense Strand | SE Q ID NO |
|---|---|---|---|---|
| AV00053 | ugguggacuucucucaauuua | 1 | uaaauugagagaaguccacca | 139 |
| AV00054 | acuucucucaauuuucuagga | 2 | uccuagaaaauugagagaagu | 140 |
| AV00055 | guuaccaauuuucuuuuguca | 3 | ugacaaaagaaaauugguaac | 141 |
| AV00056 | cauaagaggacucuuggacua | 4 | uaguccaagaguccucuuaug | 142 |
| AV00057 | gggguuuuucuuguugacaaa | 5 | uuugucaacaagaaaaacccc | 143 |
| AV00058 | uggacuucucucaauuuucua | 6 | uagaaaauugagagaagucca | 144 |
| AV00059 | cugcuaugccucaucuucuua | 7 | uaagaagaugaggcauagcag | 145 |
| AV00060 | cauuuguucagugguucguaa | 8 | uuacgaaccacugaacaaaug | 146 |
| AV00061 | gggcgcaccucucuuuacgca | 9 | ugcguaaagagaggugcgccc | 147 |
| AV00062 | uagacucgugguggacuucua | 10 | uagaaguccaccacgagucua | 148 |
| AV00063 | ggacuucucucaauuuucuaa | 11 | uuagaaaauugagagaagucc | 149 |
| AV00064 | uuggcucaguuuacuagugca | 12 | ugcacuaguaaacugagccaa | 150 |
| AV00065 | cuagugccauuuguucaguga | 13 | ucacugaacaaauggcacuag | 151 |
| AV00066 | uguuaccaauuuucuuuugua | 14 | uacaaaagaaaauugguaaca | 152 |
| AV00067 | uucgcuucaccucugcacgua | 15 | uacgugcagaggugaagcgaa | 153 |
| AV00068 | uaggaggcuguaggcauaaaa | 16 | uuuuaugccuacagccuccua | 154 |
| AV00069 | aggcuguaggcauaaauugga | 17 | uccaauuuaugccuacagccu | 155 |
| AV00070 | cuucucucaauuuucuaggga | 18 | ucccuagaaaauugagagaag | 156 |
| AV00071 | cuguuaccaauuuucuuuuga | 19 | ucaaaagaaaauugguaacag | 157 |
| AV00072 | ccgucugugccuucucaucua | 20 | uagaugagaaggcacagacgg | 158 |
| AV00073 | uacauaagaggacucuuggaa | 21 | uuccaagaguccucuuaugua | 159 |
| AV00074 | uacaggcgggguuuuucuuga | 22 | ucaagaaaaaccccgccugua | 160 |
| AV00075 | caggcgggguuuuucuuguua | 23 | uaacaagaaaaaccccgccug | 161 |
| AV00076 | cgggguuuuucuuguugacaa | 24 | uugucaacaagaaaaaccccg | 162 |
| AV00077 | cucgugguggacuucucucaa | 25 | uugagagaaguccaccacgag | 163 |
| AV00078 | cgugguggacuucucucaaua | 26 | uauugagagaaguccaccacg | 164 |
| AV00079 | gugguggacuucucucaauua | 27 | uaauugagagaaguccaccac | 165 |
| AV00080 | uuuacuagugccauuuguuca | 28 | ugaacaaauggcacuaguaaa | 166 |
| AV00081 | agugccauuuguucaguggua | 29 | uaccacugaacaaauggcacu | 167 |
| AV00082 | uuaccaauuuucuuuugucua | 30 | uagacaaaagaaaauugguaa | 168 |
| AV00083 | aggccuauugauuggaaagua | 31 | uacuuuccaaucaauaggccu | 169 |
| AV00084 | ccgauccauacugcggaacua | 32 | uaguuccgcaguauggaucgg | 170 |
| AV00085 | gugugcacuucgcuucaccua | 33 | uaggugaagcgaagugcacac | 171 |
| AV00086 | uuacauaagaggacucuugga | 34 | uccaagaguccucuuauguaa | 172 |
| AV00087 | uccugcugcuaugccucauca | 35 | ugaugaggcauagcagcagga | 173 |
| AV00088 | cugugccaaguguuugcugaa | 36 | uucagcaaacacuuggcacag | 174 |
| AV00089 | ugugccaaguguuugcugaca | 37 | ugucagcaaacacuuggcaca | 175 |
| AV00090 | cgugugcacuucgcuucacca | 38 | uggugaagcgaagugcacacg | 176 |
| AV00091 | guauaagaggacucuuggaca | 39 | uguccaagaguccucuuauac | 177 |
| AV00092 | gucuagacucgugguggacua | 40 | uaguccaccacgagucuagac | 178 |
| AV00093 | ucuagacucgugguggacuua | 41 | uaaguccaccacgagucuaga | 179 |
| AV00094 | auccugcugcuaugccucaua | 42 | uaugaggcauagcagcaggau | 180 |
| AV00095 | aagguauguugcccguuugua | 43 | uacaaacgggcaacauaccuu | 181 |
| AV00096 | aguuuacuagugccauuugua | 44 | uacaaauggcacuaguaaacu | 182 |
| AV00097 | ccauuuguucagugguucgua | 45 | uacgaaccacugaacaaaugg | 183 |
| AV00098 | cagcgcaugcguggaaccuua | 46 | uaagguuccacgcaugcgcug | 184 |
| AV00099 | augucaacgaccgaccuugaa | 47 | uucaaggucggucguugacau | 185 |
| AV00100 | guuacaggcgggguuuuucua | 48 | uagaaaaaccccgccuguaac | 186 |
| AV00101 | gcagagucuagacucguggua | 49 | uaccacgagucuagacucugc | 187 |
| AV00102 | gagucuagacucgugguggaa | 50 | uuccaccacgagucuagacuc | 188 |
| AV00103 | cuucauccugcugcuaugcca | 51 | uggcauagcagcaggaugaag | 189 |
| AV00104 | ccugcugcuaugccucaucua | 52 | uagaugaggcauagcagcagg | 190 |
| AV00105 | agguauguugcccguuuguca | 53 | ugacaaacgggcaacauaccu | 191 |
| AV00106 | gugccauuuguucagugguua | 54 | uaaccacugaacaaauggcac | 192 |
| AV00107 | cggggcgcaccucucuuuaca | 55 | uguaaagagaggugcgccccg | 193 |
| AV00108 | uuacaggcgggguuuuucuua | 56 | uaagaaaaaccccgccuguaa | 194 |
| AV00109 | acaggcgggguuuuucuugua | 57 | uacaagaaaaaccccgccugu | 195 |
| AV00110 | ucaagguauguugcccguuua | 58 | uaaacgggcaacauaccuuga | 196 |
| AV00111 | cauacugcggaacuccuagca | 59 | ugcuaggaguuccgcaguaug | 197 |
| AV00112 | gaccacggggcgcaccucuca | 60 | ugagaggugcgccccgugguc | 198 |
| AV00113 | ugucaacgaccgaccuugaga | 61 | ucucaaggucggucguugaca | 199 |
| AV00114 | uucauccugcugcuaugccua | 62 | uaggcauagcagcaggaugaa | 200 |
| AV00115 | gcuccucugccgauccauaca | 63 | uguauggaucggcagaggagc | 201 |
| AV00116 | cuccucugccgauccauacua | 64 | uaguauggaucggcagaggag | 202 |
| AV00117 | ccucugccgauccauacugca | 65 | ugcaguauggaucggcagagg | 203 |
| AV00118 | cgauccauacugcggaacuca | 66 | ugaguuccgcaguauggaucg | 204 |
| AV00119 | ggcgcaccucucuuuacgcga | 67 | ucgcguaaagagaggugcgcc | 205 |
| AV00120 | cccgucugugccuucucauca | 68 | ugaugagaaggcacagacggg | 206 |
| AV00121 | ucaacgaccgaccuugaggca | 69 | ugccucaaggucggucguuga | 207 |
| AV00053 -1 | guggacuucucucaauuua | 70 | uaaauugagagaaguccac | 208 |
| AV00054 -1 | uucucucaauuuucuagga | 71 | uccuagaaaauugagagaa | 209 |
| AV00055 -1 | uaccaauuuucuuuuguca | 72 | ugacaaaagaaaauuggua | 210 |
| AV00056 -1 | uaagaggacucuuggacua | 73 | uaguccaagaguccucuua | 211 |
| AV00057 -1 | gguuuuucuuguugacaaa | 74 | uuugucaacaagaaaaacc | 212 |
| AV00058 -1 | gacuucucucaauuuucua | 75 | uagaaaauugagagaaguc | 213 |
| AV00059 -1 | gcuaugccucaucuucuua | 76 | uaagaagaugaggcauagc | 214 |
| AV00060 -1 | uuuguucagugguucguaa | 77 | uuacgaaccacugaacaaa | 215 |
| AV00061 -1 | gcgcaccucucuuuacgca | 78 | ugcguaaagagaggugcgc | 216 |
| AV00062 -1 | gacucgugguggacuucua | 79 | uagaaguccaccacgaguc | 217 |
| AV00063 -1 | acuucucucaauuuucuaa | 80 | uuagaaaauugagagaagu | 218 |
| AV00064 -1 | ggcucaguuuacuagugca | 81 | ugcacuaguaaacugagcc | 219 |
| AV00065 -1 | agugccauuuguucaguga | 82 | ucacugaacaaauggcacu | 220 |
| AV00066 -1 | uuaccaauuuucuuuugua | 83 | uacaaaagaaaauugguaa | 221 |
| AV00067 -1 | cgcuucaccucugcacgua | 84 | uacgugcagaggugaagcg | 222 |
| AV00068 -1 | ggaggcuguaggcauaaaa | 85 | uuuuaugccuacagccucc | 223 |
| AV00069 -1 | gcuguaggcauaaauugga | 86 | uccaauuuaugccuacagc | 224 |
| AV00070 -1 | ucucucaauuuucuaggga | 87 | ucccuagaaaauugagaga | 225 |
| AV00071 -1 | guuaccaauuuucuuuuga | 88 | ucaaaagaaaauugguaac | 226 |
| AV00072 -1 | gucugugccuucucaucua | 89 | uagaugagaaggcacagac | 227 |
| AV00073 -1 | cauaagaggacucuuggaa | 90 | uuccaagaguccucuuaug | 228 |
| AV00074 -1 | caggcgggguuuuucuuga | 91 | ucaagaaaaaccccgccug | 229 |
| AV00075 -1 | ggcgggguuuuucuuguua | 92 | uaacaagaaaaaccccgcc | 230 |
| AV00076 -1 | ggguuuuucuuguugacaa | 93 | uugucaacaagaaaaaccc | 231 |
| AV00077 -1 | cgugguggacuucucucaa | 94 | uugagagaaguccaccacg | 232 |
| AV00078 -1 | ugguggacuucucucaaua | 95 | uauugagagaaguccacca | 233 |
| AV00079 -1 | gguggacuucucucaauua | 96 | uaauugagagaaguccacc | 234 |
| AV00080 -1 | uacuagugccauuuguuca | 97 | ugaacaaauggcacuagua | 235 |
| AV00081 -1 | ugccauuuguucaguggua | 98 | uaccacugaacaaauggca | 236 |
| AV00082 -1 | accaauuuucuuuugucua | 99 | uagacaaaagaaaauuggu | 237 |
| AV00083 -1 | gccuauugauuggaaagua | 100 | uacuuuccaaucaauaggc | 238 |
| AV00084 -1 | gauccauacugcggaacua | 101 | uaguuccgcaguauggauc | 239 |
| AV00085 -1 | gugcacuucgcuucaccua | 102 | uaggugaagcgaagugcac | 240 |
| AV00086 -1 | acauaagaggacucuugga | 103 | uccaagaguccucuuaugu | 241 |
| AV00087 -1 | cugcugcuaugccucauca | 104 | ugaugaggcauagcagcag | 242 |
| AV00088 -1 | gugccaaguguuugcugaa | 105 | uucagcaaacacuuggcac | 243 |
| AV00089 -1 | ugccaaguguuugcugaca | 106 | ugucagcaaacacuuggca | 244 |
| AV00090 -1 | ugugcacuucgcuucacca | 107 | uggugaagcgaagugcaca | 245 |
| AV00091 -1 | auaagaggacucuuggaca | 108 | uguccaagaguccucuuau | 246 |
| AV00092 -1 | cuagacucgugguggacua | 109 | uaguccaccacgagucuag | 247 |
| AV00093 -1 | uagacucgugguggacuua | 110 | uaaguccaccacgagucua | 248 |
| AV00094 -1 | ccugcugcuaugccucaua | 111 | uaugaggcauagcagcagg | 249 |
| AV00095 -1 | gguauguugcccguuugua | 112 | uacaaacgggcaacauacc | 250 |
| AV00096 -1 | uuuacuagugccauuugua | 113 | uacaaauggcacuaguaaa | 251 |
| AV00097 -1 | auuuguucagugguucgua | 114 | uacgaaccacugaacaaau | 252 |
| AV00098 -1 | gcgcaugcguggaaccuua | 115 | uaagguuccacgcaugcgc | 253 |
| AV00099 -1 | gucaacgaccgaccuugaa | 116 | uucaaggucggucguugac | 254 |
| AV00100 -1 | uacaggcgggguuuuucua | 117 | uagaaaaaccccgccugua | 255 |
| AV00101 -1 | agagucuagacucguggua | 118 | uaccacgagucuagacucu | 256 |
| AV00102 -1 | gucuagacucgugguggaa | 119 | uuccaccacgagucuagac | 257 |
| AV00103 -1 | ucauccugcugcuaugcca | 120 | uggcauagcagcaggauga | 258 |
| AV00104 -1 | ugcugcuaugccucaucua | 121 | uagaugaggcauagcagca | 259 |
| AV00105 -1 | guauguugcccguuuguca | 122 | ugacaaacgggcaacauac | 260 |
| AV00106 -1 | gccauuuguucagugguua | 123 | uaaccacugaacaaauggc | 261 |
| AV00107 -1 | gggcgcaccucucuuuaca | 124 | uguaaagagaggugcgccc | 262 |
| AV00108 -1 | acaggcgggguuuuucuua | 125 | uaagaaaaaccccgccugu | 263 |
| AV00109 -1 | aggcgggguuuuucuugua | 126 | uacaagaaaaaccccgccu | 264 |
| AV00110 -1 | aagguauguugcccguuua | 127 | uaaacgggcaacauaccuu | 265 |
| AV00111 -1 | uacugcggaacuccuagca | 128 | ugcuaggaguuccgcagua | 266 |
| AV00112 -1 | ccacggggcgcaccucuca | 129 | ugagaggugcgccccgugg | 267 |
| AV00113 -1 | ucaacgaccgaccuugaga | 130 | ucucaaggucggucguuga | 268 |
| AV00114 -1 | cauccugcugcuaugccua | 131 | uaggcauagcagcaggaug | 269 |
| AV00115 -1 | uccucugccgauccauaca | 132 | uguauggaucggcagagga | 270 |
| AV00116 -1 | ccucugccgauccauacua | 133 | uaguauggaucggcagagg | 271 |
| AV00117 -1 | ucugccgauccauacugca | 134 | ugcaguauggaucggcaga | 272 |
| AV00118 -1 | auccauacugcggaacuca | 135 | ugaguuccgcaguauggau | 273 |
| AV00119 -1 | cgcaccucucuuuacgcga | 136 | ucgcguaaagagaggugcg | 274 |
| AV00120 -1 | cgucugugccuucucauca | 137 | ugaugagaaggcacagacg | 275 |
| AV00121 -1 | aacgaccgaccuugaggca | 138 | ugccucaaggucggucguu | 276 |
| AV01258 | cggguuuuucuuguugacaaa | 674 | uuugucaacaagaaaaacccg | 735 |
| AV01259 | cggguuuuucuuguugacaau | 675 | auugucaacaagaaaaacccg | 736 |
| AV01260 | cggguuuuucuuguugacaac | 676 | guugucaacaagaaaaacccg | 737 |
| AV01261 | cggguuuuucuuguugacaag | 677 | cuugucaacaagaaaaacccg | 738 |
| AV01262 | uuuuucuuguugacaaa | 678 | uuugucaacaagaaaaa | 739 |
| AV01263 | guuuuucuuguugacaaa | 679 | uuugucaacaagaaaaac | 740 |
| AV01264 | aguuuuucuuguugacaaa | 680 | uuugucaacaagaaaaacu | 741 |
| AV01265 | uguuuuucuuguugacaaa | 681 | uuugucaacaagaaaaaca | 742 |
| AV01266 | gguuuuucuuguugacaaa | 682 | uuugucaacaagaaaaacc | 743 |
| AV01267 | cguuuuucuuguugacaaa | 683 | uuugucaacaagaaaaacg | 744 |
| AV01268 | agguuuuucuuguugacaaa | 684 | uuugucaacaagaaaaaccu | 745 |
| AV01269 | ugguuuuucuuguugacaaa | 685 | uuugucaacaagaaaaacca | 746 |
| AV01270 | ggguuuuucuuguugacaaa | 686 | uuugucaacaagaaaaaccc | 747 |
| AV01271 | cgguuuuucuuguugacaaa | 687 | uuugucaacaagaaaaaccg | 748 |
| AV01273 | aggguuuuucuuguugacaaa | 688 | uuugucaacaagaaaaacccu | 749 |
| AV01274 | uggguuuuucuuguugacaaa | 689 | uuugucaacaagaaaaaccca | 750 |
| AV01275 | cgggguuuuucuuguugacaaa | 690 | uuugucaacaagaaaaaccccg | 751 |
| AV01276 | gcgggguuuuucuuguugacaaa | 691 | uuugucaacaagaaaaaccccgc | 752 |
| AV01277 | ggcgggguuuuucuuguugacaaa | 692 | uuugucaacaagaaaaaccccgcc | 753 |
| AV01278 | aggcgggguuuuucuuguugacaaa | 693 | uuugucaacaagaaaaaccccgccu | 754 |
| AV01279 | caggcgggguuuuucuuguugacaaa | 694 | uuugucaacaagaaaaaccccgccug | 755 |
| AV01280 | guguuacaggcggguuuuucuuguugacaa a | 695 | uuugucaacaagaaaaaccccgccuguaaca c | 756 |
| AV01281 | cggguuuuucuuguugacaaa | 696 | uuugucaacaagaaaaacccguu | 757 |
| AV01282 | cggguuuuucuuguugacaaa | 697 | uuugucaacaagaaaaacccgga | 758 |
| AV01283 | cggguuuuucuuguugacaaa | 698 | uuugucaacaagaaaaacccg | 759 |
| AV01284 | cggguuuuucuuguugacaaa | 699 | uuugucaacaagaaaaacccg | 760 |
| AV01285 | cggguuuuucuuguugacaaa | 700 | uuugucaacaagaaaaacccg | 761 |
| AV01286 | cggguuuuucuuguuaacaaa | 701 | uuugucaacaagaaaaacccg | 762 |
| AV01287 | cggguuuuucuuguugacaga | 702 | ucugucaacaagaaaaacccg | 763 |
| AV01288 | cggguuuuucuuguugacaaa | 703 | uuugucaacaagaaaaacccg | 764 |
| AV01289 | caggguuuuucuuguugacau | 704 | augucaacaagaaaaacccug | 765 |
| AV01290 | caggguuuuucuuguugacaa | 705 | uugucaacaagaaaaacccug | 766 |
| AV01291 | caggguuuuucuuguugacac | 706 | gugucaacaagaaaaacccug | 767 |
| AV01292 | caggguuuuucuuguugacag | 707 | cugucaacaagaaaaacccug | 768 |
| AV01293 | guuuuucuuguugacau | 708 | augucaacaagaaaaac | 769 |
| AV01294 | gguuuuucuuguugacau | 709 | augucaacaagaaaaacc | 770 |
| AV01295 | agguuuuucuuguugacau | 710 | augucaacaagaaaaaccu | 771 |
| AV01296 | ugguuuuucuuguugacau | 711 | augucaacaagaaaaacca | 772 |
| AV01297 | ggguuuuucuuguugacau | 712 | augucaacaagaaaaaccc | 773 |
| AV01298 | cgguuuuucuuguugacau | 713 | augucaacaagaaaaaccg | 774 |
| AV01299 | aggguuuuucuuguugacau | 714 | augucaacaagaaaaacccu | 775 |
| AV01300 | uggguuuuucuuguugacau | 715 | augucaacaagaaaaaccca | 776 |
| AV01301 | gggguuuuucuuguugacau | 716 | augucaacaagaaaaacccc | 777 |
| AV01302 | cggguuuuucuuguugacau | 717 | augucaacaagaaaaacccg | 778 |
| AV01303 | gaggguuuuucuuguugacau | 718 | augucaacaagaaaaacccuc | 779 |
| AV01304 | aaggguuuuucuuguugacau | 719 | augucaacaagaaaaacccuu | 780 |
| AV01305 | uaggguuuuucuuguugacau | 720 | augucaacaagaaaaacccua | 781 |
| AV01306 | gcgggguuuuucuuguugacau | 721 | augucaacaagaaaaaccccgc | 782 |
| AV01307 | ggcgggguuuuucuuguugacau | 722 | augucaacaagaaaaaccccgcc | 783 |
| AV01308 | aggcgggguuuuucuuguugacau | 723 | augucaacaagaaaaaccccgccu | 784 |
| AV01309 | caggcgggguuuuucuuguugacau | 724 | augucaacaagaaaaaccccgccug | 785 |
| AV01310 | acaggcgggguuuuucuuguugacau | 725 | augucaacaagaaaaaccccgccugu | 786 |
| AV01311 | cguguuacaggcggguuuuucuuguugaca u | 726 | augucaacaagaaaaaccccgccuguaacacg | 787 |
| AV01312 | caggguuuuucuuguugacau | 727 | augucaacaagaaaaacccuguu | 788 |
| AV01313 | caggguuuuucuuguugacau | 728 | augucaacaagaaaaacccugga | 789 |
| AV01314 | caggguuuuucuuguugacau | 729 | augucaacaagaaaaacccug | 790 |
| AV01315 | caggguuuuucuuguugacau | 730 | augucaacaagaaaaacccug | 791 |
| AV01316 | caggguuuuucuuguugacau | 731 | augucaacaagaaaaacccug | 792 |
| AV01317 | caggguuuuucuugucgacau | 732 | augucaacaagaaaaacccug | 793 |
| AV01318 | caggguuuuucuuguugacgu | 733 | acgucaacaagaaaaacccug | 794 |
| AV01319 | caggguuuuucuuguugacau | 734 | augucaacaagaaaaacccug | 795 |

Table 2 shows certain chemically modified HBV RNAi agent sense strand and antisense strand sequences of the invention. In some embodiments of methods of the invention, an RNAi agent with a polynucleotide sequence shown in Table 2 is administered to a cell and/or subject. In some embodiments of the invention an RNAi agent administered to a subject comprises is a duplex identified in a row in Table 2, column one and includes the sequence modifications shown in the sense and antisense strand sequences in the same row in Table 2, columns four and seven, respectively. In some embodiments of methods of the invention, a sequence shown in Table 2 may be attached to (also referred to herein as "conjugated to") a compound capable of delivering the RNAi agent to a cell and/or tissue in a subject. A non-limiting example of a delivery compound that may be used in certain embodiments of the invention is a GalNAc-containing compound. In Table 2, the first column indicates the Duplex AV# of the base sequences as shown in Table 1. Table 2 discloses Duplex AV# and also shows chemical modifications included in sense and antisense sequence of the duplexes. For example, Table 1 shows base single-strand sequences SEQ ID NO: 1 (sense) and SEQ ID NO: 139 (antisense), which together are the double-stranded duplex identified as: Duplex AV# AV00053. Table 2 lists Duplex AV# AV00053, and shows the duplex of SEQ ID NO: 277 and SEQ ID NO: 415 includes base sequences SEQ ID NO: 1 and SEQ ID NO: 139, respectively, but with the chemical modifications shown in the sense and antisense sequences shown in columns four and seven, respectively. The "Sense strand SS#" in Table 2 column two is the assigned identifier for the Sense Sequence (including modifications) shown column four in the same row. The "Antisense strand AS#" in Table 2 column five is the assigned identifier for the Antisense sequence (including modifications) shown in column seven.

**Table 2: provides chemically modified HBV RNAi agent sense strand and antisense strand sequences. All sequences shown 5' to 3'. These sequences were used in certain in vitro testing studies described herein. The chemical modifications are indicated by: Upper case: 2'-Fluoro; lower case: 2'-OMe; thiophosphate: ***

| Duplex ID | Sense strand SS# | SEQ ID NO | Sense Strand | Antisense strand AS# | SEQ ID NO | Antisense Strand |
|---|---|---|---|---|---|---|
| AV00053 | AV00053-SS | 277 | u*g*guggacUuCuCucaauuu*a | AV00053-AS | 415 | u*A*aauuGagagAaGuCcac*c*a |
| AV00054 | AV00054-SS | 278 | a*c*uucucuCaAuUuucuagg*a | AV00054-AS | 416 | u*C*cuagAaaauUgAgAgaa*g*u |
| AV00055 | AV00055-SS | 279 | g*u*uaccaaUuUuCuuuuguc*a | AV00055-AS | 417 | u*G*acaaAagaaAaUuGgua*a*c |
| AV00056 | AV00056-SS | 280 | c*a*uaagagGaCuCuuggacu*a | AV00056-AS | 418 | u*A*guccAagagUcCuCuua*u*g |
| AV00057 | AV00057-SS | 281 | g*g*gguuuuUcUuGuugacaa*a | AV00057-AS | 419 | u*U*ugucAacaaGaAaAacc*c*c |
| AV00058 | AV00058-SS | 282 | u*g*gacuucUcUcAauuuucu*a | AV00058-AS | 420 | u*A*gaaaAuugaGaGaAguc*c*a |
| AV00059 | AV00059-SS | 283 | c*u*gcuaugCcUcAucuucuu*a | AV00059-AS | 421 | u*A*agaaGaugaGgCaUagc*a*g |
| AV00060 | AV00060-SS | 284 | c*a*uuuguuCaGuGguucgua*a | AV00060-AS | 422 | u*U*acgaAccacUgAaCaaa*u*g |
| AV00061 | AV00061-SS | 285 | g*g*gcgcacCuCuCuuuacgc*a | AV00061-AS | 423 | u*G*cguaAagagAgGuGcgc*c*c |
| AV00062 | AV00062-SS | 286 | u*a*gacucgUgGuGgacuucu*a | AV00062-AS | 424 | u*A*gaagUccacCaCgAguc*u*a |
| AV00063 | AV00063-SS | 287 | g*g*acuucuCuCaAuuuucua*a | AV00063-AS | 425 | u * U * agaaAa u ugAgAgAagu * c * c |
| AV00064 | AV00064-SS | 288 | u*u*ggcucaGuUuAcuagugc*a | AV00064-AS | 426 | u*G*cacuAguaaAcUgAgcc*a*a |
| AV00065 | AV00065-SS | 289 | c*u*agugccAuUuGuucagug*a | AV00065-AS | 427 | u*C*acugAacaaAuGgCacu*a*g |
| AV00066 | AV00066-SS | 290 | u*g*uuaccaAuUuUcuuuugu*a | AV00066-AS | 428 | u*A*caaaAgaaaAuUgGuaa*c*a |
| AV00067 | AV00067-SS | 291 | u*u*cgcuucAcCuCugcacgu*a | AV00067-AS | 429 | u*A*cgugCagagGuGaAgcg*a*a |
| AV00068 | AV00068-SS | 292 | u*a*ggaggcUgUaGgcauaaa*a | AV00068-AS | 430 | u*U*uuauGccuaCaGcCucc*u*a |
| AV00069 | AV00069-SS | 293 | a*g*gcuguaGgCaUaaauugg*a | AV00069-AS | 431 | u*C*caauUuaugCcUaCagc*c*u |
| AV00070 | AV00070-SS | 294 | c*u*ucucucAaUuUucuaggg*a | AV00070-AS | 432 | u*C*ccuaGaaaaUuGaGaga*a*g |
| AV00071 | AV00071-SS | 295 | c*u*guuaccAaUuUucuuuug*a | AV00071-AS | 433 | u*C*aaaaGaaaaUuGgUaac*a*g |
| AV00072 | AV00072-SS | 296 | c*c*gucuguGcCuUcucaucu*a | AV00072-AS | 434 | u*A*gaugAgaagGcAcAgac*g*g |
| AV00073 | AV00073-SS | 297 | u*a*cauaagAgGaCucuugga*a | AV00073-AS | 435 | u*U*ccaaGagucCuCuUaug*u*a |
| AV00074 | AV00074-SS | 298 | u*a*caggcgGgGuUuuucuug*a | AV00074-AS | 436 | u*C*aagaAaaacCcCgCcug*u*a |
| AV00075 | AV00075-SS | 299 | c*a*ggcgggGuUuUucuuguu*a | AV00075-AS | 437 | u*A*acaaGaaaaAcCcCgcc*u*g |
| AV00076 | AV00076-SS | 300 | c*g*ggguuuUuCuUguugaca*a | AV00076-AS | 438 | u*U*gucaAcaagAaAaAccc*c*g |
| AV00077 | AV00077-SS | 301 | c*u*cgugguGgAcUucucuca*a | AV00077-AS | 439 | u*U*gagaGaaguCcAcCacg*a*g |
| AV00078 | AV00078-SS | 302 | c*g*ugguggAcUuCucucaau*a | AV00078-AS | 440 | u*A*uugaGagaaGuCcAcca*c*g |
| AV00079 | AV00079-SS | 303 | g*u*gguggaCuUcUcucaauu*a | AV00079-AS | 441 | u*A*auugAgagaAgUcCacc*a*c |
| AV00080 | AV00080-SS | 304 | u*u*uacuagUgCcAuuuguuc*a | AV00080-AS | 442 | u*G*aacaAauggCaCuAgua*a*a |
| AV00081 | AV00081-SS | 305 | a*g*ugccauUuGuUcaguggu*a | AV00081-AS | 443 | u*A*ccacUgaacAaAuGgca*c*u |
| AV00082 | AV00082-SS | 306 | u*u*accaauUuUcUuuugucu*a | AV00082-AS | 444 | u*A*gacaAaagaAaAuUggu*a*a |
| AV00083 | AV00083-SS | 307 | a*g*gccuauUgAuUggaaagu*a | AV00083-AS | 445 | u*A*cuuuCcaauCaAuAggc*c*u |
| AV00084 | AV00084-SS | 308 | c*c*gauccaUaCuGcggaacu*a | AV00084-AS | 446 | u*A*guucCgcagUaUgGauc*g*g |
| AV00085 | AV00085-SS | 309 | g*u*gugcacUuCgCuucaccu*a | AV00085-AS | 447 | u*A*ggugAagcgAaGuGcac*a*c |
| AV00086 | AV00086-SS | 310 | u*u*acauaaGaGgAcucuugg*a | AV00086-AS | 448 | u*C*caagAguccUcUuAugu*a*a |
| AV00087 | AV00087-SS | 311 | u*c*cugcugCuAuGccucauc*a | AV00087-AS | 449 | u*G*augaGgcauAgCaGcag*g*a |
| AV00088 | AV00088-SS | 312 | c*u*gugccaAgUgUuugcuga*a | AV00088-AS | 450 | u * U *cagcAaacaCu UgGcac*a *g |
| AV00089 | AV00089-SS | 313 | u*g*ugccaaGuGuUugcugac*a | AV00089-AS | 451 | u*G*ucagCaaacAcUuGgca*c*a |
| AV00090 | AV00090-SS | 314 | c*g*ugugcaCuUcGcuucacc*a | AV00090-AS | 452 | u*G*gugaAgcgaAgUgCaca*c*g |
| AV00091 | AV00091-SS | 315 | g*u*auaagaGgAcUcuuggac*a | AV00091-AS | 453 | u*G*uccaAgaguCcUcUuau*a*c |
| AV00092 | AV00092-SS | 316 | g*u*cuagacUcGuGguggacu*a | AV00092-AS | 454 | u*A*guccAccacGaGuCuag*a*c |
| AV00093 | AV00093-SS | 317 | u*c*uagacuCgUgGuggacuu*a | AV00093-AS | 455 | u*A*agucCaccaCgAgUcua*g*a |
| AV00094 | AV00094-SS | 318 | a*u*ccugcuGcUaUgccucau*a | AV00094-AS | 456 | u*A*ugagGcauaGcAgCagg*a*u |
| AV00095 | AV00095-SS | 319 | a*a*gguaugUuGcCcguuugu*a | AV00095-AS | 457 | u*A*caaaCgggcAaCaUacc*u*u |
| AV00096 | AV00096-SS | 320 | a*g*uuuacuAgUgCcauuugu*a | AV00096-AS | 458 | u*A*caaaUggcaCuAgUaaa*c*u |
| AV00097 | AV00097-SS | 321 | c*c*auuuguUcAgUgguucgu*a | AV00097-AS | 459 | u*A*cgaaCcacuGaAcAaau*g*g |
| AV00098 | AV00098-SS | 322 | c*a*gcgcauGcGuGgaaccuu*a | AV00098-AS | 460 | u*A*agguUccacGcAuGcgc*u*g |
| AV00099 | AV00099-SS | 323 | a*u*gucaacGaCcGaccuuga*a | AV00099-AS | 461 | u * U *caagGucggUcGu Ugac* a* u |
| AV00100 | AV00100-SS | 324 | g*u*uacaggCgGgGuuuuucu*a | AV00100-AS | 462 | u*A*gaaaAacccCgCcUgua*a*c |
| AV00101 | AV00101-SS | 325 | g*c*agagucUaGaCucguggu*a | AV00101-AS | 463 | u*A*ccacGagucUaGaCucu*g*c |
| AV00102 | AV00102-SS | 326 | g*a*gucuagAcUcGuggugga*a | AV00102-AS | 464 | u * U *ccacCacgaGuCuAgac* u *c |
| AV00103 | AV00103-SS | 327 | c*u*ucauccUgCuGcuaugcc*a | AV00103-AS | 465 | u*G*gcauAgcagCaGgAuga*a*g |
| AV00104 | AV00104-SS | 328 | c*c*ugcugcUaUgCcucaucu*a | AV00104-AS | 466 | u*A*gaugAggcaUaGcAgca*g*g |
| AV00105 | AV00105-SS | 329 | a*g*guauguUgCcCguuuguc*a | AV00105-AS | 467 | u*G*acaaAcgggCaAcAuac*c*u |
| AV00106 | AV00106-SS | 330 | g*u*gccauuUgUuCagugguu*a | AV00106-AS | 468 | u*A*accaCugaaCaAaUggc*a*c |
| AV00107 | AV00107-SS | 331 | c*g*gggcgcAcCuCucuuuac*a | AV00107-AS | 469 | u*G*uaaaGagagGuGcGccc*c*g |
| AV00108 | AV00108-SS | 332 | u*u*acaggcGgGgUuuuucuu*a | AV00108-AS | 470 | u*A*agaaAaaccCcGcCugu*a*a |
| AV00109 | AV00109-SS | 333 | a*c*aggcggGgUuUuucuugu*a | AV00109-AS | 471 | u*A*caagAaaaaCcCcGccu*g*u |
| AV00110 | AV00110-SS | 334 | u*c*aagguaUgUuGcccguuu*a | AV00110-AS | 472 | u*A*aacgGgcaaCaUaCcuu*g*a |
| AV00111 | AV00111-SS | 335 | c*a*uacugcGgAaCuccuagc*a | AV00111-AS | 473 | u*G*cuagGaguuCcGcAgua*u*g |
| AV00112 | AV00112-SS | 336 | g*a*ccacggGgCgCaccucuc*a | AV00112-AS | 474 | u*G*agagGugcgCcCcGugg*u*c |
| AV00113 | AV00113-SS | 337 | u*g*ucaacgAcCgAccuugag*a | AV00113-AS | 475 | u*C*ucaaGgucgGuCgUuga*c*a |
| AV00114 | AV00114-SS | 338 | u*u*cauccuGcUgCuaugccu*a | AV00114-AS | 476 | u*A*ggcaUagcaGcAgGaug*a*a |
| AV00115 | AV00115-SS | 339 | g*c*uccucuGcCgAuccauac*a | AV00115-AS | 477 | u*G*uaugGaucgGcAgAgga*g*c |
| AV00116 | AV00116-SS | 340 | c*u*ccucugCcGaUccauacu*a | AV00116-AS | 478 | u*A*guauGgaucGgCaGagg*a*g |
| AV00117 | AV00117-SS | 341 | c*c*ucugccGaUcCauacugc*a | AV00117-AS | 479 | u*G*caguAuggaUcGgCaga*g*g |
| AV00118 | AV00118-SS | 342 | c*g*auccauAcUgCggaacuc*a | AV00118-AS | 480 | u*G*aguuCcgcaGuAuGgau*c*g |
| AV00119 | AV00119-SS | 343 | g*g*cgcaccUcUcUuuacgcg*a | AV00119-AS | 481 | u*C*gcguAaagaGaGgUgcg*c*c |
| AV00120 | AV00120-SS | 344 | c*c*cgucugUgCcUucucauc*a | AV00120-AS | 482 | u*G*augaGaaggCaCaGacg*g*g |
| AV00121 | AV00121-SS | 345 | u*c*aacgacCgAcCuugaggc*a | AV00121-AS | 483 | u*G*ccucAagguCgGuCguu*g*a |
| AV00053-1 | AV00053-1-SS | 346 | g*u*ggacUuCuCucaauuu*a | AV00053-1-AS | 484 | u*A*aauuGagagAaGuCca*c |
| AV00054-1 | AV00054-1-SS | 347 | u*u*cucuCaAuUuucuagg*a | AV00054-1-AS | 485 | u*C*cuagAaaauUgAgAga*a |
| AV00055-1 | AV00055-1-SS | 348 | u*a*ccaaUuUuCuuuuguc*a | AV00055-1-AS | 486 | u*G*acaaAagaaAaUuGgu*a |
| AV00056-1 | AV00056-1-SS | 349 | u*a*agagGaCuCuuggacu*a | AV00056-1-AS | 487 | u*A*guccAagagUcCuCuu*a |
| AV00057-1 | AV00057-1-SS | 350 | g*g*uuuuUcUuGuugacaa*a | AV00057-1-AS | 488 | u*U*ugucAacaaGaAaAac*c |
| AV00058-1 | AV00058-1-SS | 351 | g*a*cuucUcUcAauuuucu*a | AV00058-1-AS | 489 | u*A*gaaaAuugaGaGaAgu*c |
| AV00059-1 | AV00059-1-SS | 352 | g*c*uaugCcUcAucuucuu*a | AV00059-1-AS | 490 | u*A*agaaGaugaGgCaUag*c |
| AV00060-1 | AV00060-1-SS | 353 | u*u*uguuCaGuGguucgua*a | AV00060-1-AS | 491 | u*U*acgaAccacUgAaCaa*a |
| AV00061-1 | AV00061-1-SS | 354 | g*c*gcacCuCuCuuuacgc*a | AV00061-1-AS | 492 | u*G*cguaAagagAgGuGcg*c |
| AV00062-1 | AV00062-1-SS | 355 | g*a*cucgUgGuGgacuucu*a | AV00062-1-AS | 493 | u*A*gaagUccacCaCgAgu*c |
| AV00063-1 | AV00063-1-SS | 356 | a*c*uucuCuCaAuuuucua*a | AV00063-1-AS | 494 | u*U*agaaAauugAgAgAag*u |
| AV00064-1 | AV00064-1-SS | 357 | g*g*cucaGuUuAcuagugc*a | AV00064-1-AS | 495 | u*G*cacuAguaaAcUgAgc*c |
| AV00065-1 | AV00065-1-SS | 358 | a*g*ugccAuUuGuucagug*a | AV00065-1-AS | 496 | u*C*acugAacaaAuGgCac*u |
| AV00066-1 | AV00066-1-SS | 359 | u*u*accaAuUuUcuuuugu*a | AV00066-1-AS | 497 | u*A*caaaAgaaaAuUgGua*a |
| AV00067-1 | AV00067-1-SS | 360 | c*g*cuucAcCuCugcacgu*a | AV00067-1-AS | 498 | u*A*cgugCagagGuGaAgc*g |
| AV00068-1 | AV00068-1-SS | 361 | g*g*aggcUgUaGgcauaaa*a | AV00068-1-AS | 499 | u*U*uuauGccuaCaGcCuc*c |
| AV00069-1 | AV00069-1-SS | 362 | g*c*uguaGgCaUaaauugg*a | AV00069-1-AS | 500 | u*C*caauUuaugCcUaCag*c |
| AV00070-1 | AV00070-1-SS | 363 | u*c*ucucAaUuUucuaggg*a | AV00070-1-AS | 501 | u*C*ccuaGaaaaUuGaGag*a |
| AV00071-1 | AV00071-1-SS | 364 | g*u*uaccAaUuUucuuuug*a | AV00071-1-AS | 502 | u*C*aaaaGaaaaUuGgUaa*c |
| AV00072-1 | AV00072-1-SS | 365 | g*u*cuguGcCuUcucaucu*a | AV00072-1-AS | 503 | u*A*gaugAgaagGcAcAga*c |
| AV00073-1 | AV00073-1-SS | 366 | c*a*uaagAgGaCucuugga*a | AV00073-1-AS | 504 | u*U*ccaaGagucCuCuUau*g |
| AV00074-1 | AV00074-1-SS | 367 | c* a *ggcgGgGu U uuucuug* a | AV00074-1-AS | 505 | u*C*aagaAaaacCcCgCcu*g |
| AV00075-1 | AV00075-1-SS | 368 | g*g*cgggGuUuUucuuguu*a | AV00075-1-AS | 506 | u*A*acaaGaaaaAcCcCgc*c |
| AV00076-1 | AV00076-1-SS | 369 | g*g*guuuUuCuUguugaca*a | AV00076-1-AS | 507 | u * U *guca AcaagAa AaAcc*c |
| AV00077-1 | AV00077-1-SS | 370 | c*g*ugguGgAcUucucuca*a | AV00077-1-AS | 508 | u*U*gagaGaaguCcAcCac*g |
| AV00078-1 | AV00078-1-SS | 371 | u*g*guggAcUuCucucaau*a | AV00078-1-AS | 509 | u*A*uugaGagaaGuCcAcc*a |
| AV00079-1 | AV00079-1-SS | 372 | g*g*uggaCuUcUcucaauu*a | AV00079-1-AS | 510 | u*A*auugAgagaAgUcCac*c |
| AV00080-1 | AV00080-1-SS | 373 | u*a*cuagUgCcAuuuguuc*a | AV00080-1-AS | 511 | u*G*aacaAauggCaCuAgu*a |
| AV00081-1 | AV00081-1-SS | 374 | u*g*ccauUuGuUcaguggu*a | AV00081-1-AS | 512 | u*A*ccacUgaacAaAuGgc*a |
| AV00082-1 | AV00082-1-SS | 375 | a*c*caauUuUcUuuugucu*a | AV00082-1-AS | 513 | u*A*gacaAaagaAaAuUgg*u |
| AV00083-1 | AV00083-1-SS | 376 | g*c*cuauUgAuUggaaagu*a | AV00083-1-AS | 514 | u*A*cuuuCcaauCaAuAgg*c |
| AV00084-1 | AV00084-1-SS | 377 | g*a*uccaUaCuGcggaacu*a | AV00084-1-AS | 515 | u*A*guucCgcagUaUgGau*c |
| AV00085-1 | AV00085-1-SS | 378 | g*u*gcacUuCgCuucaccu*a | AV00085-1-AS | 516 | u*A*ggugAagcgAaGuGca*c |
| AV00086-1 | AV00086-1-SS | 379 | a*c*auaaGaGgAcucuugg*a | AV00086-1-AS | 517 | u*C*caagAguccUcUuAug*u |
| AV00087-1 | AV00087-1-SS | 380 | c*u*gcugCuAuGccucauc*a | AV00087-1-AS | 518 | u*G*augaGgcauAgCaGca*g |
| AV00088-1 | AV00088-1-SS | 381 | g*u*gccaAgUgUuugcuga*a | AV00088-1-AS | 519 | u * U *cagcAaacaCu UgGca *c |
| AV00089-1 | AV00089-1-SS | 382 | u*g*ccaaGuGuUugcugac*a | AV00089-1-AS | 520 | u*G*ucagCaaacAcUuGgc*a |
| AV00090-1 | AV00090-1-SS | 383 | u*g*ugcaCuUcGcuucacc*a | AV00090-1-AS | 521 | u*G*gugaAgcgaAgUgCac*a |
| AV00091-1 | AV00091-1-SS | 384 | a*u*aagaGgAcUcuuggac*a | AV00091-1-AS | 522 | u *G * uccaAgaguCcUcU ua *u |
| AV00092-1 | AV00092-1-SS | 385 | c*u*agacUcGuGguggacu*a | AV00092-1-AS | 523 | u*A*guccAccacGaGuCua*g |
| AV00093-1 | AV00093-1-SS | 386 | u*a*gacuCgUgGuggacuu*a | AV00093-1-AS | 524 | u*A*agucCaccaCgAgUcu*a |
| AV00094-1 | AV00094-1-SS | 387 | c*c*ugcuGcUaUgccucau*a | AV00094-1-AS | 525 | u*A*ugagGcauaGcAgCag*g |
| AV00095-1 | AV00095-1-SS | 388 | g*g*uaugUuGcCcguuugu*a | AV00095-1-AS | 526 | u*A*caaaCgggcAaCaUac*c |
| AV00096-1 | AV00096-1-SS | 389 | u*u*uacuAgUgCcauuugu*a | AV00096-1-AS | 527 | u*A*caaaUggcaCuAgUaa*a |
| AV00097-1 | AV00097-1-SS | 390 | a*u*uuguUcAgUgguucgu*a | AV00097-1-AS | 528 | u*A*cgaaCcacuGaAcAaa*u |
| AV00098-1 | AV00098-1-SS | 391 | g*c*gcauGcGuGgaaccuu*a | AV00098-1-AS | 529 | u*A*agguUccacGcAuGcg*c |
| AV00099-1 | AV00099-1-SS | 392 | g*u*caacGaCcGaccuuga*a | AV00099-1-AS | 530 | u * U *caagGucggUcGu Uga*c |
| AV00100-1 | AV00100-1-SS | 393 | u*a*caggCgGgGuuuuucu*a | AV00100-1-AS | 531 | u*A*gaaaAacccCgCcUgu*a |
| AV00101-1 | AV00101-1-SS | 394 | a*g*agucUaGaCucguggu*a | AV00101-1-AS | 532 | u*A*ccacGagucUaGaCuc*u |
| AV00102-1 | AV00102-1-SS | 395 | g*u*cuagAcUcGuggugga*a | AV00102-1-AS | 533 | u*U*ccacCacgaGuCuAga*c |
| AV00103-1 | AV00103-1-SS | 396 | u*c*auccUgCuGcuaugcc*a | AV00103-1-AS | 534 | u*G*gcauAgcagCaGgAug*a |
| AV00104-1 | AV00104-1-SS | 397 | u*g*cugcUaUgCcucaucu*a | AV00104-1-AS | 535 | u*A*gaugAggcaUaGcAgc*a |
| AV00105-1 | AV00105-1-SS | 398 | g*u*auguUgCcCguuuguc*a | AV00105-1-AS | 536 | u*G*acaaAcgggCaAcAua*c |
| AV00106-1 | AV00106-1-SS | 399 | g*c*cauuUgUuCagugguu*a | AV00106-1-AS | 537 | u*A*accaCugaaCaAaUgg*c |
| AV00107-1 | AV00107-1-SS | 400 | g*g*gcgcAcCuCucuuuac*a | AV00107-1-AS | 538 | u *G * uaaaGagagGuGcGcc*c |
| AV00108-1 | AV00108-1-SS | 401 | a*c*aggcGgGgUuuuucuu*a | AV00108-1-AS | 539 | u*A*agaaAaaccCcGcCug*u |
| AV00109-1 | AV00109-1-SS | 402 | a*g*gcggGgUuUuucuugu*a | AV00109-1-AS | 540 | u*A*caagAaaaaCcCcGcc*u |
| AV00110-1 | AV00110-1-SS | 403 | a*a*gguaUgUuGcccguuu*a | AV00110-1-AS | 541 | u*A*aacgGgcaaCaUaCcu*u |
| AV00111-1 | AV00111-1-SS | 404 | u*a*cugcGgAaCuccuagc*a | AV00111-1-AS | 542 | u*G*cuagGaguuCcGcAgu*a |
| AV00112-1 | AV00112-1-SS | 405 | c*c*acggGgCgCaccucuc*a | AV00112-1-AS | 543 | u*G*agagGugcgCcCcGug*g |
| AV00113-1 | AV00113-1-SS | 406 | u*c*aacgAcCgAccuugag*a | AV00113-1-AS | 544 | u*C*ucaaGgucgGuCgUug*a |
| AV00114-1 | AV00114-1-SS | 407 | c*a*uccuGcUgCuaugccu*a | AV00114-1-AS | 545 | u*A*ggcaUagcaGcAgGau*g |
| AV00115-1 | AV00115-1-SS | 408 | u*c*cucuGcCgAuccauac*a | AV00115-1-AS | 546 | u*G*uaugGaucgGcAgAgg*a |
| AV00116-1 | AV00116-1-SS | 409 | c*c*ucugCcGaUccauacu*a | AV00116-1-AS | 547 | u*A*guauGgaucGgCaGag*g |
| AV00117-1 | AV00117-1-SS | 410 | u*c*ugccGaUcCauacugc*a | AV00117-1-AS | 548 | u*G*caguAuggaUcGgCag*a |
| AV00118-1 | AV00118-1-SS | 411 | a*u*ccauAcUgCggaacuc*a | AV00118-1-AS | 549 | u*G*aguuCcgcaGuAuGga*u |
| AV00119-1 | AV00119-1-SS | 412 | c*g*caccUcUcUuuacgcg*a | AV00119-1-AS | 550 | u*C*gcguAaagaGaGgUgc*g |
| AV00120-1 | AV00120-1-SS | 413 | c*g*ucugUgCcUucucauc*a | AV00120-1-AS | 551 | u*G*augaGaaggCaCaGac*g |
| AV00121-1 | AV00121-1-SS | 414 | a*a*cgacCgAcCuugaggc*a | AV00121-1-AS | 552 | u*G*ccucAagguCgGuCgu*u |
| AV01258 | AV01258-SS | 796 | c*g*gguuuuUcUuGuugacaa*a | AV01258-AS | 857 | u*U*ugucAacaaGaAaAacc*c*g |
| AV01259 | AV01259-SS | 797 | c*g*gguuuuUcUuGuugacaa*u | AV01259-AS | 858 | a*U*ugucAacaaGaAaAacc*c*g |
| AV01260 | AV01260-SS | 798 | c*g*gguuuuUcUuGuugacaa*c | AV01260-AS | 859 | g*U*ugucAacaaGaAaAacc*c*g |
| AV01261 | AV01261-SS | 799 | c*g*gguuuuUcUuGuugacaa*g | AV01261-AS | 860 | c*U*ugucAacaaGaAaAacc*c*g |
| AV01262 | AV01262-SS | 800 | u*u*uuUcUuGuugacaa*a | AV01262-AS | 861 | u*U*ugucAacaaGaAa*A*a |
| AV01263 | AV01263-SS | 801 | g*u*uuuUcUuGuugacaa*a | AV01263-AS | 862 | u*U*ugucAacaaGaAaA*a*c |
| AV01264 | AV01264-SS | 802 | a*g*uuuuUcUuGuugacaa*a | AV01264-AS | 863 | u*U*ugucAacaaGaAaAa*c*u |
| AV01265 | AV01265-SS | 803 | u*g*uuuuUcUuGuugacaa*a | AV01265-AS | 864 | u*U*ugucAacaaGaAaAa*c*a |
| AV01266 | AV01266-SS | 804 | g*g*uuuuUcUuGuugacaa*a | AV01266-AS | 865 | u*U*ugucAacaaGaAaAa*c*c |
| AV01267 | AV01267-SS | 805 | c*g*uuuuUcUuGuugacaa*a | AV01267-AS | 866 | u*U*ugucAacaaGaAaAa*c*g |
| AV01268 | AV01268-SS | 806 | a*g*guuuuUcUuGuugacaa*a | AV01268-AS | 867 | u*U*ugucAacaaGaAaAac*c*u |
| AV01269 | AV01269-SS | 807 | u*g*guuuuUcUuGuugacaa*a | AV01269-AS | 868 | u*U*ugucAacaaGaAaAac*c*a |
| AV01270 | AV01270-SS | 808 | g*g*guuuuUcUuGuugacaa*a | AV01270-AS | 869 | u*U*ugucAacaaGaAaAac*c*c |
| AV01271 | AV01271-SS | 809 | c*g*guuuuUcUuGuugacaa*a | AV01271-AS | 870 | u*U*ugucAacaaGaAaAac*c*g |
| AV01273 | AV01273-SS | 810 | a*g*gguuuuUcUuGuugacaa*a | AV01273-AS | 871 | u*U*ugucAacaaGaAaAacc*c*u |
| AV01274 | AV01274-SS | 811 | u*g*gguuuuUcUuGuugacaa*a | AV01274-AS | 872 | u*U*ugucAacaaGaAaAacc*c*a |
| AV01275 | AV01275-SS | 812 | c*g*ggguuuuUcUuGuugacaa*a | AV01275-AS | 873 | u * U * ugucAacaaGaAaAaccc*c*g |
| AV01276 | AV01276-SS | 813 | g*c*gggguuuuUcUuGuugacaa*a | AV01276-AS | 874 | u * U * ugucAacaaGaAaAacccc*g*c |
| AV01277 | AV01277-SS | 814 | g*g*cgggguuuuUcUuGuugacaa*a | AV01277-AS | 875 | u * U * ugucAacaaGaAaAaccccg*c*c |
| AV01278 | AV01278-SS | 815 | a*g*gcgggguuuuUcUuGuugacaa*a | AV01278-AS | 876 | u * U * ugucAacaaGaAaAaccccgc*c* u |
| AV01279 | AV01279-SS | 816 | c*a*ggcgggguuuuUcUuGuugacaa*a | AV01279-AS | 877 | u * U * ugucAacaaGaAaAaccccgcc* u *g |
| AV01280 | AV01280-SS | 817 | g*u*guuacaggcgggguuuuUcUuGuugacaa*a | AV01280-AS | 878 | u*U*ugucAacaaGaAaAaccccgccuguaac*a* c |
| AV01281 | AV01281-SS | 818 | c*g*gguuuuUcUuGuugacaa*a | AV01281-AS | 879 | u * U * ugucAacaaGaAaAacccg* u * u |
| AV01282 | AV01282-SS | 819 | c*g*gguuuuUcUuGuugacaa*a | AV01282-AS | 880 | u * U * ugucAacaaGaAaAacccg*g* a |
| AV01283 | AV01283-SS | 820 | c*g*gguuuuUcUuGuugacaa*a | AV01283-AS | 881 | u*U*ugu(cUNA)AacaaGaAaAacc*c*g |
| AV01284 | AV01284-SS | 821 | c*g*gguuuuUcUuGuugacaa*a | AV01284-AS | 882 | u*U*uguc(aUNA)acaaGaAaAacc*c*g |
| AV01285 | AV01285-SS | 822 | c*g*gguuuuUCUuguugacaa*a | AV01285-AS | 883 | u*U*uguCaacaagaAaAacc*c*g |
| AV01286 | AV01286-SS | 823 | c*g*gguuuuUcUuGuuaacaa*a | AV01286-AS | 884 | u*U*ugucAacaaGaAaAacc*c*g |
| AV01287 | AV01287-SS | 824 | c*g*gguuuuUcUuGuugacag*a | AV01287-AS | 885 | u*C*ugucAacaaGaAaAacc*c*g |
| AV01288 | AV01288-SS | 825 | c*g*gguuuuUcUuGuugaca*a*a | AV01288-AS | 886 | u*U*ugucAacaaGaAaAacc*c*g |
| AV01289 | AV01289-SS | 826 | c*a*ggguuuUuCuUguugaca*u | AV01289-AS | 887 | a*U*gucaAcaagAaAaAccc*u*g |
| AV01290 | AV01290-SS | 827 | c*a*ggguuuUuCuUguugaca*a | AV01290-AS | 888 | u*U*gucaAcaagAaAaAccc*u*g |
| AV01291 | AV01291-SS | 828 | c*a*ggguuuUuCuUguugaca*c | AV01291-AS | 889 | g*U*gucaAcaagAaAaAccc*u*g |
| AV01292 | AV01292-SS | 829 | c* a *ggguuu U uCu Uguugaca *g | AV01292-AS | 890 | c*U*gucaAcaagAaAaAccc*u*g |
| AV01293 | AV01293-SS | 830 | g*u*uuUuCuUguugaca*u | AV01293-AS | 891 | a*U*gucaAcaagAaAa*A*c |
| AV01294 | AV01294-SS | 831 | g*g*uuuUuCuUguugaca*u | AV01294-AS | 892 | a*U*gucaAcaagAaAaA*c*c |
| AV01295 | AV01295-SS | 832 | a*g*guuuUuCuUguugaca*u | AV01295-AS | 893 | a*U*gucaAcaagAaAaAc*c*u |
| AV01296 | AV01296-SS | 833 | u*g*guuuUuCuUguugaca*u | AV01296-AS | 894 | a*U*gucaAcaagAaAaAc*c*a |
| AV01297 | AV01297-SS | 834 | g*g*guuuUuCuUguugaca*u | AV01297-AS | 895 | a*U*gucaAcaagAaAaAc*c*c |
| AV01298 | AV01298-SS | 835 | c*g*guuuUuCuUguugaca*u | AV01298-AS | 896 | a * U *guca AcaagAa Aa Ac*c*g |
| AV01299 | AV01299-SS | 836 | a*g*gguuuUuCuUguugaca*u | AV01299-AS | 897 | a*U*gucaAcaagAaAaAcc*c*u |
| AV01300 | AV01300-SS | 837 | u*g*gguuuUuCuUguugaca*u | AV01300-AS | 898 | a*U*gucaAcaagAaAaAcc*c*a |
| AV01301 | AV01301-SS | 838 | g*g*gguuuUuCuUguugaca*u | AV01301-AS | 899 | a*U*gucaAcaagAaAaAcc*c*c |
| AV01302 | AV01302-SS | 839 | c*g*gguuuUuCuUguugaca*u | AV01302-AS | 900 | a * U *guca AcaagAa Aa Acc*c*g |
| AV01303 | AV01303-SS | 840 | g*a*ggguuuUuCuUguugaca*u | AV01303-AS | 901 | a*U*gucaAcaagAaAaAccc*u*c |
| AV01304 | AV01304-SS | 841 | a*a*ggguuuUuCuUguugaca*u | AV01304-AS | 902 | a*U*gucaAcaagAaAaAccc*u*u |
| AV01305 | AV01305-SS | 842 | u*a*ggguuuUuCuUguugaca*u | AV01305-AS | 903 | a*U*gucaAcaagAaAaAccc*u*a |
| AV01306 | AV01306-SS | 843 | g*c*gggguuuUuCuUguugaca*u | AV01306-AS | 904 | a * U *guca AcaagAa Aa Acccc*g*c |
| AV01307 | AV01307-SS | 844 | g*g*cgggguuuUuCuUguugaca*u | AV01307-AS | 905 | a * U * gucaAcaagAaAaAccccg* c * c |
| AV01308 | AV01308-SS | 845 | a*g*gcgggguuuUuCuUguugaca*u | AV01308-AS | 906 | a*U*gucaAcaagAaAaAccccgc*c*u |
| AV01309 | AV01309-SS | 846 | c*a*ggcgggguuuUuCuUguugaca*u | AV01309-AS | 907 | a*U*gucaAcaagAaAaAccccgcc*u*g |
| AV01310 | AV01310-SS | 847 | a*c*aggcgggguuuUuCuUguugaca*u | AV01310-AS | 908 | a*U*gucaAcaagAaAaAccccgccu*g*u |
| AV01311 | AV01311-SS | 848 | c*g*uguuacaggcgggguuuUuCuUguugaca*u | AV01311-AS | 909 | a*U*gucaAcaagAaAaAccccgccuguaaca*c*g |
| AV01312 | AV01312-SS | 849 | c*a*ggguuuUuCuUguugaca*u | AV01312-AS | 910 | a*U*gucaAcaagAaAaAcccug*u*u |
| AV01313 | AV01313-SS | 850 | c*a*ggguuuUuCuUguugaca*u | AV01313-AS | 911 | a*U*gucaAcaagAaAaAcccug*g*a |
| AV01314 | AV01314-SS | 851 | c*a*ggguuuUuCuUguugaca*u | AV01314-AS | 912 | a*U*guc(aUNA)AcaagAaAaAccc*u*g |
| AV01315 | AV01315-SS | 852 | c*a*ggguuuUuCuUguugaca*u | AV01315-AS | 913 | a*U*guca(aUNA)caagAaAaAccc*u*g |
| AV01316 | AV01316-SS | 853 | c*a*ggguuuUUCuuguugaca*u | AV01316-AS | 914 | a*U*gucAacaagaaAaAccc*u*g |
| AV01317 | AV01317-SS | 854 | c* a *ggguuu U uCu Ugucgaca * u | AV01317-AS | 915 | a*U*gucaAcaagAaAaAccc*u*g |
| AV01318 | AV01318-SS | 855 | c*a*ggguuuUuCuUguugacg*u | AV01318-AS | 916 | a*C*gucaAcaagAaAaAccc*u*g |
| AV01319 | AV01319-SS | 856 | c*a*ggguuuUuCuUguugac*a*u | AV01319-AS | 917 | a*U*gucaAcaagAaAaAccc*u*g |

Table 3 discloses certain chemically modified HBV RNAi agent antisense strand and sense strand sequences and dsRNAs of the invention. In some embodiments of methods of the invention, RNAi agents shown in Table 3 are administered to a cell and/or subject. In some embodiments of methods of the invention, an RNAi agent comprising a polynucleotide sequence shown in Table 3 is administered to a subject. In some embodiments of the invention an RNAi agent administered to a subject comprises a duplex identified in column one in Table 3, wherein the duplex comprises the sequence modifications and/or delivery compound show in the sense and antisense strand sequences in columns four and seven, respectively, in the same row in Table 3. The sequences were used in certain *in vivo* testing studies described elsewhere herein. A sequence shown in Table 3 may in some embodiments, be attached to (also referred to herein as "conjugated to") a compound for delivery, a non-limiting example of which is a GalNAc-containing compound. Certain embodiments of delivery compounds are identified in Table 3 as "GLX-0" on sense strands in column four. As used herein and shown in Table 3, "GLX-0" indicates a GalNAc-containing compound. It will be understood that another of the GLO-n or GLS-n compounds may be substituted for the compound shown as GLO-0, with the resulting compound included in an embodiment of a method and/or a composition of the invention. In some embodiments of the invention, the delivery compound shown in Table 3 as GLX-0 is replaced with any one of compounds GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16, the structure of each of which is provided elsewhere herein. One skilled in the art will be able to prepare and use a dsRNA compound of the invention in which the attached delivery compound is one of GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16. Each row of Table 3 provides a Duplex AD# assigned to the duplex of the sense and antisense sequences in that row of the table. For example, Duplex AD# AD00166 is the duplex comprising sense strand SEQ ID NO: 553 and antisense strand SEQ ID NO: 578. Each line in Table 3 provides a sense strand and an antisense strand, and discloses the duplex of the sense and antisense strands shown. The "Sense strand SS#" in Table 3 column two is the assigned identifier for the Sense Sequence (including modifications) shown column four in the same row. The "Antisense strand AS#" in Table 3 column five is the assigned identifier for the Antisense sequence (including modifications) shown in column seven. An identifier for certain attached GalNAc-containing GLO or GLS compounds is shown as GLX-0, and it will be understood that another of the GLO or GLS compounds may substitute the compound shown as GLX-0, with the resulting compound included in an embodiment of a method and/or a composition of the invention.

**Table 3 provides chemically modified HBV RNAi agent sense strand and antisense strand sequences. All sequences are shown 5' to 3'. The sequences were used in certain in vivo testing studies described herein. A delivery molecule used in the in vivo studies is indicated as "GLX-0" at the 3' end of each sense strand. The chemical modifications are indicated as: upper case: 2'-Fluoro; lower case: 2'-OMe; thiophosphate: ***

| Duplex ID | Sense strand SS# | SEQ ID NO | Sense Strand | Antisense strand AS# | SEQ ID NO | Antisense Strand |
|---|---|---|---|---|---|---|
| AD00166 | AD00166-SS | 553 | u*g*guggacUuCuCucaauuu*a(GLX-0) | AD00166-AS | 578 | u*A*aauuGagagAaGuCcac*c*a |
| AD00167 | AD00167-SS | 554 | a*c*uucucuCaAuUuucuagg*a(GLX-0) | AD00167-AS | 579 | u*C*cuagAaaauUgAgAgaa*g*u |
| AD00168 | AD00168-SS | 555 | g*u*uaccaaUuUuCuuuuguc*a(GLX-0) | AD00168-AS | 580 | u*G*acaaAagaaAaUuGgua*a*c |
| AD00169 | AD00169-SS | 556 | c*a*uaagagGaCuCuuggacu*a(GLX-0) | AD00169-AS | 581 | u*A*guccAagagUcCuCuua*u*g |
| AD00170 | AD00170-SS | 557 | g*g*gguuuuUcUuGuugacaa*a(GLX-0) | AD00170-AS | 582 | u*U*ugucAacaaGaAaAacc*c*c |
| AD00171 | AD00171-SS | 558 | u*u*cgcuucAcCuCugcacgu*a(GLX-0) | AD00171-AS | 583 | u*A*cgugCagagGuGaAgcg*a*a |
| AD00172 | AD00172-SS | 559 | c*c*gucuguGcCuUcucaucu*a(GLX-0) | AD00172-AS | 584 | u*A*gaugAgaagGcAcAgac*g*g |
| AD00173 | AD00173-SS | 560 | a*g*gccuauUgAuUggaaagu*a(GLX-0) | AD00173-AS | 585 | u*A*cuuuCcaauCaAuAggc*c*u |
| AD00174 | AD00174-SS | 561 | g*u*gugcacUuCgCuucaccu*a(GLX-0) | AD00174-AS | 586 | u*A*ggugAagcgAaGuGcac*a*c |
| AD00175 | AD00175-SS | 562 | a*g*guauguUgCcCguuuguc*a(GLX-0) | AD00175-AS | 587 | u*G*acaaAcgggCaAcAuac*c*u |
| AD00176 | AD00176-SS | 563 | c*g*gggcgcAcCuCucuuuac*a(GLX-0) | AD00176-AS | 588 | u*G*uaaaGagagGuGcGccc*c*g |
| AD00177 | AD00177-SS | 564 | c*c*cgucugUgCcUucucauc*a(GLX-0) | AD00177-AS | 589 | u*G*augaGaaggCaCaGacg*g*g |
| AD00261 | AD00261-SS | 565 | u*g*gacuucUcUcAauuuucu*a(GLX-0) | AD00261-AS | 590 | u*A*gaaaAuugaGaGaAguc*c*a |
| AD00262 | AD00262-SS | 566 | c*a*uuuguuCaGuGguucgua*a(GLX-0) | AD00262-AS | 591 | u*U*acgaAccacUgAaCaaa*u*g |
| AD00263 | AD00263-SS | 567 | u*g*uuaccaAuUuUcuuuugu*a(GLX-0) | AD00263-AS | 592 | u*A*caaaAgaaaAuUgGuaa*c*a |
| AD00264 | AD00264-SS | 568 | a*g*gcuguaGgCaUaaauugg*a(GLX-0) | AD00264-AS | 593 | u*C*caauUuaugCcUaCagc*c*u |
| AD00265 | AD00265-SS | 569 | c*u*guuaccAaUuUucuuuug*a(GLX-0) | AD00265-AS | 594 | u*C*aaaaGaaaaUuGgUaac*a*g |
| AD00266 | AD00266-SS | 570 | u*a*caggcgGgGuUuuucuug*a(GLX-0) | AD00266-AS | 595 | u*C*aagaAaaacCcCgCcug*u*a |
| AD00267 | AD00267-SS | 571 | c*g*ggguuuUuCuUguugaca*a(GLX-0) | AD00267-AS | 596 | u*U*gucaAcaagAaAaAccc*c*g |
| AD00268 | AD00268-SS | 572 | u*u*uacuagUgCcAuuuguuc*a(GLX-0) | AD00268-AS | 597 | u*G*aacaAauggCaCuAgua*a*a |
| AD00269 | AD00269-SS | 573 | u*u*accaauUuUcUuuugucu*a(GLX-0) | AD00269-AS | 598 | u*A*gacaAaagaAaAuUggu*a*a |
| AD00270 | AD00270-SS | 574 | a*u*ccugcuGcUaUgccucau*a(GLX-0) | AD00270-AS | 599 | u*A*ugagGcauaGcAgCagg*a*u |
| AD00271 | AD00271-SS | 575 | c*a*gcgcauGcGuGgaaccuu*a(GLX-0) | AD00271-AS | 600 | u*A*agguUccacGcAuGcgc*u*g |
| AD00272 | AD00272-SS | 576 | g*c*uccucuGcCgAuccauac*a(GLX-0) | AD00272-AS | 601 | u*G*uaugGaucgGcAgAgga*g*c |
| AD00273 | AD00273-SS | 577 | u*a*cauaagAgGaCucuugga*a(GLX-0) | AD00273-AS | 602 | u*U*ccaaGagucCuCuUaug*u*a |

Table 4 shows certain chemically modified HBV RNAi agent sense-strand sequences and antisense-strand sequences of the invention and identifies duplexes comprising sense and antisense. In some embodiments of methods of the invention, an RNAi agent with a polynucleotide sequence shown in Table 4 is administered to a subject. In some embodiments of the invention an RNAi agent administered to a subject comprises is a duplex identified in a row in Table 4, column one and includes the sequence modifications and/or delivery compound show in the sense and antisense strand sequences in the same row in Table 4, columns four and seven, respectively. In some embodiments, a sequence shown in Table 4 may be attached to a compound capable of delivering the dsRNA agent to a cell and/or tissue in a subject. A non-limiting example of a delivery compound that may be used in certain embodiments of the invention is a GalNAc-containing compound. In Table 4, the term "GLS-5" indicates a GalNAc-containing compound in the sense strand as shown. It will be understood that in certain embodiments of compounds, compositions, and methods of the invention the GLS-5 is replaced by any one of compounds GLS-1, GLS-2, GLS-3, GLS-4, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16, the structure of each of which is provided elsewhere herein. The first column of Table 4 indicates a duplex identification number for the duplex disclosed in the row.

**Table 4**

| Chemically modified HBV RNAi agent sense strand and antisense strand sequences. Sequences were used in certain in vivo studies described elsewhere herein. All sequences are shown 5' to 3'. Chemical modifications are indicated as: upper case: 2'-Fluoro; lower case: 2'-OMe; thiophosphate: *; Invab = inverted abasic. | | | | | | |
|---|---|---|---|---|---|---|
| Duplex ID | Sense strand SS# | SEQ ID NO | Sense strand | Antisense strand AS# | SEQ ID NO | Antisense Strand |
| AD00166-1 | AD00166-1-SS | 603 | (GLS-5)*(Invab)*ugguggacUuCuCucaauuua*(Invab) | AD00166-1-AS | 638 | u*A*aauuGagagAaGuCcac*c*a |
| AD00167-1 | AD00167-1-SS | 604 | (GLS-5)*(Invab)*acuucucuCaAuUuucuagga*(Invab) | AD00167-1-AS | 639 | u*C*cuagAaaauUgAgAgaa*g*u |
| AD00168-1 | AD00168-1-SS | 605 | (GLS-5)*(Invab)*guuaccaaUuUuCuuuuguca*(Invab) | AD00168-1-AS | 640 | u*G*acaaAagaaAaUuGgua*a*c |
| AD00169-1 | AD00169-1-SS | 606 | (GLS-5)*(Invab)*cauaagagGaCuCuuggacua*(Invab) | AD00169-1-AS | 641 | u*A*guccAagagUcCuCuua*u*g |
| AD00170-1 | AD00170-1-SS | 607 | (GLS-5)*(Invab)*gggguuuuUcUuGuugacaaa*(Invab) | AD00170-1-AS | 642 | u*U*ugucAacaaGaAaAacc*c*c |
| AD00171-1 | AD00171-1-SS | 608 | (GLS-5)*(Invab)*uucgcuucAcCuCugcacgua*(Invab) | AD00171-1-AS | 643 | u*A*cgugCagagGuGaAgcg*a*a |
| AD00172-1 | AD00172-1-SS | 609 | (GLS-5)*(Invab)*ccgucuguGcCuUcucaucua*(Invab) | AD00172-1-AS | 644 | u*A*gaugAgaagGcAcAgac*g*g |
| AD00173-1 | AD00173-1-SS | 610 | (GLS-5)*(Invab)*aggccuauUgAuUggaaagua*(Invab) | AD00173-1-AS | 645 | u*A*cuuuCcaauCaAuAggc*c*u |
| AD00174-1 | AD00174-1-SS | 611 | (GLS-5)*(Invab)*gugugcacUuCgCuucaccua*(Invab) | AD00174-1-AS | 646 | u*A*ggugAagcgAaGuGcac*a*c |
| AD00175-1 | AD00175-1-SS | 612 | (GLS-5)*(Invab)*agguauguUgCcCguuuguca*(Invab) | AD00175-1-AS | 647 | u*G*acaaAcgggCaAcAuac*c*u |
| AD00176-1 | AD00176-1-SS | 613 | (GLS-5)*(Invab)*cggggcgcAcCuCucuuuaca*(Invab) | AD00176-1-AS | 648 | u*G*uaaaGagagGuGcGccc*c*g |
| AD00177-1 | AD00177-1-SS | 614 | (GLS-5)*(Invab)*cccgucugUgCcUucucauca*(Invab) | AD00177-1-AS | 649 | u*G*augaGaaggCaCaGacg*g*g |
| AD00261-1 | AD00261-1-SS | 615 | (GLS-5)*(Invab)*uggacuucUcUcAauuuucua*(Invab) | AD00261-1-AS | 650 | u*A*gaaaAuugaGaGaAguc*c*a |
| AD00262-1 | AD00262-1-SS | 616 | (GLS-5)*(Invab)*cauuuguuCaGuGguucguaa*(Invab) | AD00262-1-AS | 651 | u*U*acgaAccacUgAaCaaa*u*g |
| AD00263-1 | AD00263-1-SS | 617 | (GLS-5)*(Invab)*uguuaccaAuUuUcuuuugua*(Invab) | AD00263-1-AS | 652 | u*A*caaaAgaaaAuUgGuaa*c*a |
| AD00264-1 | AD00264-1-SS | 618 | (G LS-5)*(Invab)*aggcuguaGgCaUaaauugga*(Invab) | AD00264-1-AS | 653 | u*C*caauUuaugCcUaCagc*c*u |
| AD00265-1 | AD00265-1-SS | 619 | (GLS-5)*(Invab)*cuguuaccAaUuUucuuuuga*(Invab) | AD00265-1-AS | 654 | u*C*aaaaGaaaaUuGgUaac*a*g |
| AD00266-1 | AD00266-1-SS | 620 | (GLS-5)*(Invab)*uacaggcgGgGuUuuucuuga*(Invab) | AD00266-1-AS | 655 | u*C*aagaAaaacCcCgCcug*u*a |
| AD00267-1 | AD00267-1-SS | 621 | (GLS-5)*(Invab)*cgggguuuUuCuUguugacaa*(Invab) | AD00267-1-AS | 656 | u*U*gucaAcaagAaAaAccc*c*g |
| AD00268-1 | AD00268-1-SS | 622 | (GLS-5)*(Invab)*uuuacuagUgCcAuuuguuca*(Invab) | AD00268-1-AS | 657 | u*G*aacaAauggCaCuAgua*a*a |
| AD00269-1 | AD00269-1-SS | 623 | (GLS-5)*(Invab)*uuaccaauUuUcUuuugucua*(Invab) | AD00269-1-AS | 658 | u*A*gacaAaagaAaAuUggu*a*a |
| AD00270-1 | AD00270-1-SS | 624 | (GLS-5)*(Invab)*auccugcuGcUaUgccucaua*(Invab) | AD00270-1-AS | 659 | u*A*ugagGcauaGcAgCagg*a*u |
| AD00271-1 | AD00271-1-SS | 625 | (GLS-5)*(Invab)*cagcgcauGcGuGgaaccuua*(Invab) | AD00271-1-AS | 660 | u*A*agguUccacGcAuGcgc*u*g |
| AD00272-1 | AD00272-1-SS | 626 | (GLS-5)*(Invab)*gcuccucuGcCgAuccauaca*(Invab) | AD00272-1-AS | 661 | u*G*uaugGaucgGcAgAgga*g*c |
| AD00273-1 | AD00273-1-SS | 627 | (GLS-5)*(Invab)*uacauaagAgGaCucuuggaa*(Invab) | AD00273-1-AS | 662 | u*U*ccaaGagucCuCuUaug*u*a |
| AD00375 | AD00375-SS | 628 | (GLS-5)*(Invab)*cggguuuuUcUuGuugacaaa*(Invab) | AD00375-AS | 663 | u*U*ugucAacaaGaAaAacc*c*g |
| AD00376 | AD00376-SS | 629 | (GLS-5)*(Invab)*cguuaccaAuUuUcuuuugua*(Invab) | AD00376-AS | 664 | u*A*caaaAgaaaAuUgGuaa*c*g |
| AD00377 | AD00377-SS | 630 | (GLS-5)*(Invab)*guguuaccAaUuUucuuuuga*(Invab) | AD00377-AS | 665 | u*C*aaaaGaaaaUuGgUaac*a*c |
| AD00378 | AD00378-SS | 631 | (GLS-5)*(Invab)*caggguuuUuCuUguugacau*(Invab) | AD00378-AS | 666 | a*U*gucaAcaagAaAaAccc*u*g |
| AD00379 | AD00379-SS | 632 | (GLS-5)*(Invab)*cuuacuagUgCcAuuuguuca*(Invab) | AD00379-AS | 667 | u*G*aacaAauggCaCuAgua*a*g |
| AD00380 | AD00380-SS | 633 | (GLS-5)*(Invab)*cuaccaauUuUcUuuugucua*(Invab) | AD00380-AS | 668 | u*A*gacaAaagaAaAuUggu*a*g |
| AD00381 | AD00381-SS | 634 | (GLS-5)*(Invab)*cggccuauUgAuUggaaagua*(Invab) | AD00381-AS | 669 | u*A*cuuuCcaauCaAuAggc*c*g |
| AD00382 | AD00382-SS | 635 | (GLS-5)*(Invab)*cagggcgcAcCuCucuuuaca*(Invab) | AD00382-AS | 670 | u*G*uaaaGagagGuGcGccc*u*g |
| AD00383 | AD00383-SS | 636 | (GLS-5)*(Invab)*agggguuuUuCuUguugacau*(Invab) | AD00383-AS | 671 | a*U*gucaAcaagAaAaAccc*c*u |
| AD00384 | AD00384-SS | 637 | (GLS-5)*(Invab)*cgagguuuUuCuUguugacaa*(Invab) | AD00384-AS | 672 | u*U*gucaAcaagAaAaAccu*c*g |
| AD00378-1 | AD00378-1-SS | 918 | (GLS-15)*(Invab)*caggguuuUuCuUguugacau*(Invab) | AD00378-1-AS | 922 | a*U*gucaAcaagAaAaAccc*u*g |
| AD00170-2 | AD00170-2-SS | 919 | (GLS-15)*(Invab)*cggguuuuUcUuGuugacaaa*(Invab) | AD00170-2-AS | 923 | u*U*ugucAacaaGaAaAacc*c*g |
| AD00268-2 | AD00268-2-SS | 920 | (GLS-15)*(Invab)*guuacuagUgCcAuuuguuca*(Invab) | AD00268-2-AS | 924 | u*G*aacaAauggCaCuAgua*a*c |
| AD00263-2 | AD00263-2-SS | 921 | (GLS-15)*(Invab)*gguuaccaAuUuUcuuuugua*(Invab) | AD00263-2-AS | 925 | u*A*caaaAgaaaAuUgGuaa*c*c |

In some embodiments of methods and compositions of the invention, the sense strand of a dsRNA agent of the invention comprises one of: SEQ ID NO: 281, 290, 295, 300, 304, 306, 307, 331, 557, 567, 569, 571, 572, 573, 560, 563, 607 , 628-637 or 918-921. In certain embodiments of methods and compositions of the invention, the antisense strand of a dsRNA agent comprises one of: SEQ ID NO: 419, 428, 433, 438, 442, 444, 445, 469, 582, 592, 594, 596, 597, 598, 585, 588, 642, 663-672 or 922-925. In some embodiments of methods and compositions of the invention, the dsRNA agent comprises SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; SEQ ID NO: 637 and SEQ ID NO: 672; SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925.

In certain embodiments of the invention a dsRNA (also referred to herein as a "duplex") is one disclosed in one of Tables 1-4. Each row in Tables 1-4 discloses a duplex comprising the sequence of the sense strand and the sequence of the antisense strand in that table row. In addition to the duplexes disclosed in Tables 1-4, it will be understood that in some embodiments, a duplex of the invention may include sense and antisense sequences shown in Tables 1-4, that differ by zero, one, two, or three nucleotides shown in a sequence shown in Tables 1-4. Thus, in some embodiments, an antisense strand in a duplex of the invention may be SEQ ID NO: 139 with zero, one, two, or three different nucleotides than those in SEQ ID NO: 139.

It will be understood that the sequence of the sense strand and the sequence of the antisense strand in a duplex of the invention may be independently selected. Thus, a dsRNA of the invention may comprise a sense strand and an antisense strand of a duplex disclosed in a row in Tables 1-4. Alternatively, in a dsRNA of the invention, one or both of the selected sense and antisense strand in the dsRNA may include sequences shown in Tables 1-4 but with one or both of the sense and antisense sequences including 1, 2, 3, or more nucleobase substitutions from the parent sequence. The selected sequences may in some embodiments be longer or shorter than their parent sequence. Thus, dsRNA agents included in the invention can but need not include exact sequences of the sense and antisense pairs disclosed as duplexes in Tables 1-4.

In some embodiments, a dsRNA agent comprises a sense strand and an antisense strand, nucleotide positions 2 to 18 in the antisense strand comprising a region of complementarity to an HBV RNA transcript, wherein the region of complementarity comprises at least 15 contiguous nucleotides that differ by 0, 1, 2, or 3 nucleotides from one of the antisense sequences listed in one of Tables 1-4, and optionally comprising a targeting ligand. In some instances, the region of complementarity to the HBV RNA transcript comprises at least 15, 16, 17, 18, or 19 contiguous nucleotides that differ by no more than 3 nucleotides from one of the antisense sequences listed in one of Tables 1-4. In some embodiments of a dsRNA agent of the invention, the antisense strand of the dsRNA is at least substantially complementary to any one of a target region of SEQ ID NO: 673 and is provided in any one of Tables 1-4. In some embodiments, an antisense strand of a dsRNA agent of the invention is fully complementary to any one of a target region of SEQ ID NO: 673 and is provided in any one of Tables 1-4. In some embodiments a dsRNA agent includes a sense strand sequence set forth in any one of Tables 1-4, and the sense strand sequence is at least substantially complementary to the antisense strand sequence in the dsRNA agent. In other embodiments, a dsRNA agent of the invention comprises a sense strand sequence set forth in any one of Tables 1-4, and the sense strand sequence is fully complementary to the antisense strand sequence in the dsRNA agent. In some instances, a dsRNA agent of the invention comprises an antisense strand sequence set forth in any one of Tables 1-4. Some embodiments of a dsRNA agent of the invention comprises the sense and antisense sequences disclosed as duplex in any of Tables 1-4. As described herein, it will be understood that the sense and antisense strands in a duplex of the invention may be independently selected.

### Mismatches

It is known to skilled in art, mismatches are tolerated for efficacy in dsRNA, especially if the mismatches are within terminal region of dsRNA. Certain mismatches are better tolerated in a dsRNA, for example mismatches with wobble base pairs G:U and A:C are tolerated better for efficacy (Du et el., Nucleic Acids Res. 2005 Mar 21;33(5): 1671-7). In some embodiments of methods and compounds of the invention an HBV dsRNA agent may contain one or more mismatches to the HBV target sequence. In some embodiments, HBV dsRNA agent of the invention includes no mismatches. In certain embodiments, HBV dsRNA agent of the invention includes no more than 1, no more than 2, or no more than 3 mismatches to the HBV target sequence. In some embodiments of the invention, an antisense strand of an HBV dsRNA agent contains mismatches to an HBV target sequence that are not located in the center of the region of complementarity. In some embodiments, the antisense strand of the HBV dsRNA agent includes 1, 2, 3, 4, or more mismatches that are within the last 5, 4, 3, 2, or 1 nucleotides from one or both of the 5' end and the 3' end of the region of complementarity. Methods described herein and/or methods known in the art can be used to determine whether an HBV dsRNA agent containing a mismatch to an HBV target sequence is effective in inhibiting the expression of the HBV gene.

### Complementarity

As used herein, unless otherwise indicated, the term "complementary," when used to describe a first nucleotide sequence (e.g., HBV dsRNA agent sense strand) in relation to a second nucleotide sequence (e.g., HBV dsRNA agent antisense strand), means the ability of an oligonucleotide or polynucleotide including the first nucleotide sequence to hybridize [form base pair hydrogen bonds under mammalian physiological conditions (or similar conditions *in vitro*)] and form a duplex or double helical structure under certain conditions with an oligonucleotide or polynucleotide including the second nucleotide sequence. Other conditions, such as physiologically relevant conditions as can be encountered inside an organism, can apply. A skilled artisan will be able to determine the set of conditions most appropriate for a test of complementarity of two sequences in accordance with the ultimate application of the hybridized nucleotides. Complementary sequences include Watson-Crick base pairs or non-Watson-Crick base pairs and include natural or modified nucleotides or nucleotide mimics, at least to the extent that the above hybridization requirements are fulfilled. Sequence identity or complementarity is independent of modification.

Complementary sequences, for example, within an HBV dsRNA as described herein, include base-pairing of the oligonucleotide or polynucleotide comprising a first nucleotide sequence to an oligonucleotide or polynucleotide comprising a second nucleotide sequence over the entire length of one or both nucleotide sequences. Such sequences can be referred to as "fully complementary" with respect to each other herein. It will be understood that in embodiments when two oligonucleotides are designed to form, upon hybridization, one or more single-stranded overhangs, such overhangs are not regarded herein as mismatches with regard to the determination of complementarity. For example, an HBV dsRNA agent comprising one oligonucleotide 19 nucleotides in length and another oligonucleotide 20 nucleotides in length, wherein the longer oligonucleotide comprises a sequence of 19 nucleotides that is fully complementary to the shorter oligonucleotide, can yet be referred to as "fully complementary" for the purposes described herein. Thus, as used herein, "fully complementary" means that all (100%) of the bases in a contiguous sequence of a first polynucleotide will hybridize with the same number of bases in a contiguous sequence of a second polynucleotide. The contiguous sequence may comprise all or a part of a first or second nucleotide sequence.

The term "substantially complementary" as used herein means that in a hybridized pair of nucleobase sequences, at least about 85%, 86%, 87%, 88%, 89%, 90%, 91%, 92%, 93%, 94%, 95%, 96%, 97%, 98%, or 99%, but not all, of the bases in a contiguous sequence of a first polynucleotide will hybridize with the same number of bases in a contiguous sequence of a second polynucleotide. The term "substantially complementary" can be used in reference to a first sequence with respect to a second sequence if the two sequences include one or more, for example at least 1, 2, 3, 4, or 5 mismatched base pairs upon hybridization for a duplex up to 15, 16, 17, 18, 19, 20, 21, 22, 23, 24, 25, 26, 27, 28, 29 or 30 base pairs (bp), while retaining the ability to hybridize under the conditions most relevant to their ultimate application, e.g., inhibition of HBV gene expression via a RISC pathway.

The term, "partially complementary" may be used herein in reference to a hybridized pair of nucleobase sequences, in which at least 75%, but not all, of the bases in a contiguous sequence of a first polynucleotide will hybridize with the same number of bases in a contiguous sequence of a second polynucleotide. In some embodiments, "partially complementary" means at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the bases in a contiguous sequence of a first polynucleotide will hybridize with the same number of bases in a contiguous sequence of a second polynucleotide

The terms "complementary," "fully complementary," "substantially complementary," and "partially complimentary" are used herein in reference to the base matching between the sense strand and the antisense strand of an HBV dsRNA agent or between the antisense strand of an HBV dsRNA agent and a sequence of a target HBV mRNA. It will be understood that the term "antisense strand of an HBV dsRNA agent" may refer to the same sequence of an "HBV antisense polynucleotide agent".

As used herein, the term "substantially identical" or "substantial identity" used in reference to a nucleic acid sequence means a nucleic acid sequence comprising a sequence with at least about 85% sequence identity or more, preferably at least 90%, at least 91%, at least 92%, at least 93%, at least 94%, at least 95%, at least 96%, at least 97%, at least 98%, or at least 99%, compared to a reference sequence. Percentage of sequence identity is determined by comparing two optimally aligned sequences over a comparison window. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity. The inventions disclosed herein encompasses nucleotide sequences substantially identical to those disclosed herein, e.g., in Tables 1-4. In some embodiments, the sequences disclosed herein are exactly identical, or at least about 85%, 86%, 87%, 88%, 89%, 90%, 91%, 92%, 93%, 94%, 95%, 96%, 97%, 98%, or 99% percent identical to those disclosed herein, e.g., in Tables 1-4.

As used herein, the term "strand comprising a sequence" means an oligonucleotide comprising a chain of nucleotides that is described by the sequence referred to using the standard nucleotide nomenclature. The term "double-stranded RNA" or "dsRNA," as used herein, refers to an RNAi that includes an RNA molecule or complex of molecules having a hybridized duplex region comprising two anti-parallel and substantially or fully complementary nucleic acid strands, which are referred to as having "sense" and "antisense" orientations with respect to a target HBV RNA. The duplex region can be of any length that permits specific degradation of a desired target HBV RNA through a RISC pathway, but will typically range from 9 to 30 base pairs in length, e.g., 15-30 base pairs in length. Considering a duplex between 9 and 30 base pairs, the duplex can be any length in this range, for example, 9, 10, 11, 12, 13, 14, 15, 16, 17, 18, 19, 20, 21, 22, 23, 24, 25, 26, 27, 28, 29, or 30, and any sub-range therein between, including, but not limited to 15-30 base pairs, 15-26 base pairs, 15-23 base pairs, 15-22 base pairs, 15-21 base pairs, 15-20 base pairs, 15-19 base pairs, 15-18 base pairs, 15-17 base pairs, 18-30 base pairs, 18-26 base pairs, 18-23 base pairs, 18-22 base pairs, 18-21 base pairs, 18-20 base pairs, 19-30 base pairs, 19-26 base pairs, 19-23 base pairs, 19-22 base pairs, 19-21 base pairs, 19-20 base pairs, 20-30 base pairs, 20-26 base pairs, 20-25 base pairs, 20-24 base pairs, 20-23 base pairs, 20-22 base pairs, 20-21 base pairs, 21-30 base pairs, 21-26 base pairs, 21-25 base pairs, 21-24 base pairs, 21-23 base pairs, or 21-22 base pairs.

HBV dsRNA agents generated in the cell by processing with Dicer and similar enzymes are generally in the range of 19-22 base pairs in length. One strand of the duplex region of an HBV dsDNA agent comprises a sequence that is substantially complementary to a region of a target HBV RNA. The two strands forming the duplex structure can be from a single RNA molecule having at least one self-complementary region, or can be formed from two or more separate RNA molecules. Where the duplex region is formed from two strands of a single molecule, the molecule can have a duplex region separated by a single stranded chain of nucleotides (herein referred to as a "hairpin loop") between the 3'-end of one strand and the 5'-end of the respective other strand forming the duplex structure. In some embodiments of the invention, a hairpin look comprises at least 1, 2, 3, 4, 5, 6, 7, 8, 9, 10, 11, 12, 13, 14, 15, 16, 17, 18, 19, 20, or more unpaired nucleotides. Where the two substantially complementary strands of an HBV dsRNA agent are comprised by separate RNA molecules, those molecules need not, but can be covalently connected. Where the two strands are connected covalently by means other than a hairpin loop, the connecting structure is referred to as a "linker." The term "siRNA" is also used herein to refer to a dsRNA agent as described herein.

In some embodiments of the invention an HBV dsRNA agent may include a sense and antisense sequence that have no-unpaired nucleotides or nucleotide analogs at one or both terminal ends of the dsRNA agent. An end with no unpaired nucleotides is referred to as a "blunt end" and as having no nucleotide overhang. If both ends of a dsRNA agent are blunt, the dsRNA is referred to as "blunt ended." In some embodiments of the invention, only a first end of a dsRNA agent is blunt, in some embodiments only a second end of a dsRNA agent is blunt, and in certain embodiments of the invention, both ends of an HBV dsRNA agent are blunt.

In some embodiments of dsRNA agents of the invention, the dsRNA does not have one or two blunt ends. In such instances there is at least one unpaired nucleotide at the end of a strand of a dsRNA agent. For example, when a 3'-end of one strand of a dsRNA extends beyond the 5'-end of the other strand, or vice versa, there is a nucleotide overhang. A dsRNA can comprise an overhang of at least 1, 2, 3, 4, 5, 6, or more nucleotides. A nucleotide overhang can comprise or consist of a nucleotide/nucleoside analog, including a deoxynucleotide/nucleoside. It will be understood that in some embodiments a nucleotide overhang is on a sense strand of a dsRNA agent, on an antisense strand of a dsRNA agent, or on both ends of a dsRNA agent and nucleotide(s) of an overhang can be present on the 5' end, 3' end or both ends of either an antisense or sense strand of a dsRNA. In certain embodiments of the invention, one or more of the nucleotides in an overhang is replaced with a nucleoside thiophosphate.

As used herein, the term "antisense strand" or "guide strand" refers to the strand of an HBV dsRNA agent that includes a region that is at least substantially complementary to an HBV target sequence. As used herein the term "sense strand," or "passenger strand" refers to the strand of an HBV dsRNA agent that includes a region that is at least substantially complementary to a region of the antisense strand of the HBV dsRNA agent.

### Modifications

In some embodiments of the invention the RNA of an HBV RNAi agent is chemically modified to enhance stability and/or one or more other beneficial characteristics. Nucleic acids in certain embodiments of the invention may be synthesized and/or modified by methods well established in the art, for example, those described in "Current protocols in Nucleic Acid Chemistry," Beaucage, S. L. et al. (Eds.), John Wiley & Sons, Inc., New York, N.Y., USA, which is incorporated herein by reference. Modifications that can be present in certain embodiments of HBV dsRNA agents of the invention include, for example, (a) end modifications, e.g., 5' end modifications (phosphorylation, conjugation, inverted linkages, etc.) 3' end modifications (conjugation, DNA nucleotides, inverted linkages, etc.), (b) base modifications, e.g., replacement with stabilizing bases, destabilizing bases, or bases that base pair with an expanded repertoire of partners, removal of bases (abasic nucleotides), or conjugated bases, (c) sugar modifications (e.g., at the 2' position or 4' position) or replacement of the sugar, as well as (d) backbone modifications, including modification or replacement of the phosphodiester linkages. Specific examples of RNA compounds useful in certain embodiments of HBV dsRNA agents, HBV antisense polynucleotides, and HBV sense polynucleotides of the invention include, but are not limited to RNAs comprising modified backbones or no natural internucleoside linkages. As a non-limiting example, an RNA having a modified backbone may not have a phosphorus atom in the backbone. RNAs that do not have a phosphorus atom in their internucleoside backbone may be referred to as oligonucleosides. In certain embodiments of the invention, a modified RNA has a phosphorus atom in its internucleoside backbone.

It will be understood that the term "RNA molecule" or "RNA" or "ribonucleic acid molecule" encompasses not only RNA molecules as expressed or found in nature, but also analogs and derivatives of RNA comprising one or more ribonucleotide/ribonucleoside analogs or derivatives as described herein or as known in the art. The terms "ribonucleoside" and "ribonucleotide" may be used interchangeably herein. An RNA molecule can be modified in the nucleobase structure or in the ribose-phosphate backbone structure, e.g., as described herein below, and molecules comprising ribonucleoside analogs or derivatives must retain the ability to form a duplex. As non-limiting examples, an RNA molecule can also include at least one modified ribonucleoside including but not limited to a 2'-O-methyl modified nucleoside, a nucleoside comprising a 5' phosphorothioate group, a terminal nucleoside linked to a cholesteryl derivative or dodecanoic acid bisdecylamide group, a locked nucleoside, an abasic nucleoside, a 2'-deoxy-2'-fluoro modified nucleoside, a 2'-amino-modified nucleoside, 2'-alkyl-modified nucleoside, morpholino nucleoside, a phosphoramidate or a non-natural base comprising nucleoside, or any combination thereof. In some embodiments of the invention, an RNA molecule comprises at least 1, 2, 3, 4, 5, 6, 7, 8, 9, 10, 11, 12, 13, 14, 15, 16, 17, 18, 19, 20, or up to the full length of the HBV dsRNA agent molecule's ribonucleosides that are modified ribonucleosides. The modifications need not be the same for each of such a plurality of modified ribonucleosides in an RNA molecule.

dsRNA agents, HBV antisense polynucleotides, and/or HBV sense polynucleotides of the invention may, in some embodiments comprise one or more independently selected modified nucleotide and/or one or more independently selected non-phosphodiester linkage. As used herein the term "independently selected" used in reference to a selected element, such as a modified nucleotide, non-phosphodiester linkage, a targeting agent, a dsRNA agent, a duplex of the invention, etc., means that two or more selected elements can but need not be the same as each other.

As used herein, a "nucleotide base," "nucleotide," or "nucleobase" is a heterocyclic pyrimidine or purine compound, which is a standard constituent of all nucleic acids, and includes the bases that form the nucleotides adenine (a), guanine (g), cytosine (c), thymine (t), and uracil (u). A nucleobase may further be modified to include, though not intended to be limiting: universal bases, hydrophobic bases, promiscuous bases, size-expanded bases, and fluorinated bases. The term "ribonucleotide" or "nucleotide" may be used herein to refer to an unmodified nucleotide, a modified nucleotide, or a surrogate replacement moiety. Those in the art will recognize that guanine, cytosine, adenine, and uracil can be replaced by other moieties without substantially altering the base pairing properties of an oligonucleotide comprising a nucleotide bearing such replacement moiety.

In one embodiment, modified RNAs contemplated for use in methods and compositions described herein are peptide nucleic acids (PNAs) that have the ability to form the required duplex structure and that permit or mediate the specific degradation of a target RNA via a RISC pathway. In certain embodiments of the invention, an HBV RNA interference agent includes a single stranded RNA that interacts with a target HBV RNA sequence to direct the cleavage of the target HBV RNA.

Modified RNA backbones can include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included. Means of preparing phosphorus-containing linkages are routinely practiced in the art and such methods can be used to prepare certain modified HBV dsRNA agents, certain modified HBV antisense polynucleotides, and/or certain modified HBV sense polynucleotides of the invention.

Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Means of preparing modified RNA backbones that do not include a phosphorus atom are routinely practiced in the art and such methods can be used to prepare certain modified HBV dsRNA agents, certain modified HBV antisense polynucleotides, and/or certain modified HBV sense polynucleotides of the invention.

In certain embodiments of the invention, RNA mimetics are included in HBV dsRNAs, HBV antisense polynucleotides, and/or HBV sense polynucleotides, such as, but not limited to: replacement of the sugar and the internucleoside linkage, i.e., the backbone, of the nucleotide units with novel groups. In such embodiments, base units are maintained for hybridization with an appropriate HBV nucleic acid target compound. One such oligomeric compound, an RNA mimetic that has been shown to have excellent hybridization properties, is referred to as a peptide nucleic acid (PNA). In PNA compounds, the sugar backbone of an RNA is replaced with an amide containing backbone, in particular an aminoethylglycine backbone. The nucleobases are retained and are bound directly or indirectly to aza nitrogen atoms of the amide portion of the backbone. Means of preparing RNA mimetics are routinely practiced in the art and such methods can be used to prepare certain modified HBV dsRNA agents of the invention.

Some embodiments of the invention include RNAs with phosphorothioate backbones and oligonucleosides with heteroatom backbones, and in particular --CH₂--NH--CH₂-, --CH₂-N(CH₃)--O--CH₂--[known as a methylene (methylimino) or MMI backbone], --CH₂--O-N(CH₃)--CH₂--, --CH₂--N(CH₃)--N(CH₃)--CH₂-- and --N(CH₃)--CH₂----[wherein the native phosphodiester backbone is represented as --O--P--O--CH₂--]. Means of preparing RNAs with phosphorothioate backbones and oligonucleosides with heteroatom backbones are routinely practiced in the art and such methods can be used to prepare certain modified HBV dsRNA agents, certain HBV antisense polynucleotides, and/or certain HBV sense polynucleotides of the invention.

Modified RNAs can also contain one or more substituted sugar moieties. HBV dsRNAs, HBV antisense polynucleotides, and/or HBV sense polynucleotides of the invention may comprise one of the following at the 2' position: OH; F; O--, S--, or N-alkyl; O--, S--, or N-alkenyl; O--, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Exemplary suitable modifications include O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. In other embodiments, dsRNAs include one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an HBV dsRNA agent, or a group for improving the pharmacodynamic properties of an HBV dsRNA agent, HBV antisense polynucleotide, and/or HBV sense polynucleotide, and other substituents having similar properties. In some embodiments, the modification includes a 2'-methoxyethoxy (2'-O--CH₂CH₂OCH₃, also known as 2'-O-(2-methoxyethyl) or 2'-MOE) (Martin et al., Helv. Chim. Acta, 1995, 78:486-504) i.e., an alkoxy-alkoxy group. Another exemplary modification is 2'-dimethylaminooxyethoxy, i.e., a O(CH₂)₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), i.e., 2'-O--CH₂-O--CH₂--N(CH₂)₂. Means of preparing modified RNAs such as those described are routinely practiced in the art and such methods can be used to prepare certain modified HBV dsRNA agents of the invention.

Other modifications include 2'-methoxy (2'-OCH₃), 2'-aminopropoxy (2'-OCH₂CH₂CH₂NH₂) and 2'-fluoro (2'-F). Similar modifications can also be made at other positions on the RNA of an HBV dsRNA agent, HBV antisense polynucleotide, and/or HBV sense polynucleotide of the invention, particularly the 3' position of the sugar on the 3' terminal nucleotide or in 2'-5' linked HBV dsRNAs, HBV antisense polynucleotides, or HBV sense polynucleotides, and the 5' position of 5' terminal nucleotide. HBV dsRNA agents, HBV antisense polynucleotides, and/or HBV sense polynucleotides may also have sugar mimetics such as cyclobutyl moieties in place of the pentofuranosyl sugar. Means of preparing modified RNAs such as those described are routinely practiced in the art and such methods can be used to prepare certain modified HBV dsRNA agents, HBV antisense polynucleotides, and/or HBV sense polynucleotides of the invention.

An HBV dsRNA agent, HBV antisense polynucleotide, and/or HBV sense polynucleotide may, in some embodiments, include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Additional nucleobases that may be included in certain embodiments of HBV dsRNA agents of the invention are known in the art, see for example: Modified Nucleosides in Biochemistry, Biotechnology and Medicine, Herdewijn, P. Ed. Wiley-VCH, 2008; The Concise Encyclopedia Of Polymer Science And Engineering, pages 858-859, Kroschwitz, J. L, Ed. John Wiley & Sons, 1990, English et al., Angewandte Chemie, International Edition, 1991, 30, 613, Sanghvi, Y S., Chapter 15, dsRNA Research and Applications, pages 289-302, Crooke, S. T. and Lebleu, B., Ed., CRC Press, 1993. Means of preparing dsRNAs, HBV antisense strand polynucleotides and/or HBV sense strand polynucleotides that comprise nucleobase modifications and/or substitutions such as those described herein are routinely practiced in the art and such methods can be used to prepare certain modified HBV dsRNA agents, HBV sense polynucleotides, and/or HBV antisense polynucleotides of the invention.

Certain embodiments of HBV dsRNA agents, HBV antisense polynucleotides, and/or HBV sense polynucleotides of the invention include RNA modified to include one or more locked nucleic acids (LNA). A locked nucleic acid is a nucleotide with a modified ribose moiety comprising an extra bridge connecting the 2' and 4' carbons. This structure effectively "locks" the ribose in the 3'-endo structural conformation. The addition of locked nucleic acids in an HBV dsRNA agent, HBV antisense polynucleotides, and/or HBV sense polynucleotides of the invention may increase stability in serum, and to reduce off-target effects (Elmen, J. et al., (2005) Nucleic Acids Research 33(1):439-447; Mook, O R. et al., (2007)Mol Canc Ther 6(3):833-843; Grunweller, A. et al., (2003) Nucleic Acids Research 31(12):3185-3193). Means of preparing dsRNA agents, HBV antisense polynucleotides, and/or HBV sense polynucleotides that comprise locked nucleic acid(s) are routinely practiced in the art and such methods can be used to prepare certain modified HBV dsRNA agents of the invention.

Certain embodiments of HBV dsRNA compounds, sense polynucleotides, and/or antisense polynucleotides of the invention, include at least one modified nucleotide, wherein the at least one modified nucleotide comprises: a 2'-O-methyl nucleotide,2'-Fluoro nucleotide, 2'-deoxy nucleotide, 2'3'-seco nucleotide mimic, locked nucleotide, 2'-F-Arabino nucleotide, 2'-methoyxyethyl nucleotide, 2'-amino-modified nucleotide, 2'-alkyl-modified nucleotide, mopholino nucleotide, and 3'-OMe nucleotide, a nucleotide comprising a 5'-phosphorothioate group, or a terminal nucleotide linked to a cholesteryl derivative or dodecanoic acid bisdecylamide group, a 2'-amino-modified nucleotide, a phosphoramidate, or a non-natural base comprising nucleotide. In some embodiments, an HBV dsRNA compound includes an E-vinylphosphonate nucleotide at the 5' end of the antisense strand, also referred to herein as the guide strand.

Certain embodiments of HBV dsRNA compounds, 3' and 5' end of sense polynucleotides, and/or 3' end of antisense polynucleotides of the invention, include at least one modified nucleotide, wherein the at least one modified nucleotide comprises: abasic nucleotide, ribitol, inverted nucleotide, inverted abasic nucleotide, inverted 2'-OMe nucleotide, inverted 2'-deoxy nucleotide. It is known to skilled in art, including an abasic or inverted abasic nucleotide at the end of oligonucleotide enhances stability (Czauderna et al. Nucleic Acids Res. 2003;31(11):2705-2716. doi:10.1093/nar/gkg393).

Certain embodiments of HBV dsRNA compounds, antisense polynucleotides of the invention, include at least one modified nucleotide, wherein the at least one modified nucleotide comprises unlocked nucleic acid nucleotide (UNA) or/and glycol nucleic acid nucleotide (GNA). It is known to skilled in art, UNA and GNA are thermally destabilizing chemical modifications, can significantly improves the off-target profile of a siRNA compound (Janas, et al., Nat Commun. 2018;9(1):723. doi:10.1038/s41467-018-02989-4; Laursen et al., Mol BioSyst. 2010;6:862-70).

Another modification that may be included in the RNA of certain embodiments of HBV dsRNA agents, HBV antisense polynucleotides, and/or HBV sense polynucleotides of the invention, comprises chemically linking to the RNA one or more ligands, moieties or conjugates that enhance one or more characteristics of the HBV dsRNA agent, HBV antisense polynucleotide, and/or HBV sense polynucleotide, respectively. Non-limiting examples of characteristics that may be enhanced are: HBV dsRNA agent, HBV antisense polynucleotide, and/or HBV sense polynucleotide activity, cellular distribution, delivery of an HBV dsRNA agent, pharmacokinetic properties of an HBV dsRNA agent, and cellular uptake of the HBV dsRNA agent. In some embodiments of the invention, an HBV dsRNA agent comprises one or more targeting groups or linking groups, which in certain embodiments of HBV dsRNA agents of the invention are conjugated to the sense strand. A non-limiting example of a targeting group is a compound comprising N-acetyl-galactosamine (GalNAc). The terms "targeting group", "targeting agent", "linking agent", "targeting compound", and "targeting ligand" may be used interchangeably herein. In certain embodiments of the invention an HBV dsRNA agent comprises a targeting compound that is conjugated to the 5'-terminal end of the sense strand. In certain embodiments of the invention an HBV dsRNA agent comprises a targeting compound that is conjugated to the 3'-terminal end of the sense strand. In some embodiments of the invention, an HBV dsRNA agent comprises a targeting group that comprises GalNAc. In certain embodiments of the invention an HBV dsRNA agent does not include a targeting compound conjugated to one or both of the 3'-terminal end and the 5'-terminal end of the sense strand. In certain embodiments of the invention an HBV dsRNA agent does not include a GalNAc containing targeting compound conjugated to one or both of the 5'-terminal end and the 3'-terminal end of the sense strand.

Additional targeting and linking agents are well known in the art, for example, targeting and linking agents that may be used in certain embodiments of the invention include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4: 1053-1060), a thioether, e.g., beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, e.g., dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, e.g., di-hexadecyl-rac-glycerol or triethyl-ammonium 1,2-di-O-hexadecyl-rac-glycero-3-phosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

Certain embodiments of a composition comprising an HBV dsRNA agent, HBV antisense polynucleotide, and/or HBV sense polynucleotide may comprise a ligand that alters distribution, targeting, or etc. of the HBV dsRNA agent. In some embodiments of a composition comprising an HBV dsRNA agent of the invention, the ligand increases affinity for a selected target, e.g., molecule, cell or cell type, compartment, e.g., a cellular or organ compartment, tissue, organ or region of the body, as, e.g., compared to a species absent such a ligand. A ligand useful in a composition and/or method of the invention may be a naturally occurring substance, such as a protein (e.g., human serum albumin (HSA), low-density lipoprotein (LDL), or globulin); a carbohydrate (e.g., a dextran, pullulan, chitin, chitosan, inulin, cyclodextrin or hyaluronic acid); or a lipid. A ligand may also be a recombinant or synthetic molecule, such as a synthetic polymer, e.g., a synthetic polyamino acid or polyamine. Examples of polyamino acids are a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

A ligand included in a composition and/or method of the invention may comprise a targeting group, non-limiting examples of which are a cell or tissue targeting agent, e.g., a lectin, glycoprotein, lipid or protein, e.g., an antibody that binds to a specified cell type such as a kidney cell or a liver cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, vitamin A, biotin, or an RGD peptide or RGD peptide mimetic.

Other examples of ligands include dyes, intercalating agents (e.g. acridines), crosslinkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), lipophilic molecules, e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]₂, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

A ligand included in a composition and/or method of the invention may be a protein, e.g., glycoprotein, or peptide, for example a molecule with a specific affinity for a co-ligand, or an antibody, for example an antibody, that binds to a specified cell type such as, but not limited to: a liver cell. A ligand useful in an embodiment of a composition and/or method of the invention can be a hormone or hormone receptor. A ligand useful in an embodiment of a composition and/or method of the invention can be a lipid, lectin, carbohydrates, vitamin, cofactos, multivalent lactose, multivalent galactose, N-acetyl-galactosamine, N-acetyl-gulucosamine multivalent mannose, or multivalent fucose. A ligand useful in an embodiment of a composition and/or method of the invention can be a substance that can increase uptake of the HBV dsRNA agent into the cell, for example, by disrupting the cell's cytoskeleton, e.g., by disrupting the cell's microtubules, microfilaments, and/or intermediate filaments. Non-limiting examples of this type of agent are: taxon, vincristine, vinblastine, cytochalasin, nocodazole, japlakinolide, latrunculin A, phalloidin, swinholide A, indanocine, and myoservin.

In some embodiments, a ligand attached to an HBV dsRNA agent of the invention functions as a pharmacokinetic (PK) modulator. An example of a PK modulator that may be used in compositions and methods of the invention includes but is not limited to: a lipophiles, a bile acid, a steroid, a phospholipid analogue, a peptide, a protein binding agent, PEG, a vitamin, cholesterol, a fatty acid, cholic acid, lithocholic acid, dialkylglycerides, diacylglyceride, a phospholipid, a sphingolipid, naproxen, ibuprofen, vitamin E, biotin, an aptamer that binds a serum protein, etc. Oligonucleotides comprising a number of phosphorothioate linkages are also known to bind to serum protein, thus short oligonucleotides, e.g., oligonucleotides of about 5 bases, 10 bases, 15 bases or 20 bases, comprising multiple of phosphorothioate linkages in the backbone may also be used in compositions and/or methods of the invention as ligands.

### HBV dsRNA agent compositions

In some embodiments of the invention, an HBV dsRNA agent is in a composition. A composition of the invention may include one or more HBV dsRNA agent and optionally one or more of a pharmaceutically acceptable carrier, a delivery agent, a targeting agent, detectable label, etc. A non-limiting example of a targeting agent that may be useful according to some embodiments of methods of the invention is an agent that directs an HBV dsRNA agent of the invention to and/or into a cell to be treated. A targeting agent of choice will depend upon such elements as: the nature of the HBV-associated disease or condition, and on the cell type being targeted. In a non-limiting example, in some embodiments of the invention it may be desirable to target an HBV dsRNA agent to and/or into a liver cell. It will be understood that in some embodiments of methods of the invention, a therapeutic agent comprises a HBV dsRNA agent with only a delivery agent, such as a delivery agent comprising N-Acetylgalactosamine (GalNAc), without any additional attached elements. For example, in some aspects of the invention an HBV dsRNA agent may be attached to a delivery compound comprising GalNAc and included in a composition comprising a pharmaceutically acceptable carrier and administered to a cell or subject without any detectable labels, or targeting agents, etc. attached to the HBV dsRNA agent.

In cases where an HBV dsRNA agent of the invention is administered with and/or attached to one or more delivery agents, targeting agents, labeling agents, etc. a skilled artisan will be aware of and able to select and use suitable agents for use in methods of the invention. Labeling agents may be used in certain methods of the invention to determine the location of an HBV dsRNA agent in cells and tissues and may be used to determine a cell, tissue, or organ location of a treatment composition comprising an HBV dsRNA agent that has been administered in methods of the invention. Procedures for attaching and utilizing labeling agents such as enzymatic labels, dyes, radiolabels, etc. are well known in the art. It will be understood that in some embodiments of compositions and methods of the invention, a labeling agent is attached to one or both of a sense polynucleotide and an antisense polynucleotide included in an HBV dsRNA agent.

### Delivery of HBV dsRNA agents

Certain embodiments of methods of the invention, includes delivery of an HBV dsRNA agent into a cell. As used herein the term, "delivery" means facilitating or effecting uptake or absorption into the cell. Absorption or uptake of an HBV dsRNA agent can occur through unaided diffusive or active cellular processes, or by use of delivery agents, targeting agents, etc. that may be associated with an HBV dsRNA agent of the invention. Delivery means that are suitable for use in methods of the invention include, but are not limited to: *in vivo* delivery, in which an HBV dsRNA agent is in injected into a tissue site or administered systemically. In some embodiments of the invention, an HBV dsRNA agent is attached to a delivery agent.

Non-limiting examples of methods that can be used to deliver HBV dsRNA agents to cells, tissues and/or subjects include: HBV dsRNA-GalNAc conjugates, SAMiRNA technology, LNP-based delivery methods, and naked RNA delivery. These and other delivery methods have been used successfully in the art to deliver therapeutic RNAi agents for treatment of various diseases and conditions, such as but not limited to: liver diseases, acute intermittent porphyria (AIP), hemophilia, pulmonary fibrosis, etc. Details of various delivery means are found in publications such as: Nikam, R.R. & K. R. Gore (2018) Nucleic Acid Ther, 28 (4), 209-224 Aug 2018; Springer A.D. & S.F. Dowdy (2018) Nucleic Acid Ther. Jun 1; 28(3): 109-118; Lee, K. et al., (2018) Arch Pharm Res, 41(9), 867-874; and Nair, J.K. et al., (2014) J. Am. Chem. Soc. 136:16958-16961, the content each of which is incorporated by reference herein.

Some embodiments of the invention comprise use of lipid nanoparticles (LNPs) to deliver an HBV dsRNA agent of the invention to a cell, tissue, and/or subject. LNPs are routinely used for *in vivo* delivery of HBV dsRNA agents, including therapeutic HBV dsRNA agents. One benefit of using an LNP or other delivery agent is an increased stability of the HBV RNA agent when it is delivered to a subject using the LNP or other delivery agent. In some embodiments of the invention an LNP comprises a cationic LNP that is loaded with one or more HBV RNAi molecules of the invention. The LNP comprising the HBV RNAi molecule(s) is administered to a subject, the LNPs and their attached HBV RNAi molecules are taken up by cells via endocytosis, their presence results in release of RNAi trigger molecules, which mediate RNAi.

Another non-limiting example of a delivery agent that may be used in embodiments of the invention to delivery an HBV dsRNA agent of the invention to a cell, tissue and/or subject is an agent comprising GalNAc that is attached to an HBV dsRNA agent of the invention and delivers the HBV dsRNA agent to a cell, tissue, and/or subject. Examples of certain additional delivery agents comprising GalNAc that can be used in certain embodiments of methods and composition of the invention are disclosed in PCT Application: WO2020191183A1. A non-limiting example of a GalNAc targeting ligand that can be used in compositions and methods of the invention to deliver an HBV dsRNA agent to a cell is a targeting ligand cluster. Examples of targeting ligand clusters that are presented herein are referred to as: GalNAc Ligand with phosphodiester link (GLO) and GalNAc Ligand with phosphorothioate link (GLS). The term "GLX-0" may be used herein to indicate the attached GalNAC-containing compound is any one of compounds GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16, the structure of each of which is shown below, with the below with location of attachment of the GalNAc-targeting ligand to an RNAi agent of the invention at far right of each (shown with" 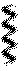 " ). It will be understood that any RNAi and dsRNA molecule of the invention can be attached to the GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16. GLO-1 through GLO-16 and GLS-1 through GLS-16 structures are shown as below.

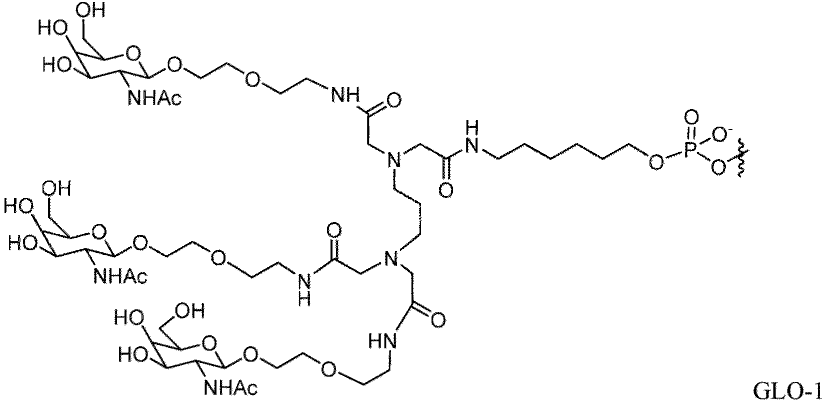

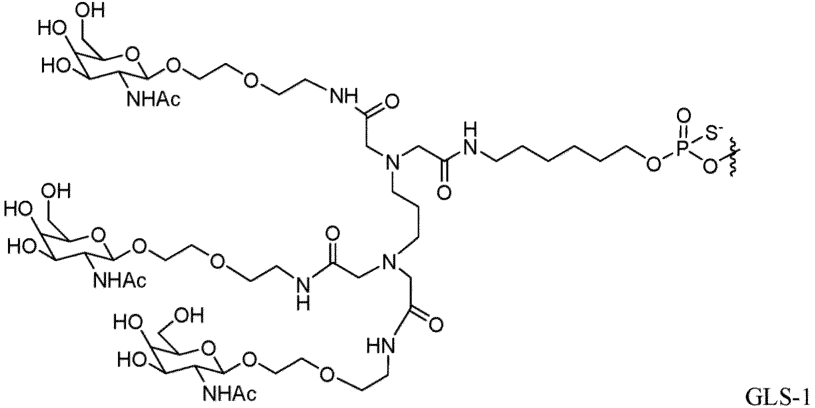

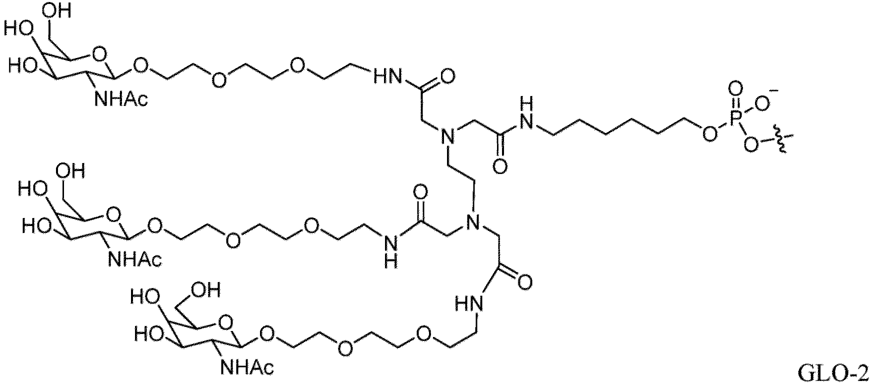

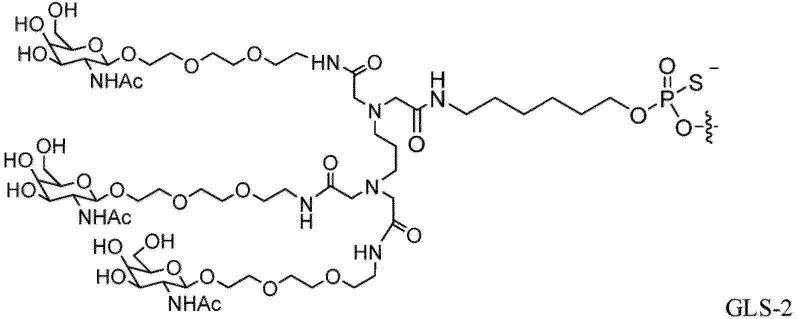

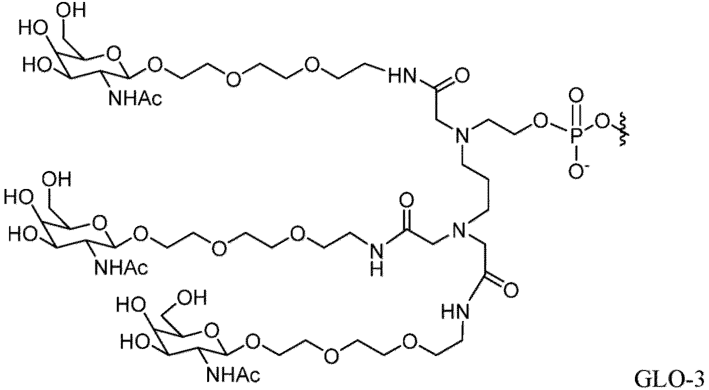

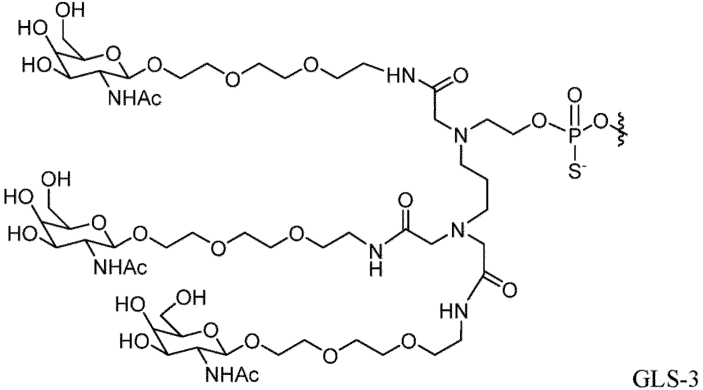

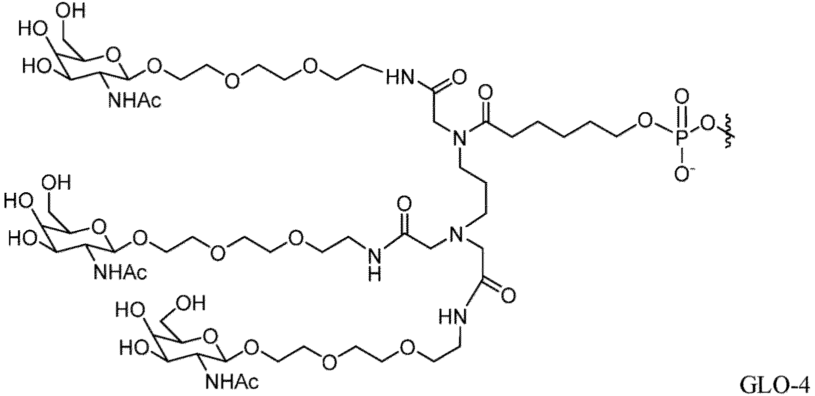

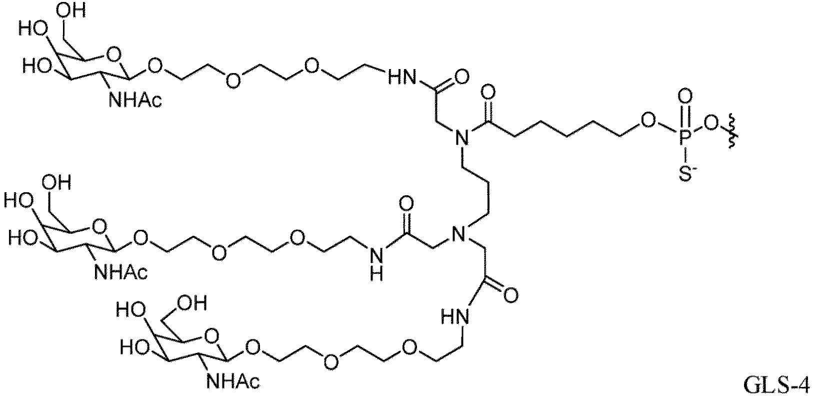

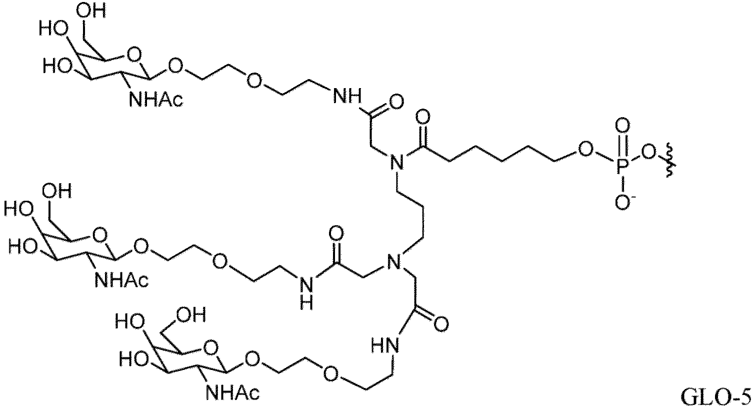

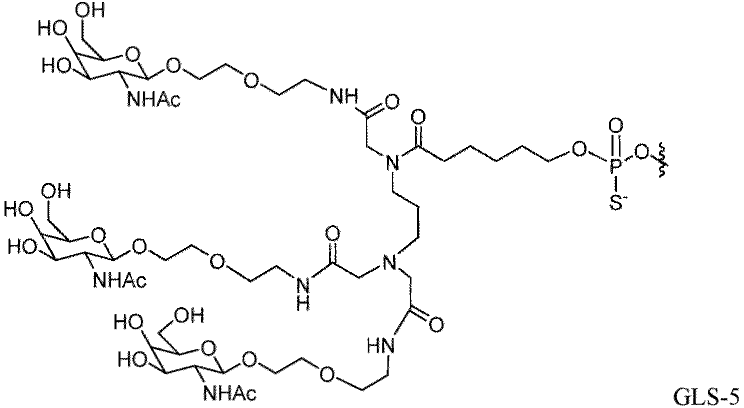

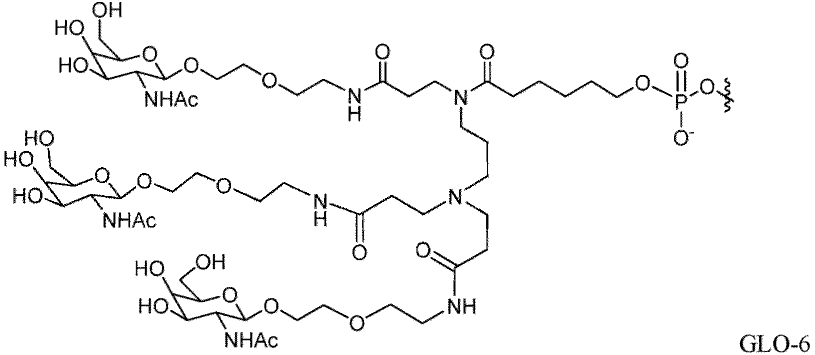

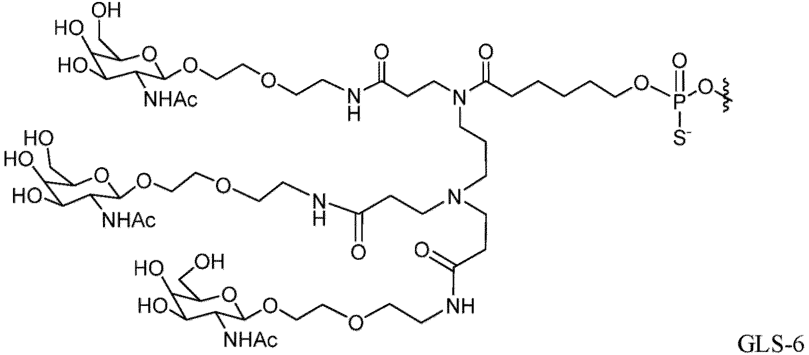

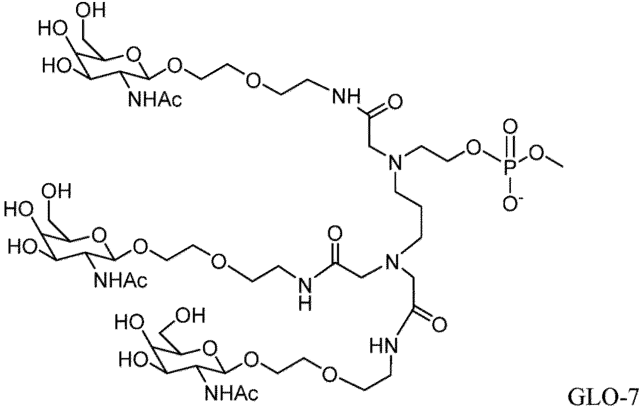

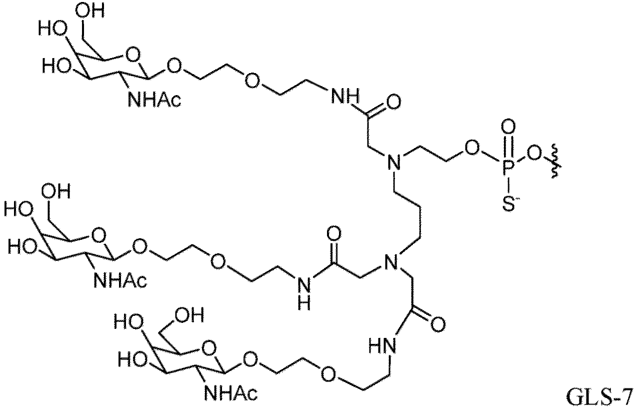

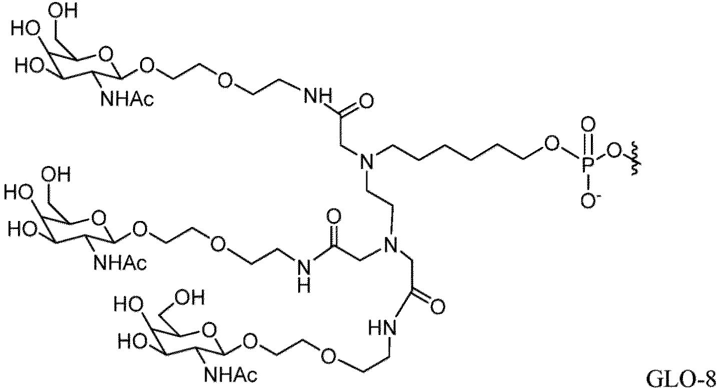

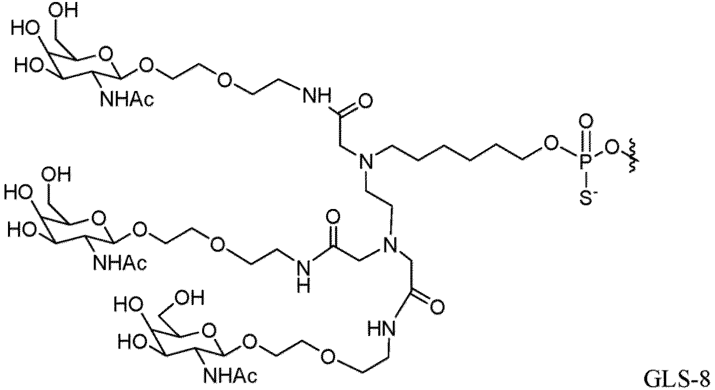

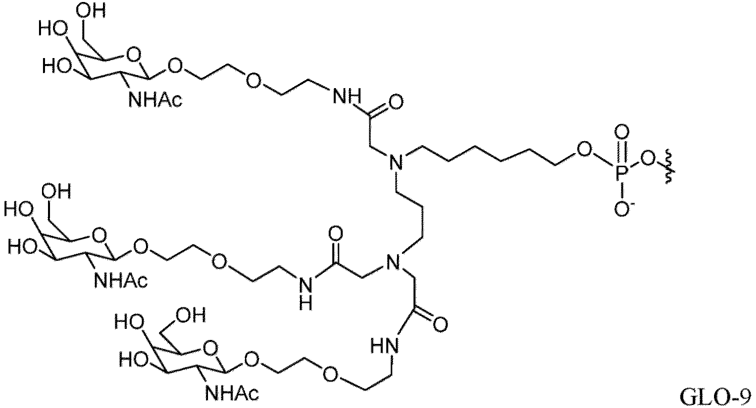

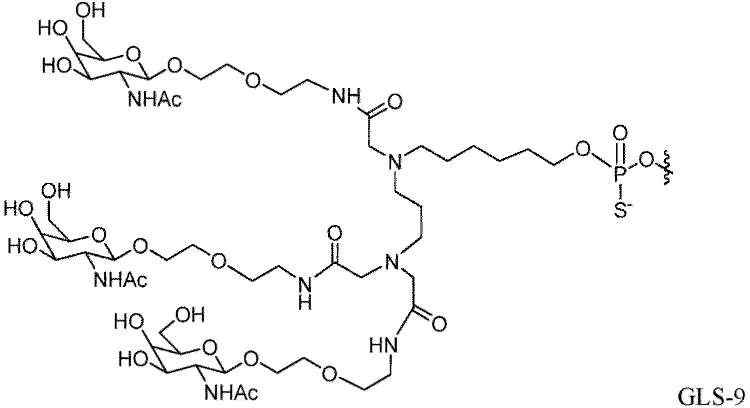

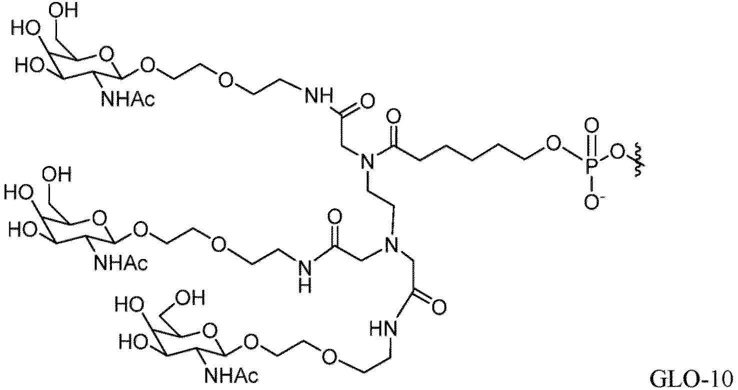

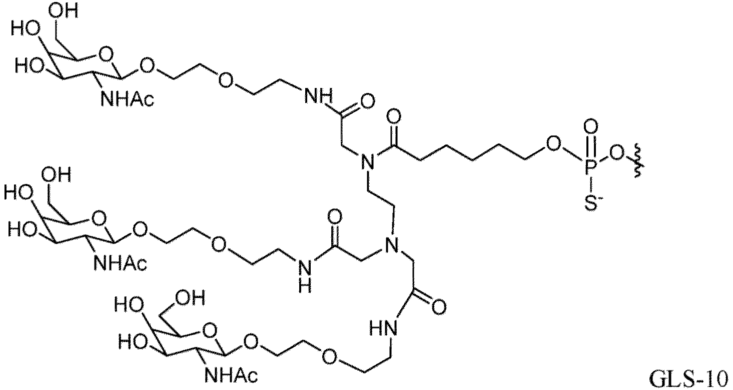

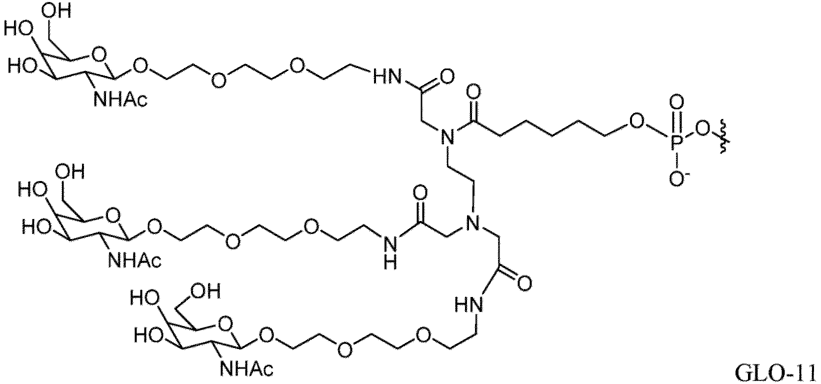

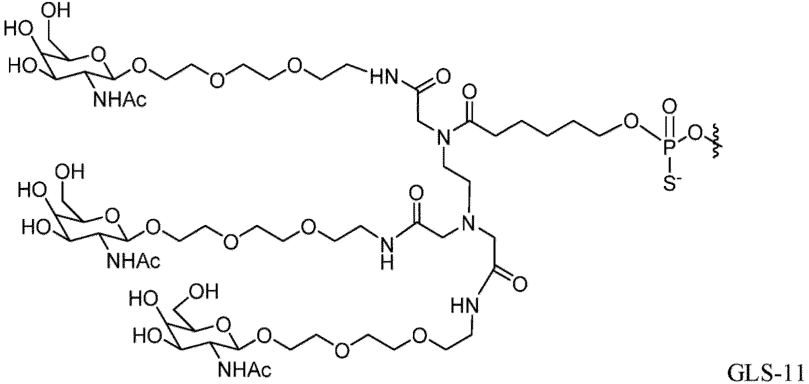

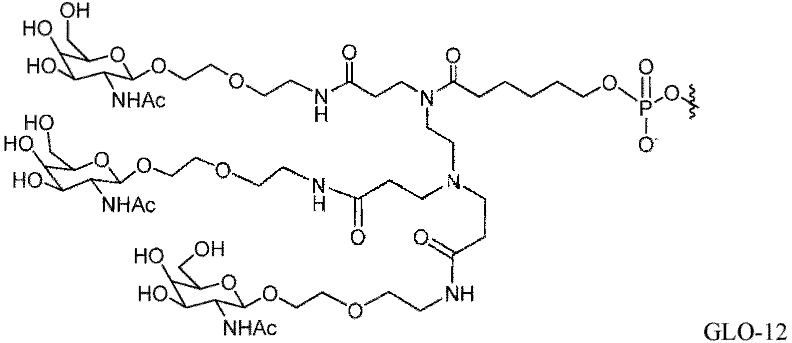

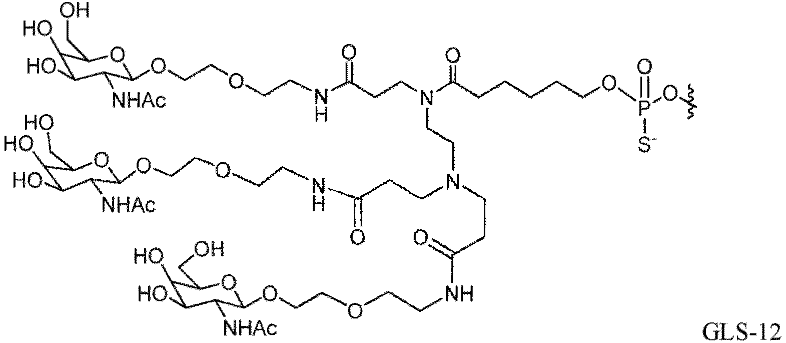

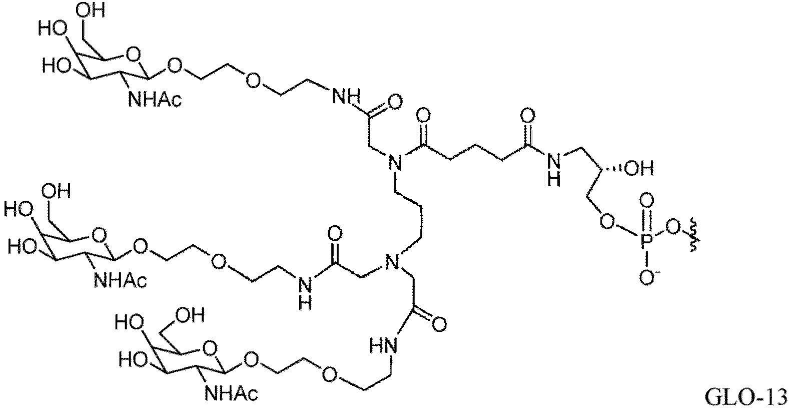

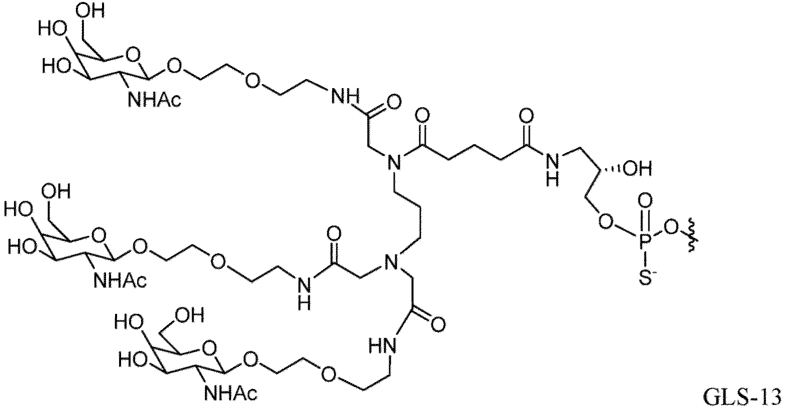

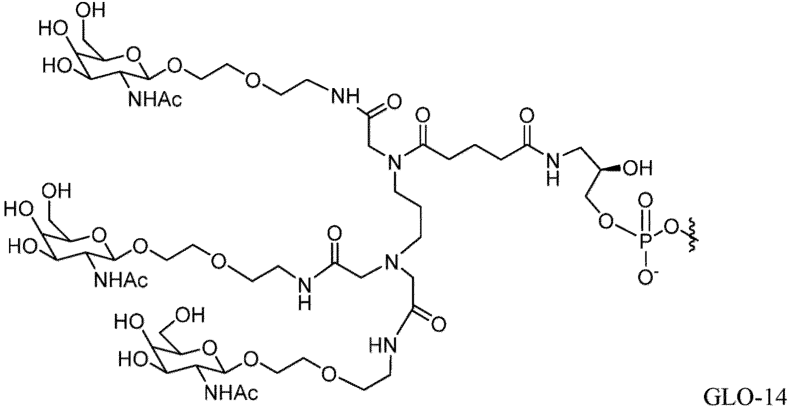

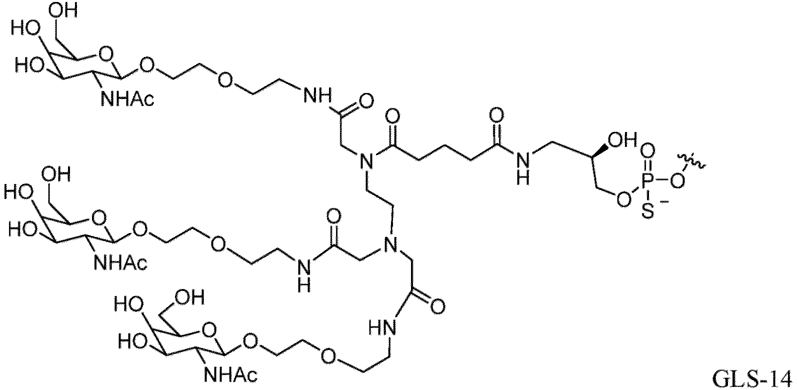

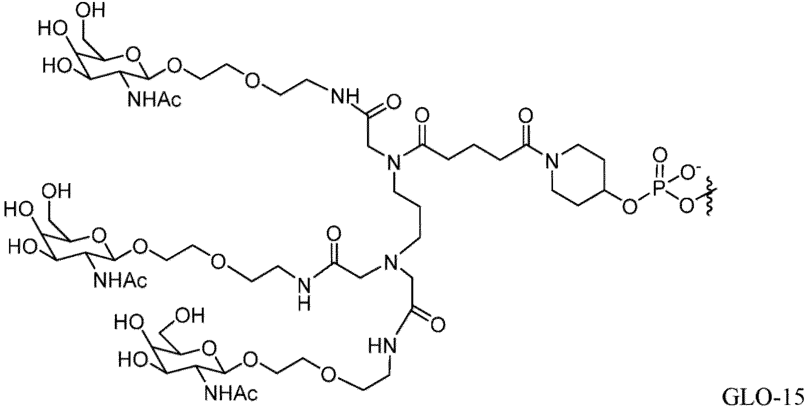

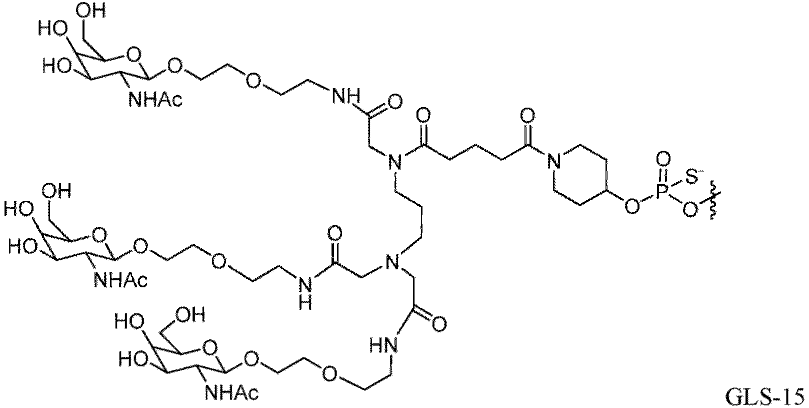

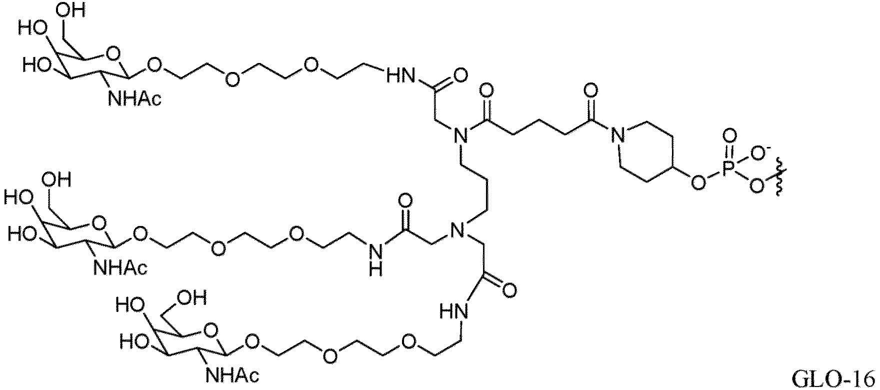

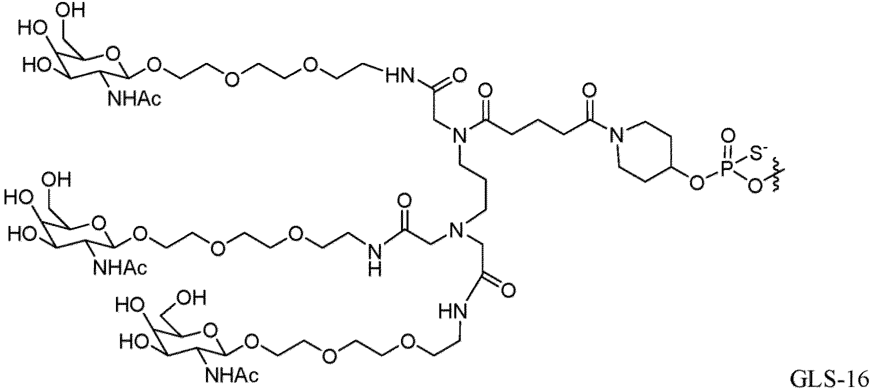

In some embodiments of the invention, *in vivo* delivery can also be by a beta-glucan delivery system, such as those described in U.S. Pat. Nos. 5,032,401 and 5,607,677, and U.S. Publication No. 2005/0281781, which are hereby incorporated by reference in their entirety. *In vitro* introduction of an HBV RNAi agent into a cell may also be done using art-known methods such as electroporation and lipofection. In certain embodiments of methods of the invention, an HBV dsRNA is delivered without a targeting agent. These RNAs may be delivered as "naked" RNA molecules. As a non-limiting example, an HBV dsRNA of the invention may be administered to a subject to treat an HBV-associated disease or condition in the subject, such as a liver disease, in a pharmaceutical composition comprising the RNAi agent, but not including a targeting agent such as a GalNAc targeting compound.

In addition to certain delivery means described herein, it will be understood that RNAi delivery means, such as but not limited to those described herein and those used in the art, can be used in conjunction with embodiments of HBV RNAi agents and treatment methods described herein.

HBV dsRNA agents of the invention may be administered to a subject in an amount and manner effective to reduce a level and activity of HBV polypeptide in a cell and/or subject. In some embodiments of methods of the invention one or more HBV dsRNA agents are administered to a cell and/or subject to treat a disease or condition associated with HBV expression and activity. Methods of the invention, in some embodiments, include administering one or more independently selected HBV dsRNA agents to a subject in need of such treatment to reduce a disease or condition associated with HBV expression in the subject. HBV dsRNA agents or HBV antisense polynucleotide agents of the invention can be administered to reduce HBV expression and/or activity in one more of *in vitro, ex vivo,* and *in vivo* cells.

In some embodiments of the invention, a level, and thus an activity, of HBV polypeptide, and thus a function of the virus, in a cell is reduced by delivering (e.g. introducing) an HBV dsRNA agent into a cell. Targeting agents and methods may be used to aid in delivery of an HBV dsRNA agent to a specific cell type, cell subtype, organ, spatial region within a subject, and/or to a sub-cellular region within a cell. An HBV dsRNA agent can be administered in certain methods of the invention singly or in combination with one or more additional HBV dsRNA agents. In some embodiments 2, 3, 4, or more independently selected HBV dsRNA agents are administered to a subject. In some embodiments of compositions and methods of the invention, comprise two, three, or more independently selected therapeutic molecules. In some embodiments of methods of the invention, two, three, four, or more HBV siRNAs targeting one, two, or more different positions/regions of HBV mRNA are administered to a cell and/or a subject. In such instances a composition of the invention may comprise two, three, four, or more independently selected HBV siRNAs each conjugated to an independently selected delivery compound. Certain embodiments of agents and/or compositions of the invention comprise divalent siRNA compounds (also referred to herein as linked siRNA compounds), which comprise two independently selected HBV siRNAs that are both conjugated to the same delivery compound.

In certain embodiments of the invention, an HBV dsRNA agent is administered to a subject to treat an HBV-associated disease or condition in conjunction with one or more additional therapeutic regimens for treating the HBV-associate disease or condition. Such combinations may result in a synergistic effect. Non-limiting examples of additional therapeutic regimens are: administering one or more HBV antisense polynucleotides of the invention, administering a non-HBV dsRNA therapeutic agent, and a behavioral modification. An additional therapeutic regimen may be administered at a time that is one or more of: prior to, simultaneous with, and following administration of an HBV dsRNA agent of the invention. It will be understood that simultaneous with as used herein, within five minutes of time zero, within 10 minutes of time zero, within 30 minutes of time zero, within 45 minutes of time zero, and within 60 minutes of time zero, with "time zero" the time of administration of the HBV dsRNA agent of the invention to the subject.

In a non-limiting example, in some embodiments of the invention, an additional therapeutic regimen administered to a subject includes one or more of: one or more HBV antisense polynucleotides, one or more additional HBV dsRNA therapeutic agents, one or more non-HBV dsRNA and/or siRNA therapeutic agent, and a behavioral modification. A non-limiting example of non-HBV dsRNA therapeutic regimen comprises administering to the subject one or more of: an antiviral agent; an antiviral nucleoside or nucleotide analogue (NUC), non-limiting examples of which are: Tenofovir disoproxil fumarate (TDF), Tenofovir alafenamide, Lamivudine, Adefovir dipivoxil, Entecavir (ETV), and Telbivudine; a viral polymerase inhibitor; a reverse transcriptase inhibitor; an immune stimulator; a therapeutic vaccine; a viral entry inhibitor; an oligonucleotide that inhibits the secretion or release of HBsAg; a capsid inhibitor; a covalently closed circular (ccc) HBV DNA inhibitor, and other therapeutic agents and/or procedures such as but not limited to: a liver transplant and chemotherapy. Non-limiting examples of an immune stimulator that may be used in a method of the invention are: pegylated interferon alfa 2a (PEG-IFN-a2a), Interferon alfa-2b, a recombinant human interleukin-7, and a Toll-like receptor 7 (TLR7) agonist, and a checkpoint inhibitor (e.g., PD1 inhibitor).

Non-limiting examples of behavioral modifications are: a dietary regimen, a rest regimen, an isolation regimen, and counseling. These and other therapeutic agents and behavior modifications are known in the art and used to treat an HBV-associated disease or condition in a subject and may be administered to a subject in combination with the administration of one or more HBV dsRNA agents of the invention to treat the HBV-associated disease or condition. An HBV dsRNA agent of the invention administered to a cell or subject to treat an HBV-associated disease or condition may act in a synergistic manner with one or more other therapeutic agents or activities and increase the effectiveness of the one or more therapeutic agents or activities and/or to increase the effectiveness of the HBV dsRNA agent at treating the HBV-associated disease or condition.

Treatment methods of the invention that include administration of an HBV dsRNA agent can be used in an asymptomatic subject infected with HBV and/or when one or more symptoms of an HBV-associated disease or condition is present, including at an early stage, mid-stage, and late stage of the disease or condition and all times before and after any of these stages. Methods of the invention may also be to treat subjects who have previously been treated for an HBV-associated disease or condition with one or more other therapeutic agents and/or therapeutic activities that were not successful, were minimally successful, and/or are no longer successful at treating the HBV-associated disease or condition in the subject.

### Vector Encoded dsRNAs

In certain embodiments of the invention, an HBV dsRNA agent can be delivered into a cell using a vector. HBV dsRNA agent transcription units can be included in a DNA or RNA vector. Prepare and use of such vectors encoding transgenes for delivering sequences into a cell and or subject are well known in the art. Vectors can be used in methods of the invention that result in transient expression of HBV dsRNA, for example for at least 1, 2, 3, 4, 5, 6, 7, 8, 9, 10 or more hours, 1, 2, 3, 4, 5, 6, 7, 8, 9, 10 or more weeks. The length of the transient expression can be determined using routine methods based on elements such as, but not limited to the specific vector construct selected and the target cell and/or tissue. Such transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

An individual strand or strands of an HBV dsRNA agent can be transcribed from a promoter on an expression vector. Where two separate strands are to be expressed to generate, for example, a dsRNA, two separate expression vectors can be co-introduced to a cell using means such as transfection or infection. In certain embodiments each individual strand of an HBV dsRNA agent of the invention can be transcribed by promoters that are both included on the same expression vector. In certain embodiments of the invention an HBV dsRNA agent is expressed as inverted repeat polynucleotides joined by a linker polynucleotide sequence such that the HBV dsRNA agent has a stem and loop structure.

Non-limiting examples of RNA expression vectors are DNA plasmids or viral vectors. Expression vectors useful in embodiments of the invention can be compatible with eukaryotic cells. Eukaryotic cell expression vectors are routinely used in the art and are available from a number of commercial sources. Delivery of HBV dsRNA expressing vectors can be systemic, such as by intravenous or intramuscular administration, by administration to target cells ex-planted from a subject followed by reintroduction into the subject, or by any other means that allows for introduction into a desired target cell.

Viral vector systems that may be included in an embodiment of a method of the include, but are not limited to, (a) adenovirus vectors; (b) retrovirus vectors, including but not limited to lentiviral vectors, moloney murine leukemia virus, etc.; (c) adeno-associated virus vectors; (d) herpes simplex virus vectors; (e) SV 40 vectors; (f) polyoma virus vectors; (g) papilloma virus vectors; (h) picornavirus vectors; (i) pox virus vectors such as an orthopox, e.g., vaccinia virus vectors or avipox, e.g. canary pox or fowl pox; and (j) a helper-dependent or gutless adenovirus. Constructs for the recombinant expression of an HBV dsRNA agent may include regulatory elements, such as promoters, enhancers, etc., which may be selected to provide constitutive or regulated/inducible expression. Viral vector systems, and the use of promoters and enhancers, etc. are routine in the art and can be used in conjunction with methods and compositions described herein.

Certain embodiments of the invention include use of viral vectors for delivery of HBV dsRNA agents into cells. Numerous adenovirus-based delivery systems are routinely used in the art for deliver to, for example, lung, liver, the central nervous system, endothelial cells, and muscle. Non-limiting examples of viral vectors that may be used in methods of the invention are: AAV vectors, a pox virus such as a vaccinia virus, a Modified Virus Ankara (MVA), NYVAC, an avipox such as fowl pox or canary pox.

Certain embodiments of the invention include methods of delivering HBV dsRNA agents into cells using a vector and such vectors may be in a pharmaceutically acceptable carrier that may, but need not, include a slow release matrix in which the gene delivery vehicle is imbedded. In some embodiments, a vector for delivering an HBV dsRNA can be produced from a recombinant cell, and a pharmaceutical composition of the invention may include one or more cells that produced the HBV dsRNA delivery system.

### Pharmaceutical Compositions Containing HBV dsRNA

Certain embodiments of the invention include use of pharmaceutical compositions containing an HBV dsRNA agent and a pharmaceutically acceptable carrier. The pharmaceutical composition containing the HBV dsRNA agent can be used in methods of the invention to reduce HBV gene expression and HBV activity in a cell and is useful to treat an HBV-associated disease or condition. Such pharmaceutical compositions can be formulated based on the mode of delivery. Non-limiting examples of formulations for modes of delivery are: a composition formulated for subcutaneous delivery, a composition formulated for systemic administration via parenteral delivery, a composition formulated for intravenous (IV) delivery, a composition formulated for intrathecal delivery, a composition formulated for direct delivery into brain, etc. Administration of a pharmaceutic composition of the invention to deliver an HBV dsRNA agent into a cell may be done using one or more means such as: topical (e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal or intramuscular injection or infusion; subdermal, e.g., via an implanted device; or intracranial, e.g., by intraparenchymal, intrathecal or intraventricular, administration. An HBV dsRNA agent can also be delivered directly to a target tissue, for example directly into the liver, directly into a kidney, etc. It will be understood that "delivering an HBV dsRNA agent" into a cell encompasses delivering an HBV dsRNA agent or HBV antisense polynucleotide agent, respectively, directly as well as expressing an HBV dsRNA agent in a cell from an encoding vector that is delivered into a cell, or by any suitable means with which the HBV dsRNA becomes present in a cell. Preparation and use of formulations and means for delivering inhibitory RNAs are well known and routinely used in the art.

As used herein, a "pharmaceutical composition" comprises a pharmacologically effective amount of an HBV dsRNA agent of the invention and a pharmaceutically acceptable carrier. The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract. Agents included in drug formulations are described further herein below.

As used herein terms such as: "pharmacologically effective amount," "therapeutically effective amount" and "effective amount" refers to that amount of an HBV dsRNA agent of the invention to produce the intended pharmacological, therapeutic or preventive result. For example, if a given clinical treatment is considered effective when there is at least a 10% reduction in a measurable parameter associated with a disease or disorder, a therapeutically effective amount of a drug for the treatment of that disease or disorder is the amount necessary to effect at least a 10% reduction in that parameter. For example, a therapeutically effective amount of an HBV dsRNA agent can reduce HBV polypeptide levels by at least 10%.

### Effective amounts

Methods of the invention, in some aspects comprise contacting a cell with an HBV dsRNA agent in an effective amount to reduce HBV gene expression in the contacted cell. Certain embodiments of methods of the invention comprise administering an HBV dsRNA agent or an HBV antisense polynucleotide agent to a subject in an amount effective to reduce HBV gene expression and treat an HBV-associated disease or condition in the subject. An "effective amount" used in terms of reducing expression of HBV and/or for treating an HBV-associated disease or condition, is an amount necessary or sufficient to realize a desired biologic effect. For example, an effective amount of an HBV dsRNA agent to treat an HBV-associated disease or condition could be that amount necessary to (i) slow or halt progression of the disease or condition; or (ii) reverse, reduce, or eliminate one or more symptoms of the disease or condition. In some aspects of the invention, an effective amount is that amount of an HBV dsRNA agent that when administered to a subject in need of a treatment of an HBV-associated disease or condition, results in a therapeutic response that prevents and/or treats the disease or condition. According to some aspects of the invention, an effective amount is that amount of an HBV dsRNA agent of the invention that when combined or co-administered with another therapeutic treatment for an HBV-associated disease or condition, results in a therapeutic response that prevents and/or treats the disease or condition. In some embodiments of the invention, a biologic effect of treating a subject with an HBV dsRNA agent of the invention may be the amelioration and or absolute elimination of symptoms resulting from the HBV-associated disease or condition. In some embodiments of the invention, a biologic effect is the complete abrogation of the HBV-associated disease or condition, as evidenced for example, by a diagnostic test that indicates the subject is free of the HBV-associated disease or condition. Additional art-known means of assessing the status of an HBV-associated disease or condition can be used to determine an effect of an agent and/or methods of the invention on an HBV-associated disease or condition.

Typically an effective amount of an HBV dsRNA agent to decrease HBV polypeptide activity to a level to treat an HBV-associated disease or condition will be determined in clinical trials, establishing an effective dose for a test population versus a control population in a blind study. In some embodiments, an effective amount will be that results in a desired response, e.g., an amount that diminishes an HBV-associated disease or condition in cells, tissues, and/or subjects with the disease or condition. Thus, an effective amount of an HBV dsRNA agent to treat an HBV-associated disease or condition that can be treated by reducing HBV polypeptide activity may be the amount that when administered decreases the amount of HBV polypeptide activity in the subject to an amount that is less than the amount that would be present in the cell, tissue, and/or subject without the administration of the HBV dsRNA agent or HBV antisense polynucleotide agent. In certain aspects of the invention the level of HBV polypeptide activity, and/or HBV gene expression present in a cell, tissue, and/or subject that has not been contacted with or administered an HBV dsRNA agent of the invention is referred to as a "control" amount. In some embodiments of methods of the invention a control amount for a subject is a pre-treatment amount for the subject, in other words, a level in a subject before administration of an HBV agent can be a control level for that subject and compared to a level of HBV polypeptide activity and/or HBV gene expression in the subject following siRNA administered to the subject. In the case of treating an HBV-associated disease or condition the desired response may be reducing or eliminating one or more symptoms of the disease or condition in the cell, tissue, and/or subject. The reduction or elimination may be temporary or may be permanent. It will be understood that the status of an HBV-associated disease or condition can be monitored using methods of determining HBV polypeptide activity, HBV gene expression, symptom evaluation, clinical testing, etc. In some aspects of the invention, a desired response to treatment of an HBV-associated disease or condition is delaying the onset or even preventing the onset of the disease or condition.

An effective amount of a compound that decreases HBV polypeptide activity may also be determined by assessing physiological effects of administration of an HBV dsRNA agent on a cell or subject, such as a decrease of an HBV-associated disease or condition following administration. Assays and/or symptomatic monitoring of a subject can be used to determine efficacy of an HBV dsRNA agent of the invention, which may be administered in a pharmaceutical compound of the invention, and to determine the presence or absence of a response to the treatment. A non-limiting example, is that one or more art-known tests of serum profiles. Another non-limiting example, is that one or more art-known tests of liver function can be used to determine the status of the HBV-associated liver disease or condition in a subject before and after treatment of the subject with an HBV dsRNA agent of the invention. In another non-limiting example, one or more art-known tests of HBV viral load level are used to determine the status of an HBV-associated disease in a subject. In this example the disease includes a level of HBV viral load and the tests are used to determine an HBV viral load level in the subject before and after treatment of the subject with an HBV dsRNA agent of the invention.

Some embodiments of the invention include methods of determining an efficacy of an dsRNA agent of the invention administered to a subject, to treat an HBV-associated disease or condition by assessing and/or monitoring one or more "physiological characteristics" of the HBV-associated disease or condition in the subject. Non-limiting examples of physiological characteristics of an HBV-associated disease or condition are level of alanine aminotransferase (ALT) in a subject, a level of aspartate aminotransferase (AST) in a subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject, and one or more physical symptoms such as but not limited to: pain, fever, etc. Routine means of determining such physiological characteristic are known in the art and include, but are not limited to, blood tests, serum analysis, tissue biopsy, imaging studies, physical examination, etc.

It will be understood that the amount of an HBV dsRNA agent administered to a subject can be modified based, at least in part, on such determinations of disease and/or condition status and/or physiological characteristics determined for a subject. The amount of a treatment may be varied for example by increasing or decreasing the amount of an HBV-dsRNA agent or HBV antisense polynucleotide agent, by changing the composition in which the HBV dsRNA agent or HBV antisense polynucleotide agent, respectively, is administered, by changing the route of administration, by changing the dosage timing and so on. The effective amount of an HBV dsRNA agent will vary with the particular condition being treated, the age and physical condition of the subject being treated; the severity of the condition, the duration of the treatment, the nature of the concurrent therapy (if any), the specific route of administration, and additional factors within the knowledge and expertise of the health practitioner. For example, an effective amount may depend upon the desired level of HBV polypeptide activity and or HBV gene expression that is effective to treat the HBV-associated disease or condition. A skilled artisan can empirically determine an effective amount of a particular HBV dsRNA agent of the invention for use in methods of the invention without necessitating undue experimentation. Combined with the teachings provided herein, by selecting from among various HBV dsRNA agents or HBV antisense polynucleotide agents of the invention, and weighing factors such as potency, relative bioavailability, patient body weight, severity of adverse side-effects and preferred mode of administration, an effective prophylactic or therapeutic treatment regimen can be planned that is effective to treat the particular subject. As used in embodiments of the invention, an effective amount of an HBV dsRNA agent of the invention can be that amount that when contacted with a cell results in a desired biological effect in the cell.

It will be recognized that HBV gene silencing may be determined in any cell expressing HBV, either constitutively or by genomic engineering, and by any appropriate assay. In some embodiments of the invention, HBV gene expression is reduced by at least 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% by administration of an HBV dsRNA agent of the invention. In some embodiments of the invention, HBV gene expression is reduced by at between 5% and 10%, 5% and 25%, 10% and 50%, 10% and 75%, 25% and 75%, 25% and 100%, or 50% and 100% by administration of an HBV dsRNA agent of the invention.

### Dosing

HBV dsRNA agents and HBV antisense polynucleotide agents are delivered in pharmaceutical compositions in dosages sufficient to inhibit expression of HBV genes. In certain embodiments of the invention, a dose of HBV dsRNA agent is in a range of 0.01 to 200.0 milligrams per kilogram body weight of the recipient per day, generally in the range of 1 to 50 mg per kilogram body weight, 5 to 40 mg/kg body weight, 10 to 30 mg/kg body weight, 1 to 20 mg/kg body weight, 1 to 10 mg/kg body weight, 4 to 15 mg/kg body weight per day, inclusive. For example, the HBV dsRNA agent can be administered in an amount that is from about 0.01 mg/kg, 0.05 mg/kg, 0.1 mg/kg, 0.2 mg/kg, 0.3 mg/kg, 0.4 mg/kg, 0.5 mg/kg, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 11 mg/kg, 12 mg/kg, 13mg/kg, 14 mg/kg, 15 mg/kg, 16 mg/kg, 17 mg/kg, 18 mg/kg, 19 mg/kg, 20 mg/kg, 21 mg/kg, 22 mg/kg, 23mg/kg, 24 mg/kg, 25 mg/kg, 26 mg/kg, 27 mg/kg, 28 mg/kg, 29 mg/kg, 30 mg/kg, 31 mg/kg, 32 mg/kg, 33mg/kg, 34 mg/kg, 35 mg/kg, 36 mg/kg, 37 mg/kg, 38 mg/kg, 39 mg/kg, 40 mg/kg, 41 mg/kg, 42 mg/kg, 43mg/kg, 44 mg/kg, 45 mg/kg, 46 mg/kg, 47 mg/kg, 48 mg/kg, 49 mg/kg, through 50 mg/kg body per single dose.

Various factors may be considered in the determination of dosage and timing of delivery of an HBV dsRNA agent of the invention. The absolute amount of an HBV dsRNA agent delivered will depend upon a variety of factors including a concurrent treatment, the number of doses and the individual subject parameters including age, physical condition, size and weight. These are factors well known to those of ordinary skill in the art and can be addressed with no more than routine experimentation. In some embodiments, a maximum dose can be used, that is, the highest safe dose according to sound medical judgment.

Methods of the invention may in some embodiments include administering to a subject 1, 2, 3, 4, 5, 6, 7, 8, 9, 10, or more doses of an HBV dsRNA agent or HBV antisense polynucleotide agent. In some instances, a pharmaceutical compound, (e.g., comprising an HBV dsRNA agent or comprising an HBV antisense polynucleotide agent) can be administered to a subject at least daily, every other day, weekly, every other week, monthly, etc. Doses may be administered once per day or more than once per day, for example, 2, 3, 4, 5, or more times in one 24 hour period. A pharmaceutical composition of the invention may be administered once daily, or the HBV dsRNA agent may be administered as two, three, or more sub-doses at appropriate intervals throughout the day or even using continuous infusion or delivery through a controlled release formulation. In some embodiments of methods of the invention, a pharmaceutical composition of the invention is administered to a subject one or more times per day, one or more times per week, one or more times per month, or one or more times per year.

Methods of the invention, in some aspects, include administration of a pharmaceutical compound alone, in combination with one or more other HBV dsRNA agents or HBV antisense polynucleotide agents, and/or in combination with other drug therapies or treatment activities or regimens that are administered to subjects with an HBV-associated disease or condition. Pharmaceutical compounds may be administered in pharmaceutical compositions. Pharmaceutical compositions used in methods of the invention may be sterile and contain an amount of an HBV dsRNA agent that will reduce activity of an HBV polypeptide to a level sufficient to produce the desired response in a unit of weight or volume suitable for administration to a subject. A dose administered to a subject of a pharmaceutical composition that includes an HBV dsRNA agent to reduce HBV protein activity can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

### Treatment

HBV-associated diseases and conditions in which a decrease in a level and/or activity of HBV polypeptide is effective to treat the disease or condition, can be treated using methods and HBV dsRNA agents of the invention to inhibit HBV expression. Examples of diseases and conditions that may be treated with an HBV dsRNA agent of the invention and a treatment method of the invention, include, but are not limited to: hepatitis B, HBV infection, chronic hepatitis B, hepatitis D virus infection (delta hepatitis or HDV), liver injury, cirrhosis, acute hepatitis B, acute fulminant hepatitis, liver inflammation, liver fibrosis, and hepatocellular carcinoma. Such diseases and conditions may be referred to herein as "HBV-associated diseases and conditions" and "diseases and conditions caused and/or modulated by HBV." It will be understood that Hepatitis D virus (HDV) infection in a subject is an HBV-associated disease because although it does not infect patient by itself, HDV can co-infect a subject who is infected with HBV.

In certain aspects of the invention, a subject may be administered an HBV dsRNA agent of the invention at a time that is one or more of before or after diagnosis of an HBV-associated disease or condition. In some aspects of the invention, a subject is at risk of having or developing an HBV-associated disease or condition. A subject at risk of developing an HBV-associated disease or condition is one who has an increased probability of developing the HBV-associated disease or condition, compared to a control risk of developing the HBV-associated disease or condition. In some embodiments of the invention, a level of risk may be statistically significant compared to a control level of risk. A subject at risk may include, for instance, a subject who has been and/or is at risk of being (1) in close contact with an HBV carrying subject, (2) has been and/or is at risk of being in contact with blood, semen, and/or vaginal fluid of a subject infected with HBV, (3) has a preexisting disease and/or abnormality such as, but not limited to an immune deficiency that makes the subject more susceptible to an HBV infection and resulting HBV-associated disease or condition than a control subject without the preexisting disease or abnormality; (4) a subject having a personal medical history of the HBV-associated disease or condition; (5) and a subject who has previously been treated for an HBV-associated disease or condition. It will be understood that a preexisting disease and/or a abnormality that makes the subject more susceptible to an HBV-associated disease or condition, may be a disease or abnormality that when present has been previously identified as having a correlative relation to a higher likelihood of developing an HBV-associated disease or condition.

It will be understood that an HBV dsRNA agent may be administered to a subject based on a medical status of the individual subject. For example, a health-care provided for a subject may assess an ALT level measured in a sample obtained from a subject and determine it is desirable to reduce the subject's ALT level, by administration of an HBV dsRNA agent of the invention. In this example, the ALT level may be considered to be a physiological characteristic of an HBV-associated condition, even if the subject is not diagnosed as having an HBV-associated disease such as one disclosed herein. A healthcare provider may monitor changes in the subject's ALT level, as a measure of efficacy of the administered HBV dsRNA agent of the invention. In a non-limiting example, a biological sample, such as a blood or serum sample is obtained from a subject and an HBV viral load for the subject is determined in the sample. Upon a finding of the presence of an HBV viral load in the subject, an HBV dsRNA agent is administered to the subject and a subsequent blood or serum sample is obtained from the subject following the administration and the HBV viral load determined using the subsequent sample and the results compared to the results determined in the subject's pre-administration (prior) sample. A reduction in the subject's HBV viral load level in the later sample compared to the pre-administration level indicates the administered HBV dsRNA agent efficacy in reducing the HBV viral load level in the subject.

Certain embodiments of methods of the invention include adjusting a treatment that includes administering an HBV dsRNA agent or an HBV antisense polynucleotide agent of the invention to a subject based at least in part on assessment of a change in one or more of the subject's physiological characteristics of an HBV-associated disease or condition resulting from the treatment. For example, in some embodiments of the invention, an effect of an administered dsRNA agent of the invention may be determined for a subject and used to assist in adjusting an amount of a dsRNA agent of the invention subsequently administered to the subject. In a non-limiting example, a subject is administered a dsRNA agent of the invention, the presence and/or level of one or more HBV viral antigens is determined in a biological sample obtained from the subject after the administration, and based at least in part on the determined level, a greater amount of the dsRNA agent is determined to be desirable in order to increase the physiological effect of the administered agent, for example to reduce or further reduce the subject's presence and/or level of the one or more HBV viral antigens. In another non-limiting example, a subject is administered a dsRNA agent of the invention, the subject's HBV viral load is determined after the administration and based at least in part on the determined HBV viral load, a lower amount, higher amount, or same amount of the dsRNA agent is desirable to administer to the subject.

Thus, some embodiments of the invention include assessing a change in one or more physiological characteristics of resulting from a subject's previous treatment to adjust an amount of a dsRNA agent of the invention subsequently administered to the subject. Some embodiments of methods of the invention include 1, 2, 3, 4, 5, 6, or more determinations of a physiological characteristic of an HBV-associated disease or condition to assess and/or monitor the efficacy of an administered HBV dsRNA agent of the invention, and optionally using the determinations to adjust one or more of: a dose, administration regimen, and or administration frequency of a dsRNA agent of the invention to treat an HBV-associated disease or condition in a subject. In some embodiments of methods of the invention, a desired result of administering an effective amount of a dsRNA agent of the invention to a subject is a reduction in one or more of the ALT; AST; HBV viral load, HBV covalently closed circular DNA (cccDNA); one or more HBV antigens; HBcAg; HBsAg; and HBeAg in the subject indicates a reduction of HBV gene expression in the subject. In certain embodiments of methods of the invention, a desired result of administering an effective amount of a HBV dsRNA agent of HBV antisense polynucleotide agent of the invention to a subject is an increase in anti-Hepatitis B virus antibodies in the subject compared to a baseline pre-treatment physiological characteristic of the HBV-associated disease or condition and/or to a control physiological characteristic of the HBV-associated disease or condition, indicates a reduction in HBV gene expression in the subject.

As used herein, the terms "treat", "treated", or "treating" when used with respect to an HBV-associated disease or condition may refer to a prophylactic treatment that decreases the likelihood of a subject developing the HBV-associated disease or condition, and also may refer to a treatment after the subject has developed an HBV-associated disease or condition in order to eliminate or reduce the level of the HBV-associated disease or condition, prevent the HBV-associated disease or condition from becoming more advanced (e.g., more severe), and/or slow the progression of the HBV-associated disease or condition in a subject compared to the subject in the absence of the therapy to reduce activity in the subject of HBV polypeptide.

Certain embodiments of agents, compositions, and methods of the invention can be used to inhibit HBV gene expression. As used herein in reference to expression of an HBV gene, the terms "inhibit," "silence," "reduce," "down-regulate," and "knockdown" mean the expression of the HBV gene, as measured by one or more of: a level of RNA transcribed from the gene, a level of activity of HBV expressed, and a level of HBV polypeptide, protein or protein subunit translated from the mRNA in a cell, group of cells, tissue, organ, or subject in which the HBV gene is transcribed, is reduced when the cell, group of cells, tissue, organ, or subject is contacted with (e.g., treated with) an HBV dsRNA agent of the invention, compared to a control level of RNA transcribed from the HBV gene, a level of activity of expressed HBV, or a level of HBV translated from the mRNA, respectively. In some embodiments, a control level is a level in a cell, tissue, organ or subject that has not been contacted with (e.g. treated with) the HBV dsRNA agent or HBV antisense polynucleotide agent.

### Administration methods

A variety of administration routes for an HBV dsRNA agent are available for use in methods of the invention. The particular delivery mode selected will depend at least in part, upon the particular condition being treated and the dosage required for therapeutic efficacy. Methods of this invention, generally speaking, may be practiced using any mode of administration that is medically acceptable, meaning any mode that produces effective levels of treatment of an HBV-associated disease or condition without causing clinically unacceptable adverse effects. In some embodiments of the invention, an HBV dsRNA agent may be administered via an oral, enteral, mucosal, subcutaneous, and/or parenteral route. The term "parenteral" includes subcutaneous, intravenous, intrathecal, intramuscular, intraperitoneal, and intrasternal injection, or infusion techniques. Other routes include but are not limited to nasal (e.g., via a gastro-nasal tube), dermal, vaginal, rectal, sublingual, and inhalation. Delivery routes of the invention may include intrathecal, intraventricular, or intracranial. In some embodiments of the invention, an HBV dsRNA agent may be placed within a slow release matrix and administered by placement of the matrix in the subject. In some aspects of the invention, an HBV dsRNA agent may be delivered to a subject cell using nanoparticles coated with a delivery agent that targets a specific cell or organelle. Various delivery means, methods, agents are known in the art. Non-limiting examples of delivery methods and delivery agents are additionally provided elsewhere herein. In some aspects of the invention, the term "delivering" in reference to an HBV dsRNA agent may mean administration to a cell or subject of one or more "naked" HBV dsRNA agent sequences and in certain aspects of the invention "delivering" means administration to a cell or subject via transfection means, delivering a cell comprising an HBV dsRNA agent to a subject, delivering a vector encoding an HBV dsRNA agent into a cell and/or subject, etc. Delivery of an HBV dsRNA agent using a transfection means may include administration of a vector to a cell and/or subject.

In some methods of the invention one or more HBV dsRNA agents or HBV antisense polynucleotide agents may be administered in formulations, which may be administered in pharmaceutically acceptable solutions, which may routinely contain pharmaceutically acceptable concentrations of salt, buffering agents, preservatives, compatible carriers, adjuvants, and optionally other therapeutic ingredients. In some embodiments of the invention an HBV dsRNA agent may be formulated with another therapeutic agent for simultaneous administration. According to methods of the invention, an HBV dsRNA agent may be administered in a pharmaceutical composition. In general, a pharmaceutical composition comprises an HBV dsRNA agent and optionally, a pharmaceutically-acceptable carrier. Pharmaceutically-acceptable carriers are well-known to those of ordinary skill in the art. As used herein, a pharmaceutically-acceptable carrier means a non-toxic material that does not interfere with the effectiveness of the biological activity of the active ingredients, e.g., the ability of the HBV dsRNA agent to inhibit HBV gene expression in a cell or subject. Numerous methods to administer and deliver dsRNA agents or HBV antisense polynucleotide agents for therapeutic use are known in the art and may be utilized in methods of the invention.

Pharmaceutically acceptable carriers include diluents, fillers, salts, buffers, stabilizers, solubilizers and other materials that are well-known in the art. Exemplary pharmaceutically acceptable carriers are described in U.S. Pat. No. 5,211,657 and others are known by those skilled in the art. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

Some embodiments of methods of the invention include administering one or more HBV dsRNA agents or HBV antisense polynucleotide agents directly to a tissue. In some embodiments, the tissue to which the compound is administered is a tissue in which the HBV-associated disease or condition is present or is likely to arise, non-limiting examples of which are the liver or kidney. Direct tissue administration may be achieved by direct injection or other means. Many orally delivered compounds naturally travel to and through the liver and kidneys and some embodiments of treatment methods of the invention include oral administration of one or more HBV dsRNA agents to a subject. HBV dsRNA agents or HBV antisense polynucleotide agents, either alone or in conjunction with other therapeutic agents, may be administered once, or alternatively they may be administered in a plurality of administrations. If administered multiple times, the HBV dsRNA agent may be administered via different routes. For example, though not intended to be limiting, a first (or first several) administrations may be made via subcutaneous means and one or more additional administrations may be oral and/or systemic administrations.

For embodiments of the invention in which it is desirable to administer an HBV dsRNA agent systemically, the HBV dsRNA agent may be formulated for parenteral administration by injection, e.g., by bolus injection or continuous infusion. Formulations for injection may be presented in unit dosage form, e.g., in ampoules or in multi-dose containers, with or without an added preservative. HBV dsRNA agent formulations (also referred to as pharmaceutical compositions) may take such forms as suspensions, solutions or emulsions in oily or aqueous vehicles, and may contain formulatory agents such as suspending, stabilizing and/or dispersing agents.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day may be used as needed to achieve appropriate systemic or local levels of one or more HBV dsRNA agents or HBV antisense polynucleotide agents and to achieve appropriate reduction in HBV protein activity.

In yet other embodiments, methods of the invention include use of a delivery vehicle such as biocompatible microparticle, nanoparticle, or implant suitable for implantation into a recipient, e.g., a subject. Exemplary bioerodible implants that may be useful in accordance with this method are described in PCT Publication No. WO 95/24929 (incorporated by reference herein), which describes a biocompatible, biodegradable polymeric matrix for containing a biological macromolecule.

Both non-biodegradable and biodegradable polymeric matrices can be used in methods of the invention to deliver one or more HBV dsRNA agents or HBV antisense polynucleotide agents to a subject. In some embodiments, a matrix may be biodegradable. Matrix polymers may be natural or synthetic polymers. A polymer can be selected based on the period of time over which release is desired, generally in the order of a few hours to a year or longer. Typically, release over a period ranging from between a few hours and three to twelve months can be used. The polymer optionally is in the form of a hydrogel that can absorb up to about 90% of its weight in water and further, optionally is cross-linked with multivalent ions or other polymers.

In general, HBV dsRNA agents or HBV antisense polynucleotide agents may be delivered in some embodiments of the invention using the bioerodible implant by way of diffusion, or by degradation of the polymeric matrix. Exemplary synthetic polymers for such use are well known in the art. Biodegradable polymers and non-biodegradable polymers can be used for delivery of HBV dsRNA agents or HBV antisense polynucleotide agents using art-known methods. Bioadhesive polymers such as bioerodible hydrogels (see H. S. Sawhney, C. P. Pathak and J. A. Hubell in Macromolecules, 1993, 26, 581-587, the teachings of which are incorporated by reference herein) may also be used to deliver HBV dsRNA agents or HBV antisense polynucleotide agents for treatment of an HBV-associated disease or condition. Additional suitable delivery systems can include time-release, delayed release or sustained release delivery systems. Such systems can avoid repeated administrations of an HBV dsRNA agent or HBV antisense polynucleotide agent, increasing convenience to the subject and the medical care professional. Many types of release delivery systems are available and known to those of ordinary skill in the art. (See for example: U.S. Pat. Nos. 5,075,109; 4,452,775; 4,675,189; 5,736,152; 3,854,480; 5,133,974; and 5,407,686 (the teaching of each of which is incorporated herein by reference). In addition, pump-based hardware delivery systems can be used, some of which are adapted for implantation.

Use of a long-term sustained release implant may be suitable for prophylactic treatment of subjects and for subjects at risk of developing a recurrent HBV-associated disease or condition. Long-term release, as used herein, means that the implant is constructed and arranged to deliver a therapeutic level of an HBV dsRNA agent for at least up to 10 days, 20 days, 30 days, 60 days, 90 days, six months, a year, or longer. Long-term sustained release implants are well-known to those of ordinary skill in the art and include some of the release systems described above.

Therapeutic formulations of HBV dsRNA agents or HBV antisense polynucleotide agents may be prepared for storage by mixing the molecule or compound having the desired degree of purity with optional pharmaceutically acceptable carriers, excipients or stabilizers [Remington's Pharmaceutical Sciences 21^{st} edition, (2006)], in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{®}, PLURONICS^{®} or polyethylene glycol (PEG).

### Cells, Subjects, and Controls

Methods of the invention may be used in conjunction with cells, tissues, organs and/or subjects. In some aspects of the invention a subject is a human or vertebrate mammal including but not limited to a dog, cat, horse, cow, goat, mouse, rat, and primate, e.g., monkey. Thus, the invention can be used to treat HBV-associated diseases or conditions in human and non-human subjects. In some aspects of the invention a subject may be a farm animal, a zoo animal, a domesticated animal or non-domesticated animal and methods of the invention can be used in veterinary prevention and treatment regimens. In some embodiments of the invention, the subject is a human and methods of the invention can be used in human prevention and treatment regimens.

Non-limiting examples of subjects to which the present invention can be applied are subjects who are diagnosed with, suspected of having, or at risk of having a disease or condition associated with a higher than desirable HBV expression and/or activity, also referred to as "elevated levels of HBV expression". Non-limiting examples of diseases and conditions associated with a higher than desirable levels of HBV expression and/or activity are described elsewhere herein. Methods of the invention may be applied to a subject who, at the time of treatment, has been diagnosed as having the disease or condition associated with a higher than desirable HBV expression and/or activity, or a subject who is considered to be at risk for having or developing a disease or condition associated with a higher than desirable HBV expression and/or activity. In some aspects of the invention a disease or condition associated with a higher than desirable HBV level of expression and/or activity is an acute disease or condition, and in certain aspects of the invention a disease or condition associated with a higher than desirable HBV level of expression and/or activity is a chronic disease or condition.

In a non-limiting example, an HBV dsRNA agent of the invention is administered to a subject diagnosed with, suspected of having, or at risk of having, acute hepatitis B, which is a disease in which it is desirable to reduce HBV expression. Methods of the invention may be applied to the subject who, at the time of treatment, has been diagnosed as having the disease or condition, or a subject who is considered to be at risk for having or developing the disease or condition.

In another non-limiting example, an HBV dsRNA agent of the invention is administered to a subject diagnosed with, suspected of having, or at risk of having, Hepatitis D (HDV), which is a disease in which it is desirable to reduce HBV expression. Methods of the invention may be applied to the subject who, at the time of treatment, has been diagnosed as having the disease or condition, or a subject who is considered to be at risk for having or developing the disease or condition.

A cell to which methods of the invention may be applied include cells that are *in vitro, in vivo*, *ex vivo* cells. Cells may be in a subject, in culture, and/or in suspension, or in any other suitable state or condition. A cell to which a method of the invention may be applied can be a liver cell, a hepatocyte, a cardiac cell, a pancreatic cell, a cardiovascular cell, kidney cell or other type of vertebrate cell, including human and non-human mammalian cells. In certain aspects of the invention, a cell to which methods of the invention may be applied is a healthy, normal cell that is not known to be a disease cell. In certain embodiments of the invention a cell to which methods and compositions of the invention are applied to a liver cell, a hepatocyte, a cardiac cell, a pancreatic cell, a cardiovascular cell, and/or a kidney cell. In certain aspects of the invention, a control cell is a normal cell, but it will be understood that a cell having a disease or condition may also serve as a control cell in particular circumstances for example to compare results in a treated cell having a disease or condition versus an untreated cell having the disease or condition, etc. A cell suitable for treatment using an embodiment of the invention can be a cell that expresses an HBV gene, including a cell infected with HBV, or a cell comprising an expression vector that includes an HBV genome or portion of ah HBV gene.

A level of HBV polypeptide activity can be determined and compared to control level of HBV polypeptide activity, according to methods of the invention. A control may be a predetermined value, which can take a variety of forms. It can be a single cut-off value, such as a median or mean. It can be established based upon comparative groups, such as in groups having lower levels of HBV polypeptide and/or HBV polypeptide activity and groups having increased levels of HBV polypeptide and/or HBV polypeptide activity. Another non-limiting example of comparative groups may be groups having one or more symptoms of or a diagnosis of an HBV-associated disease or condition; groups without having one or more symptoms of or a diagnosis of the disease or condition; groups of subjects to whom an siRNA treatment of the invention has been administered; groups of subjects to whom an siRNA treatment of the invention has not been administered. Typically, a control may be based on apparently healthy normal individuals in an appropriate age bracket or apparently healthy cells. It will be understood that controls according to the invention may be, in addition to predetermined values, samples of materials tested in parallel with the experimental materials. Examples include samples from control populations or control samples generated through manufacture to be tested in parallel with the experimental samples. In some embodiments of the invention, a control may include a cell or subject not contacted or treated with an HBV dsRNA agent of the invention and in such instances, a control level of HBV polypeptide and/or HBV polypeptide activity can be compared to a level of HBV polypeptide and/or HBV polypeptide activity in a cell or subject contacted with an HBV dsRNA agent of the invention.

In some embodiments of the invention a level of HBV polypeptide determined for a subject can be a control level against which a level of HBV polypeptide determined for the same subject at a different time is compared. In a non-limiting example, a level of HBV is determined in a biological sample obtained from a subject who has not been administered an HBV treatment of the invention. In some embodiments, the biological sample is a serum sample or a tissue sample. The level of HBV polypeptide determined in the sample obtained from the subject can serve as a baseline or control value for the subject. After one or more administrations of an HBV dsRNA agent to the subject in a treatment method of the invention, one or more additional serum samples can be obtained from the subject and the level of HBV polypeptide in the subsequent sample or samples can be compared to the control/baseline level for the subject. Such comparisons can be used to assess onset, progression, or recession of an HBV associated disease or condition in the subject. For example, a level of HBV polypeptide or level of HBV viral load, in the baseline sample obtained from the subject that is higher than a level obtained from the same subject after the subject has been administered an HBV dsRNA agent of the invention indicates regression of the HBV-associated disease or condition and indicates efficacy of the administered HBV dsRNA agent of the invention for treatment of the HBV-associated disease or condition. In some embodiments, a statistically significant decrease of a level of HBV polypeptide in a sample from a subject compared to a level determined in a previously obtained sample from the subject shows regression of the HBV-associated disease or condition.

In some aspects of the invention, values of one or more of a level of HBV polypeptide and/or HBV polypeptide activity determined for a subject may serve as control values for later comparison of levels of HBV polypeptide and/or HBV activity, in that same subject, thus permitting assessment of changes from a "baseline" HBV polypeptide activity in a subject. Thus, an initial HBV polypeptide level and/or initial HBV polypeptide activity level may be present and/or determined in a subject and methods and compounds of the invention may be used to decrease the level of HBV polypeptide and/or HBV polypeptide activity in the subject, with the initial level serving as a control level for that subject.

Using methods of the invention, HBV dsRNA agents of the invention may be administered to a subject. Efficacy of the administration and treatment of the invention can be assessed when a level of HBV polypeptide or HBV viral load in a serum sample and/or tissue sample obtained from a subject is decreased by at least 0.5%, 1%, 5%, 10%, 20%, 30%, 40%, 50%, 60%, 70%, 80%, 90%, 95%, 96%, 97%, 98%, 99%, or 100% compared to a pre-administration level of HBV polypeptide or HBV viral load in a serum sample and/or tissue sample obtained from the subject at a prior time point, or compared to a non-contacted control level, for example a level of HBV polypeptide or HBV viral load in a control serum sample. It will be understood that a level of HBV polypeptide, HBV viral load, the presence of one or more HBV antigens, a level of HBV polypeptide activity, and other physiological characteristics described herein correlate with a level of HBV gene expression. Certain embodiments of methods of the invention comprise administering an HBV dsRNA of the invention to a subject in an amount effective to inhibit HBV gene expression and thereby reduce a level of HBV polypeptide and reduce a level of HBV polypeptide activity in the subj ect.

Some embodiments of the invention, include determining presence, absence, and/or an amount (also referred to herein as a level) of HBV polypeptide in one or more biological samples obtained from one or more subjects. The determination can be used to assess efficacy of a treatment method of the invention. For example, methods and compositions of the invention can be used to determine a level of HBV polypeptide and/or HBV viral load in a biological sample obtained from a subject previously treated with administration of an HBV dsRNA agent of the invention. A level of HBV polypeptide and/or HBV viral load determine in the biological sample obtained from the treated subject that is lower by at least 0.5%, 1%, 5%, 10%, 20%, 30%, 40%, 50%, 60%, 70%, 80%, 90%, 95%, 99%, 99.9%, 99.99% or more compared to a pretreatment level of HBV polypeptide or HBV viral load, respectively, determined for the subject, or compared to a non-contacted control biological sample level, indicates a level of efficacy of the treatment administered to the subject. In some embodiments of methods of the invention, contact of a cell (also referred to herein as treatment) with an siRNA agent of the invention results in inhibition of HBV gene expression in the cell by at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or about 100%, for example, to below the level of detection of the assay.

In some embodiments of the invention a physiological characteristic of an HBV-associated disease or condition determined for a subject can be a control determination against which a determination of the physiological characteristic in the same subject at a different time is compared. In a non-limiting example, a physiological characteristic such as an ALT level, an AST level, and/or a viral load level is determined in a biological sample obtained from a subject who has not been administered an HBV treatment of the invention. The ALT level, AST level, and/or viral load level (and/or other physiological characteristic of an HBV disease or condition) determined in the sample obtained from the subject can serve as a baseline or control value for the subject. After one or more administrations of an HBV dsRNA agent to the subject in a treatment method of the invention, one or more additional biological samples can be obtained from the subject and the ALT level, an AST level, and/or a viral load level in the subsequent sample or samples are compared to the control/baseline level and/or ratio, respectively, for the subject. Such comparisons can be used to assess onset, progression, or recession of an HBV associated disease or condition in the subject. For example, an ALT level, an AST level, and/or a viral load level in the baseline sample obtained from the subject that is higher than an ALT level, an AST level, and/or a viral load level determined in a sample obtained from the same subject after the subject has been administered an HBV dsRNA agent of the invention indicates regression of the HBV-associated disease or condition and indicates efficacy of the administered HBV dsRNA agent of the invention for treatment of the HBV-associated disease or condition.

In some aspects of the invention, values of one or more of a physiological characteristic of an HBV-associated disease or condition determined for a subject may serve as control values for later comparison of the physiological characteristics in that same subject, thus permitting assessment of changes from a "baseline" physiological characteristic in a subject. Thus, an initial physiological characteristic may be present and/or determined in a subject and methods and compounds of the invention may be used to decrease the level of HBV polypeptide and/or HBV polypeptide activity in the subject, with the initial physiological characteristic determination serving as a control for that subject.

Using methods of the invention, HBV dsRNA agents of the invention may be administered to a subject in an effective amount to treat an HBV disease or condition. Efficacy of the administration and treatment of the invention can be assessed by determining a change in one or more physiological characteristics of the HBV disease or condition. In non-limiting examples, an ALT level, an AST level, HBV covalently closed circular DNA (cccDNA); one or more HBV antigens; one or more of: HBcAg, HBsAg and HBeAg; and a viral load level determined in a biological sample obtained from a subject following treatment with a method of the invention, that is lower by at least 0.5%, 1%, 5%, 10%, 20%, 30%, 40%, 50%, 60%, 70%, 80%, 90%, 95%, 99%, 99.9%, 99.99%, or 100% compared to a pre-administration level of the physiological characteristic in a biological sample obtained from the subject at a pre-treatment time point, or compared to the determined physiological characteristic in a non-contacted control, demonstrates efficacy of the treatment administered to the subject. It will be understood that a determination of an HBV physiological characteristic as described herein, correlates with a level of HBV gene expression. Certain embodiments of methods of the invention comprise administering an HBV dsRNA agent of the invention to a subject in an amount effective to inhibit HBV gene expression and thereby reduce a an ALT level, an AST level, HBV covalently closed circular DNA (cccDNA); one or more HBV antigens; one or more of: HBcAg, HBsAg and HBeAg; and a viral load level, or otherwise positively impact a physiological characteristic of an HBV-associated disease or condition in the subject.

Some embodiments of the invention, include determining presence, absence, and/or a change in a physiological characteristic of an HBV-associated disease or condition using methods such as but not limited to: (1) assessing one or more biological samples obtained from one or more subjects for the physiological characteristic; (2) taking a cell and/or tissue biopsy from a subject (for example but not limited to a liver biopsy); and (3) or physical examination of the subject. The determination can be used to assess efficacy of a treatment method of the invention.

### Kits

Also within the scope of the invention are kits that comprise one or more HBV dsRNA agents of the invention and instructions for its use in methods of the invention. Kits of the invention may include one or more of an HBV dsRNA agent, HBV sense polynucleotide, and HBV antisense polynucleotide agent that may be used to treat an HBV-associated disease or condition. Kits containing one or more HBV dsRNA agents, HBV sense polynucleotides, and HBV antisense polynucleotide agents can be prepared for use in treatment methods of the invention. Components of kits of the invention may be packaged either in aqueous medium or in lyophilized form. A kit of the invention may comprise a carrier being compartmentalized to receive in close confinement therein one or more container means or series of container means such as test tubes, vials, flasks, bottles, syringes, or the like. A first container means or series of container means may contain one or more compounds such as an HBV dsRNA agent and/or one or more HBV sense or antisense polynucleotide molecules. A second container means or series of container means may contain a targeting agent, a labelling agent, a delivery agent, etc. that may be included as a portion of an HBV dsRNA agent to be administered in an embodiment of a treatment method of the invention.

A kit of the invention may also include instructions. Instructions typically will be in written form and will provide guidance for carrying-out a treatment embodied by the kit and for making a determination based upon that treatment.

The following examples are provided to illustrate specific instances of the practice of the present invention and are not intended to limit the scope of the invention. As will be apparent to one of ordinary skill in the art, the present invention will find application in a variety of compositions and methods.

### Examples

### Example 1.

### Synthesis of HBV RNAi Agents.

HBV RNAi agent duplexes shown in Table 2-4, above, were synthesized in accordance with the following general procedures:
Sense and antisense strand sequences of siRNA were synthesized on oligonucleotide synthesizers using a well-established solid phase synthesis method based on phosphoramidite chemistry. Oligonucleotide chain propagation is achieved through 4-step cycles: a deprotection, a coupling, a capping and an oxidation or a sulfurization step for addition of each nucleotide. Syntheses were performed on a solid support made of controlled pore glass (CPG, 500 or 1000 Å). Monomer phosphoramidites were purchased from commercial sources. Phosphoramidites with GalNAc ligand cluster (GLPA1 and GLPA2 as non-limiting examples) were synthesized according to the procedures of Examples 2-3 herein. For siRNAs used for in vitro screening (Table 2.), syntheses were carried out at 20 nmol scale, and for siRNAs used for in vivo testing (Table 3, 4 and 5), syntheses were carried out at scale of 1 µmol or larger. In the case where the GalNAc ligand (GLO-0 as a non-limiting example) is attached at 3'-end of sense strand, GalNAc ligand attached CPG solid support was used. In the case where the GalNAc ligand (GLS-1 or GLS-2 as non-limiting example) is attached at 5'-end of sense strand, a GalNAc phosphoramidite (GLPA1 or GLPA2 as a non-limiting example) was used for the last coupling reaction. Trichloroacetic acid (TCA) 3% in dichloromethane was used for deprotection of 4,4'-dimethoxytrityl protecting group (DMT). 5-Ethylthio-1H-tetrazole was used as an activator. I₂ in THF/Py/H₂O and phenylacetyl disulfide (PADS) in pyridine/MeCN was used for oxidation and sulfurization reactions, respectively. After the final solid phase synthesis step, cyanoethyl protecting group was removed with 20% diethylamine in acetonitrile. Protecting groups for nucleobase were removed and solid support bound oligomer was cleaved by treating with 28% ammonium hydroxide solution at 65 °C for 2 hours. The aqueous solution contains crude single strand product was concentrated and purified by ion pairing reversed phase HPLC (IP-RP-HPLC). Purified single strand oligonucleotide product from IP-RP-HPLC was converted to sodium salt by dissolving in 1.0 M NaOAc and precipitation by addition of ice cold EtOH. Annealing of equimolar complementary sense stand and antisense strand oligonucleotide in water was performed to form the double strand siRNA product, which was lyophilized to afford a fluffy white solid.

### Example 2. Preparation of Intermediate-A and Intermediate-B.

As shown in Scheme 1 below, Intermediate-A was synthesized by treating commercially available galactosamine pentaacetate with trimethylsilyl trifluoromethanesulfonate (TMSOTf) in dichloromethane (DCM). This was followed by glycosylation with Cbz protected 2-(2-aminoethoxy)ethan-1-ol to give Compound II. The Cbz protecting group was removed by hydrogenation to afford Intermediate-A as a trifluoroacetate (TFA) salt. Intermediate B was synthesized based on the same scheme except Cbz protected 2-(2-(2-aminoethoxy)ethoxy)ethan-1-ol was used as the starting material.

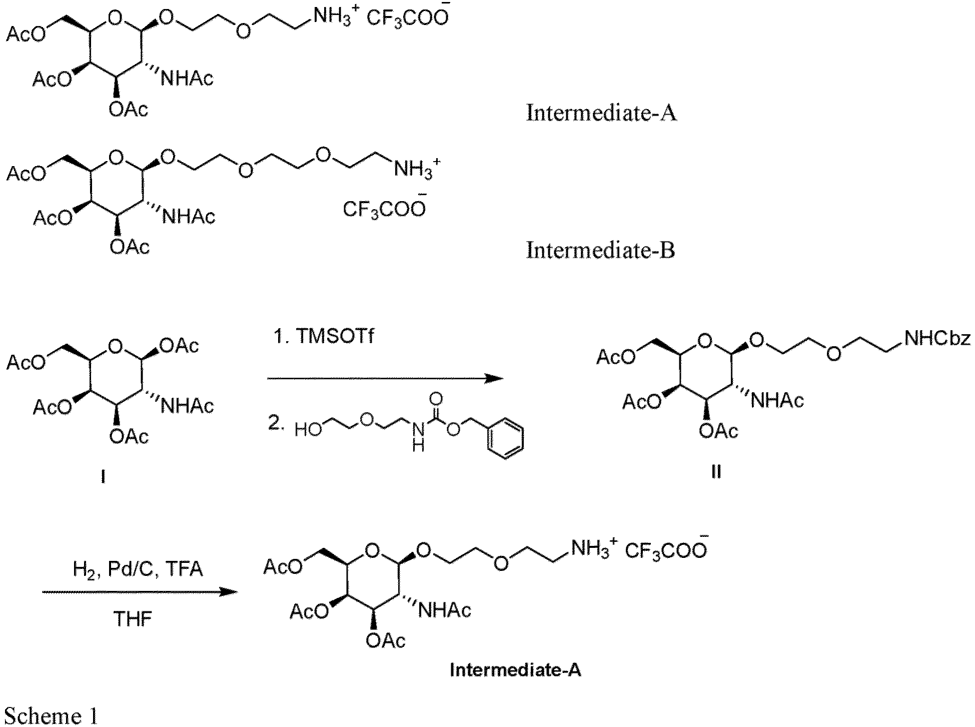

To a solution of Compound I (20.0 g, 51.4 mmol) in 100 mL 1,2-dichloroethane (DCE) was added TMSOTf (17.1 g, 77.2 mmol). The resulting reaction solution was stirred at 60 °C for 2 hrs, and then at 25 °C for 1 hr. Cbz protected 2-(2-aminoethoxy)ethan-1-ol (13.5 g, 56.5 mmol) in DCE (100 mL) dried over 4 Å powder molecular sieves (10 g) was added dropwise to the above mentioned reaction solution at 0 °C under N₂ atmosphere. The resulting reaction mixture was stirred at 25 °C for 16 hrs under N₂ atmosphere. The reaction mixture was filtered and washed with sat. NaHCO₃ (200 mL), water (200 mL) and sat. brine (200 mL). The organic layer was dried over anhydrous Na₂SO₄, filtered and concentrated under reduced pressure to give a crude product, which was triturated with 2-Methyltetrahydrofuran/heptane (5/3, v/v, 1.80 L) for 2 hrs. Resulting mixture was filtered and dried to give Compound II (15.0 g, 50.3% yield) as a white solid.

To a dried and argon purged hydrogenation bottle was carefully added 10% Pd/C (1.50 g), followed by 10 mL tetrahydrofuran (THF) and then a solution of Compound II (15.0 g, 26.4 mmol) in THF (300 mL) and TFA (trifluoroacetic acid, 3.00 g, 26.4 mmol). The resulting mixture was degassed and purged with H₂ three times and stirred at 25 °C for 3 hrs under H₂ (45 psi) atmosphere. Thin-layer chromatography (TLC, solvent: DCM:MeOH = 10:1) indicated Compound II was consumed completely. The reaction mixture was filtered and concentrated under reduced pressure. Residue was dissolved in anhydrous DCM (500 mL) and concentrated. This process was repeated 3 times to give Intermediate-A (14.0 g, 96.5% yield) as a foamy white solid. ¹H NMR (400 MHz DMSO-*d₆*): δ ppm 7.90 (d, *J* = 9.29 Hz, 1 H), 7.78 (br s, 3 H), 5.23 (d, *J* = 3.26 Hz, 1 H), 4.98 (dd, *J* = 11.29, 3.26 Hz, 1 H), 4.56 (d, *J* = 8.53 Hz, 1 H), 3.98 - 4.07 (m, 3 H), 3.79 - 3.93 (m, 2 H), 3.55 - 3.66 (m, 5 H), 2.98 (br d, *J* = 4.77 Hz, 2 H), 2.11 (s, 3 H), 2.00 (s, 3 H), 1.90 (s, 3 H), 1.76 (s, 3 H).
Intermediate-B was synthesized using similar procedures for synthesis of Intermediate-A. ¹H NMR (400 MHz DMSO-*d₆*): δ ppm 7.90 (br d, *J* = 9.03 Hz, 4 H), 5.21 (d, *J* = 3.51 Hz, 1 H), 4.97 (dd, *J* = 11.1 Hz, 1 H), 4.54 (d, *J* = 8.53 Hz, 1 H), 3.98 - 4.06 (m, 3 H), 3.88 (dt, *J* = 10.9 Hz, 1 H), 3.76 - 3.83 (m, 1 H), 3.49 - 3.61 (m, 9 H), 2.97 (br s, 2 H), 2.10 (s, 3 H), 1.99 (s, 3 H), 1.88 (s, 3 H), 1.78 (s, 3 H). Mass calc. for C₂₀H₃₄N₂O₁₁: 478.22; found: 479.3 (M+H⁺).

### Example 3. Synthesis of GalNAc ligand cluster phosphoramidite GLPA1, GLPA2 and GLPA15.

Scheme 2 below was followed to prepare GLPA1 and GLPA2. Starting from benzyl protected propane-1,3-diamine, it was alkylated with *tert*-butyl 2-bromoacetate to afford triester Compound I. The benzyl protecting group was removed by hydrogenation to afford secondary amine Compound II. Amide coupling with 6-hydroxyhexanoic acid afforded Compound III. *tert*-Butyl protecting groups were then removed upon treatment of HCl in dioxane to generate triacid Compound IV. Amide coupling between triacid compound IV and Intermediate-A or Intermediate-B was performed to afford Compound Va or Vb. Phosphoramidite GLPA1 or GLPA2 was synthesized by phosphitylation of Compound Va or Vb with 2-Cyanoethyl N,N-diisopropylchlorophosphoramidite and a catalytic amount of 1H-tetrazole.

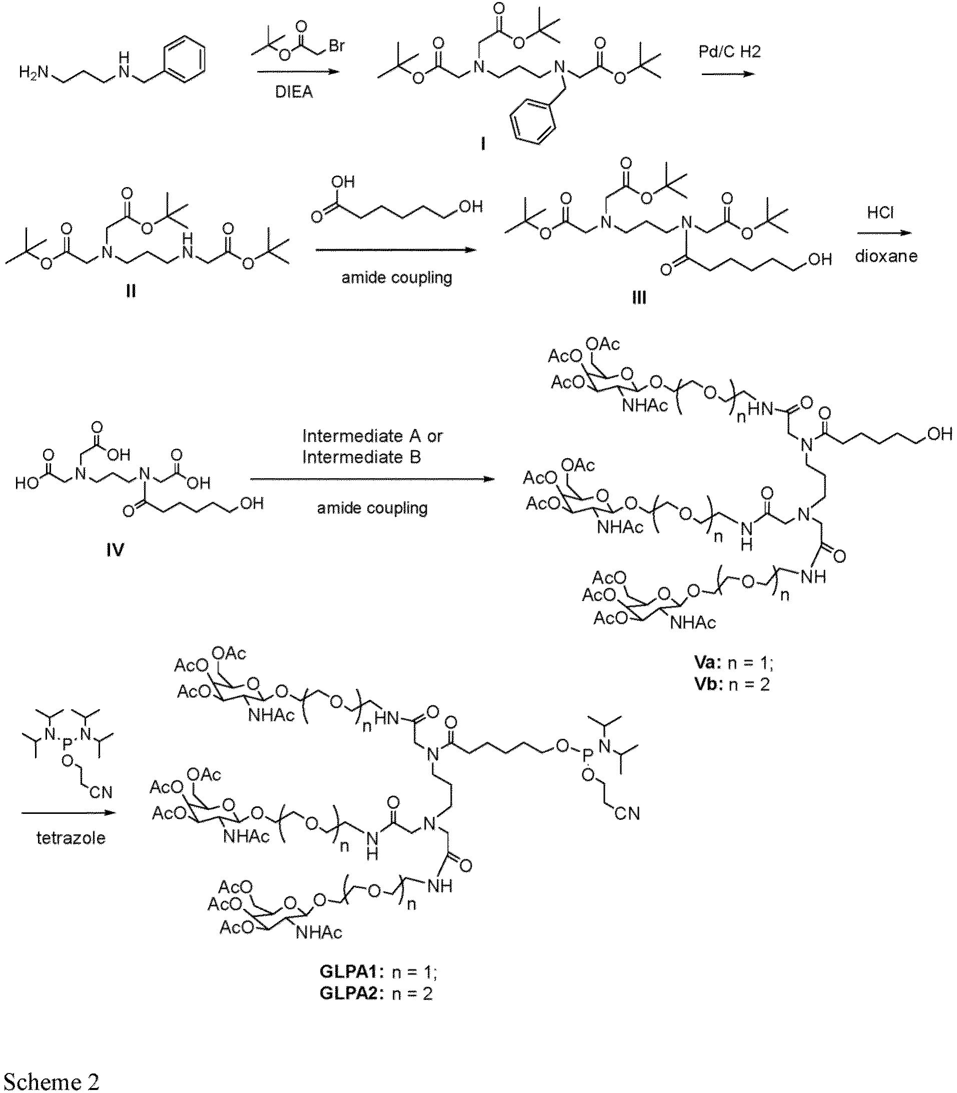

To a solution of N-Benzyl-1,3-propanediamine (5.00 g, 30.4 mmol) in dimethylformamide (DMF, 100 mL) was added *tert*-butyl 2-bromoacetate (23.7 g, 121 mmol), followed by addition of diisopropylethylamine (DIEA, 23.61 g, 182 mmol) dropwise. The resulting reaction mixture was stirred at 25-30 °C for 16 hrs. LCMS showed N-Benzyl-1,3-propanediamine was consumed completely. Reaction mixture was diluted with H₂O (500 mL) and extracted with EtOAc (500 mL × 2). The combined organics were washed with sat. brine (1 L), dried over anhydrous Na₂SO₄, filtered, and concentrated under reduced pressure to give crude product, which was purified by silica gel column chromatography (gradient: petroleum ether:ethyl acetate from 20:1 to 5:1). Compound I (12.1 g, 78.4% yield) was obtained as a colorless oil. ¹H NMR (400 MHz, CDCl₃): δ ppm 7.26 - 7.40 (m, 5 H), 3.79 (s, 2 H), 3.43 (s, 4 H), 3.21 (s, 2 H), 2.72 (dt, *J* = 16.9, 7.34 Hz, 4 H), 1.70 (quin, *J* = 7.2 Hz, 2 H), 1.44 - 1.50 (m, 27 H).

A dried hydrogenation bottle was purged with Argon three times. Pd/C (200 mg, 10%) was added, followed by MeOH (5 mL) and then a solution of Compound I (1.00 g, 1.97 mmol) in MeOH (5 mL). The reaction mixture was degassed under vacuum and refilled with H₂. This process was repeated three times. The mixture was stirred at 25°C for 12 hrs under H₂ (15 psi) atmosphere. LCMS showed Compound I was consumed completely. The reaction mixture was filtered under reduced pressure under N₂ atmosphere. Filtrate was concentrated under reduced pressure to give Compound II (655 mg, 79.7% yield) as yellow oil, which was used for the next step without further purification. ¹H NMR (400 MHz, CDCl₃): δ ppm 3.44 (s, 4 H), 3.31 (s, 2 H), 2.78 (t, *J* = 7.1 Hz, 2 H), 2.68 (t, *J* = 6.9 Hz, 2 H), 1.88 (br s, 1 H), 1.69 (quin, *J* = 7.03 Hz, 2 H), 1.44 - 1.50 (s, 27 H).

A mixture of Compound II (655 mg, 1.57 mmol) , 6-hydroxyhexanoic acid (249 mg, 1.89 mmol), DIEA (1.02 g, 7.86 mmol) , 1-ethyl-3-(3-dimethylaminopropyl)carbodiimide hydrochloride (EDCI, 904 mg, 4.72 mmol), and 1-Hydroxybenzotriazole (HOBt, 637 mg, 4.72 mmol) in DMF (6 mL) was degassed and purged with N₂ three times, and then was stirred at 25°C for 3 hrs under N₂ atmosphere. LCMS indicated desired product. The reaction mixture was diluted with H₂O (10 mL) and extracted with EtOAc 20 mL (10 mL × 2). Organics were combined and washed with sat. brine (20 mL), dried over anhydrous Na₂SO₄, filtered, and concentrated to give crude product, which was purified by silica gel column chromatography (gradient: petroleum ether:ethyl acetate from 5:1 to 1:1) to afford Compound III (650 mg, 77.8% yield) as a yellow oil. ¹H NMR (400 MHz, CDCl₃): δ ppm 3.90 - 3.95 (s, 2 H), 3.63 (t, *J* = 6.40 Hz, 2 H), 3.38 - 3.45 (m, 6 H), 2.72 (t, *J* = 6.65 Hz, 2 H), 2.40 (t, *J* = 7.28 Hz, 2 H), 1.55 - 1.75 (m, 8 H), 1.44 (s, 27 H). Mass calc. for C₂₇H₅₀N₂O₈: 530.36; found: 531.3 (M+H⁺).

A mixture of Compound III (5.5 g, 10.3 mmol) in HCl/dioxane (2M, 55 mL) was stirred at 25 °C for 3 hrs. LCMS showed complete consumption of Compound III. Reaction mixture was filtered, washed with EtOAc (50 mL), and dried under reduced pressure to give crude product. It was dissolved in CH₃CN (50 mL), volatiles were removed under vacuum. This process was repeated three times to give Compound IV (2.05 g, 54.5% yield) as a white solid. ¹H NMR (400 MHz, D₂O): δ ppm 4.21 (s, 1 H), 4.07 (d, *J* = 4.5 Hz, 4 H), 3.99 (s, 1 H), 3.45 - 3.52 (m, 3 H), 3.42 (t, *J* = 6.5 Hz, 1 H), 3.32 - 3.38 (m, 1 H), 3.24 - 3.31 (m, 1 H), 2.37 (t, *J* = 7.4 Hz, 1 H), 2.24 (t, *J* = 7.4 Hz, 1 H), 1.99 (dt, *J* = 15.5, 7.53 Hz, 1 H), 1.85 - 1.94 (m, 1 H), 1.85 - 1.94 (m, 1 H), 1.39 - 1.56 (m, 4 H), 1.19 - 1.31 (m, 2 H).

A mixture of Compound IV (500 mg, 1.05 mmol), Intermediate-A (2.02 g, 3.67 mmol), DIEA (813 mg, 6.30 mmol), EDCI (704 mg, 3.67 mmol) and HOBt (496 mg, 3.67 mmol) in DMF (10 mL) was degassed and purged with N₂ for 3 times, and then the mixture was stirred at 25 °C for 3 hrs under N2 atmosphere. LCMS indicated desired product. The reaction mixture was quenched by addition of H₂O (10 mL), extracted with DCM (10 mL × 2). The combined organics were extracted with 10% citric acid (20 mL). The aqueous phase was neutralized with saturated NaHCO₃ solution and re-extracted with DCM (10 mL × 2). Organics were dried over sodium sulfate, filtered and concentrated under reduced pressure to give Compound Va (570 mg, 0.281 mmol, 26.8% yield) as a white solid. ¹H NMR: (400 MHz, CDCl₃) ppm δ 7.84 - 8.12 (m, 3 H), 6.85 - 7.15 (m, 2 H), 6.66 - 6.81 (m, 1 H), 5.36 (br d, *J* = 2.7 Hz, 3 H), 5.11 - 5.27 (m, 3 H), 4.63 - 4.85 (m, 3 H), 3.90 - 4.25 (m, 18 H), 3.37 - 3.75 (m, 28 H), 3.15 - 3.28 (m, 4 H), 2.64 (br d, *J* = 6.53 Hz, 2 H), 2.30 - 2.46 (m, 2 H), 2.13 - 2.18 (m, 9 H), 2.05 (s, 9 H), 1.94 - 2.03 (m, 18 H), 1.68 (br s, 2 H), 1.45 (br s, 2 H), 1.12 (br t, *J* = 7.0 Hz, 2 H).

To a solution of Compound Va (260 mg, 0.161 mmol) in anhydrous DCM (5 mL) was added diisopropylammonium tetrazolide (30.3 mg, 0.177 mmol), followed by dropwise addition of 3-bis(diisopropylamino)phosphanyloxypropanenitrile (194 mg, 0.645 mmol) at ambient temperature under N₂. The reaction mixture was stirred at 20 ~ 25 °C for 2 hrs. LCMS indicated Compound Va was consumed completely. After cooling to -20 °C, the reaction mixture was added to a stirred solution of brine/saturated aq. NaHCO₃ (1:1, 5 mL) at 0 °C. After stirring for 1 min, DCM (5 mL) was added. Layers were separated. Organics were washed with brine/saturated aq. NaHCO₃ solution (1:1, 5 mL), dried over Na₂SO₄, filtered, and concentrated to ~ 1 mL of volume. The residue solution was added dropwise to 20 mL methyl tert-butyl ether (MTBE) with stirring. This resulted in precipitation of white solid. The mixture was centrifuged, and solid was collected. The solid was redissolved in 1 mL of DCM and precipitated by addition of MTBE (20 mL). Solid was again isolated by centrifuge. The solid collected was dissolved in anhydrous CH₃CN. Volatiles were removed. This process was repeated two more times to afford GalNAc ligand phosphoramidite compound GLPA1 (153 mg, 84.4 µmol) as a white solid. ¹H NMR (400 MHz, CDCl₃): ppm δ 7.71 - 8.06 (m, 2 H), 6.60 - 7.06 (m, 3 H), 5.37 (br d, J = 3.0 Hz, 3 H), 5.18 - 5.32 (m, 3 H), 4.70 - 4.86 (m, 3 H), 3.92 - 4.25 (m, 18 H), 3.42 - 3.85 (m, 30 H), 3.25 (m, 4 H), 2.59 - 2.75 (m, 4 H), 2.27 - 2.44 (m, 2 H), 2.15 - 2.20 (s, 9 H) 2.07 (s, 9 H), 1.96 - 2.03 (m, 18 H), 1.65 (br s, 4 H), 1.44 (br d, J = 7.28 Hz, 2 H), 1.14 - 1.24 (m, 12 H). ³¹P NMR (CDCl₃): ppm δ 147.15.

GalNAc ligand phosphoramidite compound GLPA2 was synthesized using the same procedure except Intermediate-B was used. ¹H NMR (400 MHz, CDCl₃): ppm δ 7.94 - 8.18 (m, 1 H), 7.69 (br s, 1 H), 6.66 - 7.10 (m, 3 H), 5.35 (d, *J* = 3.5 Hz, 3 H), 5.07 - 5.25 (m, 3 H), 4.76 - 4.86 (m, 3 H), 4.01 - 4.31 (m, 10 H), 3.91 - 4.01 (m, 8 H), 3.74 - 3.86 (m, 4 H), 3.52 - 3.71 (m, 30 H), 3.42 - 3.50 (m, 6 H), 3.15 - 3.25 (m, 4 H), 2.52 - 2.70 (m, 4 H), 2.22 - 2.45 (m, 2 H), 2.15 - 2.22 (s, 9 H), 2.06 (s, 9 H), 1.95 - 2.03 (m, 18 H), 1.77 (br s, 2 H), 1.58 - 1.66 (m, 4 H), 1.40 (m, 2 H), 1.08 - 1.24 (m, 12 H). ³¹P NMR (CDCl₃): ppm δ 147.12.

Scheme 3 below was followed to prepare GLPA15.

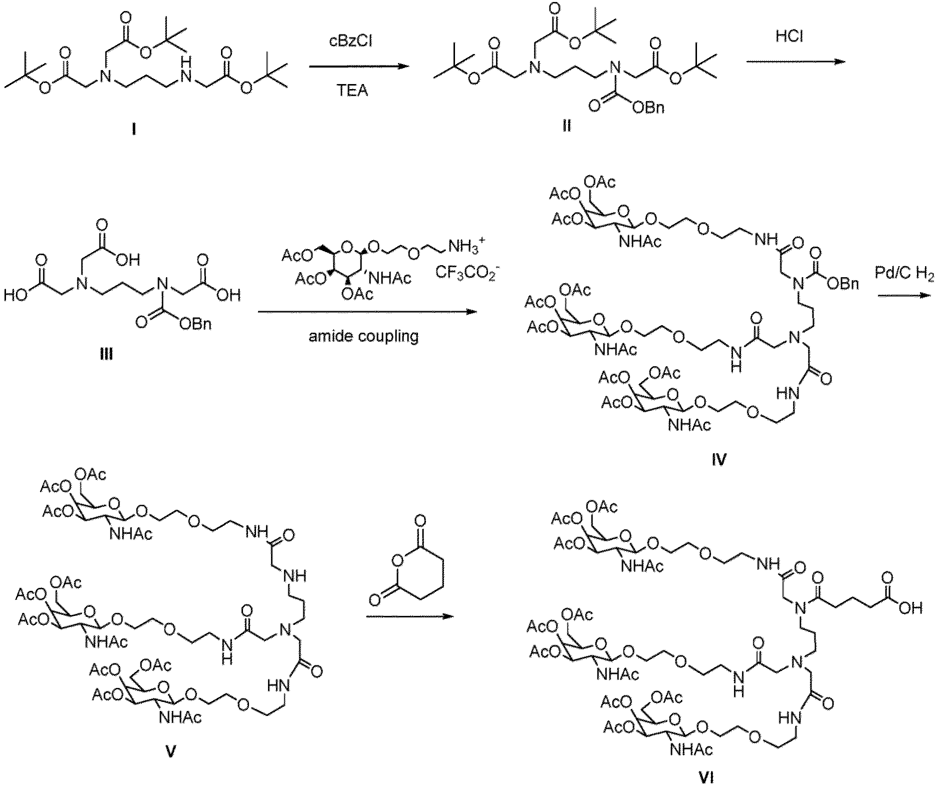

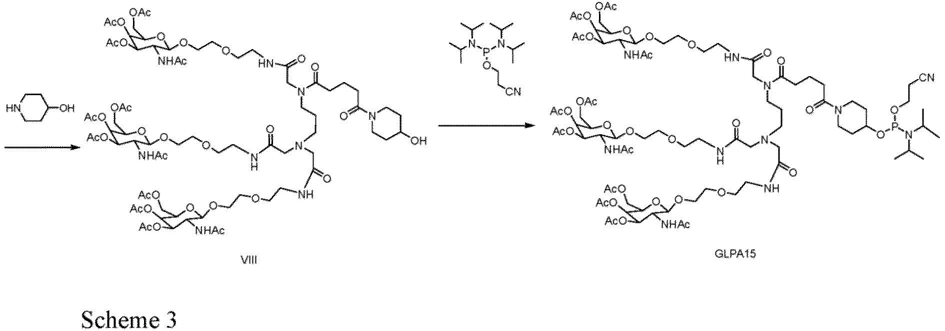

Starting from secondary amine Compound I (Compound II in Scheme 2), Cbz protection was introduced to afford Compound II. The tert-Butyl groups of Compound II were removed by treatment with acid to give triacid Compound III. Amide coupling of Compound III with Intermediate-A afforded Compound IV. The Cbz protecting group of Compound IV was removed by hydrogenation to afford secondary amine Compound V, which was reacted with glutaric anhydride to afford carboxylic Compound VI. Compound VI reacted with piperidin-4-ol under amide coupling reaction condition to affords Compound VII. Phosphoramidite Compound GLPA15 was synthesized by treating Compound VII with 2-Cyanoethyl N,N diisopropylchlorophosphoramidite and a catalytic amount of 1H-tetrazole.

¹H NMR (400 MHz in DMSO-d6): δ ppm 8.05 (br d, J = 6.50 Hz, 2 H), 7.81 (br d, J=9.01 Hz, 3 H), 5.22 (d, J=3.25 Hz, 3 H), 4.98 (dd, J=11.26, 3.25 Hz, 3 H), 4.55 (br d, J=8.50 Hz, 3 H), 4.03 (s, 9 H), 3.64 - 3.97 (m, 12 H), 3.55 - 3.63 (m, 6 H), 3.50 (br s, 5 H), 3.40 (br d, J=6.13 Hz, 6 H), 3.17 - 3.30 (m, 9 H), 3.07 (br d, J=14.26 Hz, 4 H), 2.76 (t, J=5.82 Hz, 2 H), 2.18 - 2.47 (m, 6 H), 2.10 (s, 9 H), 1.99 (s, 9 H), 1.89 (s, 9 H), 1.78 (s, 9 H), 1.52 - 1.74 (m, 6 H), 1.12 - 1.19 (m, 12 H). 31P NMR (DMSO-d6): ppm δ 145.25.

In certain studies, a method used to attach a targeting group comprising GalNAc (also referred to herein as a GalNAc delivery compound) to the 5'-end of a sense strand included use of a GalNAc phosphoramidite (GLPA1) in the last coupling step in the solid phase synthesis, using a synthetic process such as the process used if oligonucleotide chain propagation of adding a nucleotide to the 5'-end of the sense strand is performed.

In some studies a method of attaching a targeting group comprising GalNAc to the 3'-end of a sense strand comprised use of a solid support (CPG) that included a GLO-n. In some studies, a method of attaching a targeting group comprising GalNAc to the 3'-end of a sense strand comprises attaching a GalNAc targeting group to CPG solid support through an ester bond and using the resulting CPG with the attached GalNAc targeting group when synthesizing the sense strand, which results in the GalNAc targeting group attached at the 3'-end of the sense strand.

### Example 4. In Vitro Screening of HBV siRNA Duplexes

HBV transfected HepG2.2.15 cell line was used for in vitro evaluation of HBV siRNA agents. HepG2.2.15 cells were seeded into 96 well plates and were transfected with HBV siRNA agents at final concentration of 0.04 nM, 0.2 nM and 1 nM using Lipofectamine^{®} RNAiMax as the transfecting agent. Supernatant was harvested at days 72 hours after seeding. HBsAg levels were determined by ELISA assay. Knockdown activity was calculated by comparing HBsAg level from samples transfected with HBV siRNA agents to the non-transfected control sample (Lipofectamine^{®} RNAiMax only). Experiment was performed in triplicates. Result is summarized in Table 5 and 7. Even at 0.04 nM, multiple siRNAs demonstrated good activity suppressing HBsAg expression. A subset of siRNAs demonstrated good to excellent activity were selected for in vivo testing.

**Table 5 provides experimental results of in vitro studies using various HBV RNAi agents to inhibit HBsAg expression. Studies are described elsewhere herein.**

| Duplex ID | Average Inhibition % | | | | | |
|---|---|---|---|---|---|---|
| | 1 nM | | 0.2 nM | | 0.04 nM | |
| | Average Inh. % | STD | Average Inh. % | STD | Average Inh. % | STD |
| AV00053 | 90.63 | 1.42 | 75.93 | 3.06 | 12.27 | 14.97 |
| AV00054 | 94.67 | 2.68 | 89.97 | 0.76 | 33.87 | 10.02 |
| AV00055 | 95.73 | 0.45 | 93.83 | 1.59 | 73.23 | 8.74 |
| AV00056 | 96.67 | 0.50 | 85.30 | 4.16 | 31.87 | 14.20 |
| AV00057 | 96.43 | 1.26 | 97.07 | 1.18 | 82.40 | 5.53 |
| AV00058 | 95.00 | 1.31 | 90.57 | 2.92 | 36.03 | 13.59 |
| AV00059 | 91.20 | 2.00 | 76.17 | 4.09 | 10.47 | 18.77 |
| AV00060 | 97.80 | 1.51 | 87.50 | 3.80 | 53.10 | 12.17 |
| AV00061 | 84.50 | 2.36 | 60.90 | 2.21 | 6.27 | 16.95 |
| AV00062 | 98.07 | 0.21 | 95.33 | 1.00 | 46.67 | 15.18 |
| AV00063 | 95.93 | 2.06 | 83.13 | 4.39 | 13.00 | 9.62 |
| AV00064 | 95.77 | 0.72 | 83.53 | 4.57 | 30.53 | 8.46 |
| AV00065 | 96.27 | 2.23 | 77.33 | 7.61 | 33.60 | 2.25 |
| AV00066 | 92.13 | 4.90 | 92.70 | 2.27 | 84.50 | 0.10 |
| AV00067 | 100.00 | 0.00 | 88.33 | 5.35 | 36.33 | 1.50 |
| AV00068 | 100.00 | 0.00 | 100.00 | 0.00 | 65.80 | 0.56 |
| AV00069 | 98.80 | 0.85 | 99.43 | 0.42 | 54.20 | 2.97 |
| AV00070 | 98.17 | 1.93 | 46.30 | 24.08 | 23.23 | 4.67 |
| AV00071 | 98.67 | 2.31 | 100.00 | 0.00 | 73.80 | 3.10 |
| AV00072 | 100.00 | 0.00 | 90.63 | 14.86 | 59.87 | 4.67 |
| AV00073 | 69.40 | 20.35 | 9.10 | 47.84 | 15.57 | 5.60 |
| AV00074 | 96.33 | 5.03 | 87.77 | 21.19 | 47.83 | 2.30 |
| AV00075 | 98.70 | 1.30 | 93.40 | 5.67 | 23.60 | 7.57 |
| AV00076 | 99.87 | 0.23 | 99.23 | 1.33 | 67.47 | 3.49 |
| AV00077 | 85.17 | 1.46 | 70.33 | 4.53 | 16.77 | 11.78 |
| AV00078 | 99.10 | 0.56 | 97.57 | 0.35 | 45.03 | 6.45 |
| AV00079 | 95.10 | 4.20 | 86.73 | 4.76 | 31.93 | 8.95 |
| AV00080 | 97.47 | 1.82 | 86.80 | 9.95 | 66.50 | 5.65 |
| AV00081 | 93.23 | 1.10 | 86.40 | 2.65 | 27.10 | 9.13 |
| AV00082 | 99.50 | 0.30 | 94.13 | 6.13 | 66.97 | 4.65 |
| AV00083 | 97.47 | 1.86 | 97.93 | 0.50 | 68.70 | 3.44 |
| AV00084 | 89.00 | 0.98 | 43.77 | 15.95 | -0.50 | 6.14 |
| AV00085 | 95.97 | 3.27 | 91.37 | 2.48 | 29.77 | 3.66 |
| AV00086 | 86.07 | 5.26 | 59.50 | 6.22 | 8.57 | 14.39 |
| AV00087 | 89.30 | 6.01 | 72.20 | 12.25 | 8.97 | 15.45 |
| AV00088 | 94.27 | 1.76 | 73.57 | 13.73 | 17.63 | 6.99 |
| AV00089 | 90.40 | 2.42 | 63.83 | 1.03 | 13.13 | 6.35 |
| AV00090 | 96.60 | 1.61 | 89.40 | 3.03 | 53.97 | 2.35 |
| AV00091 | 98.57 | 1.10 | 91.00 | 2.76 | 27.00 | 6.85 |
| AV00092 | 97.50 | 1.04 | 76.47 | 6.39 | 26.00 | 4.83 |
| AV00093 | 90.03 | 2.39 | 69.10 | 12.06 | 17.60 | 2.89 |
| AV00094 | 99.27 | 0.67 | 93.07 | 4.28 | 50.97 | 2.95 |
| AV00095 | 74.97 | 11.41 | 45.83 | 4.92 | 12.93 | 6.32 |
| AV00096 | 91.47 | 5.18 | 57.00 | 8.34 | 16.63 | 5.01 |
| AV00097 | 95.07 | 4.41 | 88.97 | 4.10 | 37.70 | 5.44 |
| AV00098 | 94.43 | 4.24 | 90.77 | 5.54 | 47.20 | 3.93 |
| AV00099 | 71.93 | 7.96 | 50.13 | 3.15 | 2.30 | 6.85 |
| AV00100 | 87.47 | 7.27 | 63.53 | 9.72 | -6.77 | 8.80 |
| AV00101 | 85.37 | 4.18 | 42.27 | 3.23 | -26.43 | 7.90 |
| AV00102 | 94.37 | 5.36 | 67.73 | 3.02 | -8.20 | 11.91 |
| AV00103 | 94.00 | 4.22 | 89.53 | 3.81 | -3.00 | 28.80 |
| AV00104 | 87.47 | 6.82 | 49.17 | 8.36 | -12.00 | 22.88 |
| AV00105 | 99.90 | 0.10 | 96.67 | 3.31 | 14.30 | 11.61 |
| AV00106 | 99.87 | 0.23 | 97.80 | 0.17 | 29.37 | 6.09 |
| AV00107 | 99.47 | 0.35 | 90.70 | 3.33 | 19.50 | 17.00 |
| AV00108 | 35.03 | 21.35 | 12.67 | 11.81 | -19.73 | 14.65 |
| AV00109 | 96.70 | 3.29 | 83.33 | 8.02 | 7.23 | 25.36 |
| AV00110 | 100.00 | 0.00 | 98.57 | 1.69 | 42.07 | 13.88 |
| AV00111 | 75.00 | 4.19 | 27.70 | 12.06 | -13.33 | 15.88 |
| AV00112 | 87.77 | 7.50 | 45.77 | 15.87 | -4.43 | 14.34 |
| AV00113 | 27.57 | 11.66 | -24.07 | 8.65 | -29.53 | 15.30 |
| AV00114 | 93.57 | 3.10 | 78.80 | 2.17 | 13.97 | 14.85 |
| AV00115 | 97.80 | 1.08 | 87.17 | 4.20 | 41.20 | 11.26 |
| AV00116 | 83.93 | 4.89 | 67.27 | 6.91 | 9.20 | 10.00 |
| AV00117 | 77.70 | 7.43 | 38.80 | 7.43 | 3.97 | 13.49 |
| AV00118 | 94.00 | 1.56 | 73.37 | 1.80 | 11.07 | 6.13 |
| AV00119 | 89.57 | 0.80 | 58.37 | 3.71 | 19.47 | 7.41 |
| AV00120 | 99.37 | 0.31 | 93.83 | 3.79 | 69.20 | 5.57 |
| AV00121 | 74.83 | 5.31 | 30.57 | 4.15 | 7.47 | 8.98 |

**Table 7 provides experimental results of in vitro studies using various HBV RNAi agents to inhibit HBsAg expression. Studies are described elsewhere herein.**

| Duplex ID | Average Inhibition % | | | |
|---|---|---|---|---|
| | 1 nM | | 0.04 nM | |
| | Average Inh. % | STD | Average Inh. % | STD |
| AV01258 | 66.03 | 1.07 | 38.84 | 2.63 |
| AV01259 | 72.73 | 4.06 | 65.13 | 9.34 |
| AV01260 | 58.50 | 3.97 | 34.51 | 4.09 |
| AV01261 | 59.10 | 1.43 | 21.00 | 1.75 |
| AV01262 | -15.58 | 20.65 | -17.77 | 8.32 |
| AV01263 | 15.93 | 4.77 | -17.45 | 1.53 |
| AV01264 | 59.66 | 2.33 | 13.49 | 6.44 |
| AV01265 | 41.12 | 1.62 | -0.45 | 7.85 |
| AV01266 | 62.29 | 1.64 | 17.87 | 0.70 |
| AV01267 | 71.24 | 0.85 | 39.46 | 1.50 |
| AV01268 | 60.34 | 0.74 | 55.60 | 0.92 |
| AV01269 | 65.40 | 4.10 | 39.61 | 4.23 |
| AV01270 | 64.13 | 0.55 | 38.87 | 4.02 |
| AV01271 | 63.81 | 1.41 | 43.45 | 0.03 |
| AV00057 | 67.84 | 1.86 | 56.32 | 0.52 |
| AV01273 | 63.25 | 2.93 | 57.11 | 2.13 |
| AV01274 | 79.59 | 3.34 | 64.74 | 0.86 |
| AV01275 | 74.55 | 0.72 | 61.25 | 3.99 |
| AV01276 | 66.64 | 4.38 | 48.82 | 4.31 |
| AV01277 | 74.44 | 6.63 | 58.12 | 4.08 |
| AV01278 | 68.11 | 6.86 | 47.17 | 2.04 |
| AV01279 | 60.16 | 7.07 | 37.95 | 5.21 |
| AV01280 | 67.92 | 1.28 | 14.83 | 1.57 |
| AV01281 | 79.62 | 2.15 | 65.34 | 0.75 |
| AV01282 | 80.38 | 0.46 | 61.42 | 3.25 |
| AV01283 | 72.42 | 1.26 | 42.27 | 0.13 |
| AV01284 | 74.42 | 5.62 | 48.43 | 6.25 |
| AV01285 | 76.18 | 1.72 | 47.59 | 8.98 |
| AV01286 | 85.49 | 0.35 | 66.22 | 0.19 |
| AV01287 | 64.66 | 8.63 | 27.79 | 1.00 |
| AV01288 | 73.01 | 3.07 | 67.60 | 2.03 |
| AV01289 | 83.68 | 1.19 | 73.41 | 2.26 |
| AV01290 | 70.77 | 2.12 | 48.65 | 1.49 |
| AV01291 | 70.65 | 1.84 | 45.80 | 4.70 |
| AV01293 | 67.51 | 7.01 | 32.56 | 0.71 |
| AV01294 | 25.93 | 2.24 | 9.62 | 3.68 |
| AV01295 | 61.92 | 3.37 | 22.19 | 9.25 |
| AV01296 | 78.13 | 3.35 | 59.18 | 3.85 |
| AV01297 | 87.50 | 0.34 | 63.20 | 1.81 |
| AV01298 | 79.02 | 1.49 | 61.64 | 1.84 |
| AV01299 | 81.97 | 2.66 | 70.71 | 1.67 |
| AV01300 | 82.36 | 1.15 | 70.33 | 3.88 |
| AV01301 | 79.96 | 4.2 | 62.60 | 1.72 |
| AV01302 | 90.58 | 2.54 | 60.88 | 1.49 |
| AV01303 | 92.07 | 2.04 | 65.97 | 1.79 |
| AV01304 | 92.79 | 6.13 | 71.91 | 1.29 |
| AV01305 | 88.40 | 4.12 | 55.36 | 4.69 |
| AV01306 | 78.13 | 1.3 | 14.30 | 6.85 |
| AV01307 | 88.38 | 1.46 | 57.92 | 8.49 |
| AV01308 | 85.08 | 0.4 | 58.98 | 0.73 |
| AV01309 | 86.50 | 2.65 | 51.86 | 0.57 |
| AV01310 | 72.74 | 12.22 | 41.82 | 10.07 |
| AV01311 | 57.74 | 1.11 | 21.57 | 13.04 |
| AV01312 | 82.52 | 2.71 | 69.91 | 12.08 |
| AV01313 | 89.11 | 2.47 | 75.06 | 2.05 |
| AV01314 | 95.40 | 2.23 | 88.25 | 3.28 |
| AV01315 | 94.62 | 0.72 | 62.81 | 9.21 |
| AV01316 | 93.43 | 0.52 | 69.42 | 5.12 |
| AV01317 | 92.85 | 2.39 | 78.99 | 1.25 |
| AV01318 | 88.86 | 1.02 | 60.18 | 13.13 |
| AV01319 | 90.65 | 0.40 | 77.74 | 4.85 |

### Example 5. In vivo Screening of siRNA agents

In vivo efficacy of HBV siRNA agents was evaluated using the AAV-HBV mouse model. Male C57BL/6 mice, 5 weeks old, were infected with 1×10^11 rAAV8-1.3 HBV viral genomes via tail vein injection. 14 days after infection, blood samples were taken and HBsAg in mouse serum was measured by ELISA assay. Mice with high expression level of HBsAg (log HBsAg IU/ml > 4.9) were selected for the study. At day 18 post infection, each mouse was administrated subcutaneously with a single 3 mg/kg dose of HBV siRNA or PBS control. Blood samples were drawn at day 7 and day 14 post dosing. Serum HBsAg was measured by ELISA assay. The HBsAg level for each animal at a time point was divided by the pretreatment level of expression (day 14 post infection) in that animal in order to determine the ratio of expression "normalized to pre-treatment". The knockdown activity was calculated by dividing the "normalized to pre-treatment" ratio for an individual animal by the average "normalized to pre-treatment" ratio of all mice in the PBS control group. Result is summarized in Table 6. Highly efficacious HBV siRNAs were identified. In particular, mice treated with siRNA agent AD00170, AD00263, AD00265, AD00267, AD00268, AD00269, AD00173 and AD00176, showed more than 96% reduction of HBsAg (normalized to pre-treatment). In this example, the GLO-0 in said compounds in Table 3 refers to the compound GalNAc3 in Jayaprakash, et al., (2014) J. Am. Chem. Soc., 136, 16958-16961.

**Table 6. HBV siRNA single 3 mpk subcutaneous dose screening in AAV-HBV transduced mouse. Percent of reduction of HBsAg in mouse serum was normalized to HBsAg level pre-dosing of siRNA and to the PBS control group.**

| Duplex ID | Percent reduction of HBsAg in mouse serum 7 and 14 days after treatment of siRNA | |
|---|---|---|
| | Day 7 | Day 14 |
| AD00166 | 75.98% | 39.95% |
| AD00167 | 70.82% | 81.65% |
| AD00168 | 95.79% | 89.35% |
| AD00169 | 94.07% | 87.96% |
| AD00170 | 99.68% | 99.34% |
| AD00171 | 48.41% | 35.98% |
| AD00172 | 83.22% | 87.40% |
| AD00173 | 96.00% | 95.92% |
| AD00174 | 44.78% | 3.08% |
| AD00175 | 57.40% | 48.30% |
| AD00176 | 91.10% | 96.39% |
| AD00177 | 64.43% | 72.23% |
| AD00261 | 68.52% | 88.31% |
| AD00262 | 87.71% | 93.28% |
| AD00263 | 98.90% | 98.97% |
| AD00264 | 55.87% | 69.62% |
| AD00265 | 86.59% | 96.14% |
| AD00266 | 90.70% | 95.80% |
| AD00267 | 99.93% | 99.91% |
| AD00268 | 98.95% | 98.55% |
| AD00269 | 96.95% | 98.01% |
| AD00270 | 85.03% | 77.79% |
| AD00271 | 79.51% | 90.03% |
| AD00272 | 76.43% | 79.85% |
| AD00273 | 55.91% | 56.05% |

### Example 6. In vivo testing of siRNA agents in AAV-HBV mouse model

In vivo efficacy of HBV siRNA agents was evaluated using the AAV-HBV mouse model. Male C57BL/6 mice, 5 weeks old, were infected with 1×10^11 rAAV8-1.3 HBV viral genomes via tail vein injection. 14 days after infection, blood samples were taken and HBsAg in mouse serum was measured by ELISA assay. Mice with high expression level of HBsAg (log HBsAg IU/ml > 4.9) were selected for the study. At day 18 post infection, each mouse was administrated subcutaneously with a single 3 mg/kg dose of HBV siRNA or PBS control. Blood samples were drawn at day 7, day 14 and day 21 post dosing. Serum HBsAg and HBeAg was measured by ELISA assay. The HBsAg and HBeAg levels for each animal at a time point was divided by the pretreatment level of expression (day 14 post infection) in that animal in order to determine the ratio of expression "normalized to pre-treatment". The knockdown activity was calculated by dividing the "normalized to pre-treatment" ratio for an individual animal by the average "normalized to pre-treatment" ratio of all mice in the PBS control group. Result is summarized in Table 8 and Table 9. Highly efficacious HBV siRNAs were identified. In particular, mice treated with siRNA agent AD00170, AD00263, AD00267, AD00378, AD00383 and AD00384, showed more than 96% reduction of HBsAg (normalized to pre-treatment). In this example, the GLO-0 in said compounds in Table 3 refers to the compound GalNAc3 in Jayaprakash , et al., (2014) J. Am. Chem. Soc., 136, 16958-16961.

**Table 8. HBV siRNA single 3 mpk subcutaneous dose screening in AAV-HBV transduced mouse. Percent of reduction of HBsAg in mouse serum was normalized to HBsAg level pre-dosing of siRNA and to the PBS control group.**

| Duplex ID | Percent reduction of HBsAg in mouse serum 7, 14 and 21 days after treatment of siRNA | | |
|---|---|---|---|
| | Day 7 | Day 14 | Day 21 |
| AD00170 | 98.55±0.47 | 99.69±0.30 | 99.73±0.22 |
| AD00263 | 99.04±0.33 | 99.54±0.37 | 99.29±0.24 |
| AD00266 | 70.27±14.02 | 80.71±4.17 | 77.49±11.30 |
| AD00267 | 98.65±0.75 | 99.81±0.09 | 99.82±0.07 |
| AD00268 | 93.44±4.32 | 96.94±3.01 | 93.68±2.65 |
| AD00269 | 94.45±3.19 | 97.49±1.56 | 93.99±3.79 |
| AD00173 | 88.6013.02 | 93.87±1.67 | 83.08±5.71 |
| AD00176 | 77.00±5.59 | 87.82±3.79 | 67.18±12.36 |
| AD00378 | 98.97±0.69 | 99.72±0.23 | 99.68±0.11 |
| AD00383 | 98.8610.61 | 99.84±0.05 | 99.76±0.08 |
| AD00384 | 96.06±1.61 | 99.74±0.11 | 99.5110.21 |

**Table 9. HBV siRNA single 3 mpk subcutaneous dose screening in AAV-HBV transduced mouse. Percent of reduction of HBeAg in mouse serum was normalized to HBeAg level pre-dosing of siRNA and to the PBS control group.**

| Duplex ID | Percent reduction of HBeAg in mouse serum 7, 14 and 21 days after treatment of siRNA | | |
|---|---|---|---|
| | Day 7 | Day 14 | Day 21 |
| AD00170 | 74.23±4.07 | 86.47±4.25 | 91.9312.16 |
| AD00263 | 91.31±1.44 | 90.83±1.37 | 88.46±2.17 |
| AD00266 | 25.13±16.91 | 42.77±13.33 | 35.92±17.54 |
| AD00267 | 79.30±4.46 | 90.58±1.67 | 93.04±2.38 |
| AD00268 | 89.25±6.66 | 91.13±1.46 | 88.05±0.93 |
| AD00269 | 86.86±4.17 | 83.80±4.01 | 80.65±3.31 |
| AD00173 | 77.93±3.51 | 80.73±4.02 | 70.20±4.24 |
| AD00176 | 72.77±6.78 | 73.11±6.83 | 69.58±8.28 |
| AD00378 | 84.6312.26 | 93.37±1.47 | 95.82±1.28 |
| AD00383 | 85.49±1.62 | 93.78±1.22 | 96.29±1.11 |
| AD00384 | 77.80±5.93 | 89.81±3.52 | 95.47±1.61 |

### Example 7. In vivo testing of siRNA agents in AAV-HBV mouse model

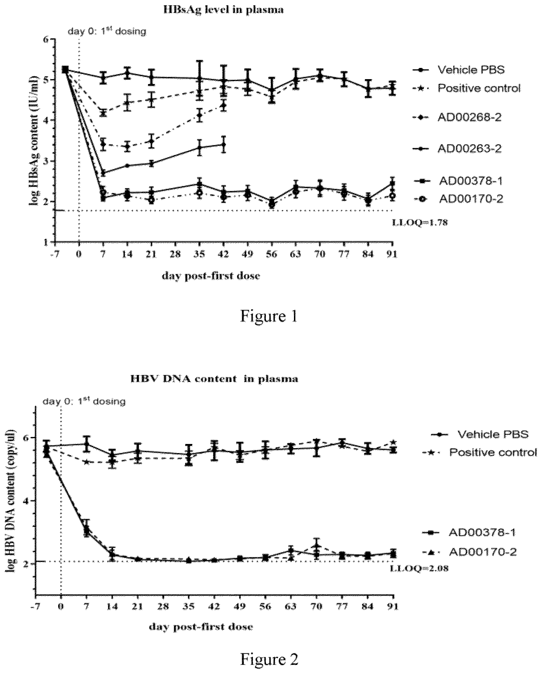

In vivo efficacy of HBV siRNA agents was evaluated using the AAV-HBV mouse model. Male C57BL/6 mice, 5 weeks old, were infected with 1×10^11 rAAV8-1.3 HBV viral genomes via tail vein injection. 14 days after infection, blood samples were taken and HBsAg in mouse serum was measured by ELISA assay. Mice with high expression level of HBsAg (log HBsAg IU/ml > 4.9) were selected for the study. At day 18 post infection, each mouse was administrated subcutaneously with a single 3 mg/kg dose of HBV siRNA or PBS control. Blood samples were drawn at day 7, day 14, day 21, day 28, day 35, day 42, day 49, day 56, day 63, day 70, day 77, day 84 and day 91 post dosing. HBsAg level was measured by ELISA assay and HBV DNA level was determined by qPCR. Results are summarized in Figure 1 and Figure 2. In this example, the positive control is AD-81890 in WO2020036862.

### Equivalents

Although several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto; the invention may be practiced otherwise than as specifically described and claimed. The present invention is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

All references, patents and patent applications and publications that are cited or referred to in this application are incorporated herein in their entirety herein by reference.

## Claims

1. A double-stranded ribonucleic acid (dsRNA) agent for inhibiting expression of Hepatitis B virus (HBV) in a cell, wherein the dsRNA agent comprises a sense strand and an antisense strand, nucleotide positions 2 to 18 in the antisense strand comprising a region of complementarity to an HBV RNA transcript, wherein the region of complementarity comprises at least 15 contiguous nucleotides that differ by 0, 1, 2, or 3 nucleotides from one of the antisense sequences listed in one of Tables 1-4, and optionally comprising a targeting ligand.

2. The dsRNA agent of claim 1, wherein the region of complementarity to the HBV RNA transcript comprises at least 15, 16, 17, 18, or 19 contiguous nucleotides that differ by no more than 3 nucleotides from one of the antisense sequences listed in one of Tables 1-4.

3. The dsRNA agent of claim 1 or 2, wherein the antisense strand of dsRNA is at least substantially complementary to any one of a target region of SEQ ID NO: 673 and is provided in any one of Tables 1-4.

4. The dsRNA agent of claim 3, wherein the antisense strand of dsRNA is fully complementary to any one of a target region of SEQ ID NO: 673 and is provided in any one of Tables 1-4.

5. The dsRNA agent of claim 1, wherein the dsRNA agent comprises a sense strand sequence set forth in any one of Tables 1-4, wherein the sense strand sequence is at least substantially complementary to the antisense strand sequence in the dsRNA agent.

6. The dsRNA agent of claim 1, wherein the dsRNA agent comprises a sense strand sequence set forth in any one of Tables 1-4, wherein the sense strand sequence is fully complementary to the antisense strand sequence in the dsRNA agent.

7. The dsRNA agent of claim 1, wherein the dsRNA agent comprises an antisense strand sequence set forth in any one of Tables 1-4.

8. The dsRNA agent of claim 1, wherein the dsRNA agent comprises the sequences set forth as a duplex sequence in any of Tables 1-4.

9. The dsRNA of claim 1, wherein the dsRNA agent comprises at least one modified nucleotide.

10. The dsRNA agent of claim 1, wherein all or substantially all of the nucleotides of the antisense strand are modified nucleotides.

11. The dsRNA agent of claim 9 or 10, wherein the at least one modified nucleotide comprises: a 2'-O-methyl nucleotide,2'-Fluoro nucleotide, 2'-deoxy nucleotide, 2'3'-seco nucleotide mimic, locked nucleotide, unlocked nucleic acid nucleotide (UNA), glycol nucleic acid nucleotide (GNA), 2'-F-Arabino nucleotide, 2'-methoyxyethyl nucleotide, abasic nucleotide, ribitol, inverted nucleotide, inverted abasic nucleotide, inverted 2'-OMe nucleotide, inverted 2'-deoxy nucleotide, 2'-amino-modified nucleotide, 2'-alkyl-modified nucleotide, mopholino nucleotide, and 3'-OMe nucleotide, a nucleotide comprising a 5'-phosphorothioate group, or a terminal nucleotide linked to a cholesteryl derivative or dodecanoic acid bisdecylamide group, a 2'-amino-modified nucleotide, a phosphoramidate, or a non-natural base comprising nucleotide.

12. The dsRNA agent of claim 9 or 10, wherein the dsRNA agent comprises an E-vinylphosphonate nucleotide at the 5' end of the antisense strand.

13. The dsRNA agent of claim 1, wherein the dsRNA agent comprises at least one phosphorothioate internucleoside linkage.

14. The dsRNA agent of claim 1, wherein the sense strand comprises at least one phosphorothioate internucleoside linkage.

15. The dsRNA agent of claim 1, wherein the antisense strand comprises at least one phosphorothioate internucleoside linkage.

16. The dsRNA agent of claim 1, wherein the sense strand comprises 1, 2, 3, 4, 5, or 6, phosphorothioate internucleoside linkages.

17. The dsRNA agent of claim 1, wherein the antisense strand comprises 1, 2, 3, 4, 5, or 6, phosphorothioate internucleoside linkages.

18. The dsRNA agent of claim 1, wherein all or substantially all of the nucleotides of the sense strand and the antisense strand are modified nucleotides.

19. The dsRNA agent of claim 1, wherein the modified sense strand is a modified sense strand sequence set forth in one of Tables 2-4.

20. The dsRNA agent of claim 1, wherein the modified antisense strand is a modified antisense strand sequence set forth in one of Tables 2-4.

21. The dsRNA agent of claim 1, wherein the sense strand is complementary or substantially complementary to the antisense strand, and the region of complementarity is between 16 and 23 nucleotides in length.

22. The dsRNA agent of claim 1, wherein the region of complementarity is 19-21 nucleotides in length.

23. The dsRNA agent of claim 1, wherein each strand is no more than 30 nucleotides in length.

24. The dsRNA agent of claim 1, wherein each strand is no more than 25 nucleotides in length.

25. The dsRNA agent of claim 1, wherein each strand is no more than 23 nucleotides in length.

26. The dsRNA agent of claim 1, wherein the dsRNA agent comprises at least one modified nucleotide and further comprises one or more targeting groups or linking groups.

27. The dsRNA agent of claim 26, wherein the one or more targeting groups or linking groups are conjugated to the sense strand.

28. The dsRNA agent of claim 26 or 27, wherein the targeting group or linking group comprises N-acetyl-galactosamine (GalNAc).

29. The dsRNA agent of claim 26 or 27, wherein the targeting group has a structure: 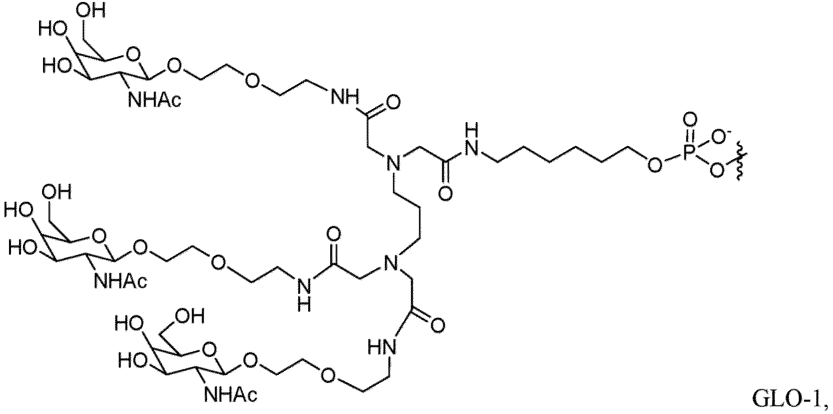 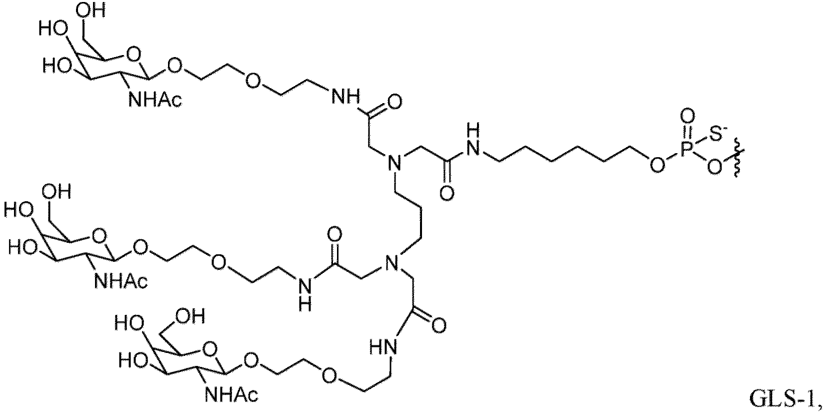 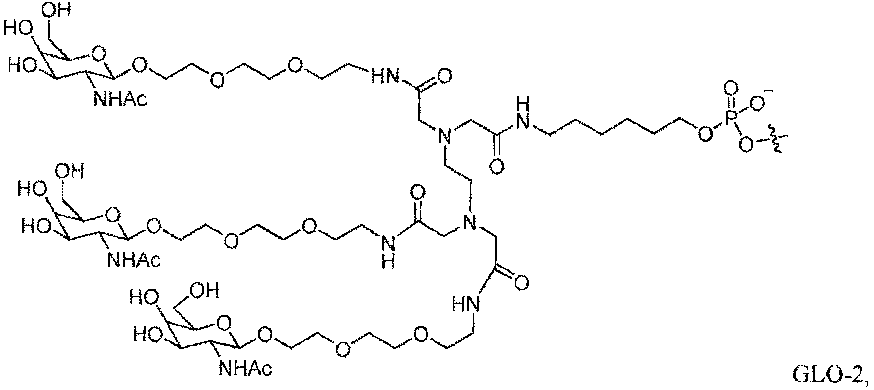 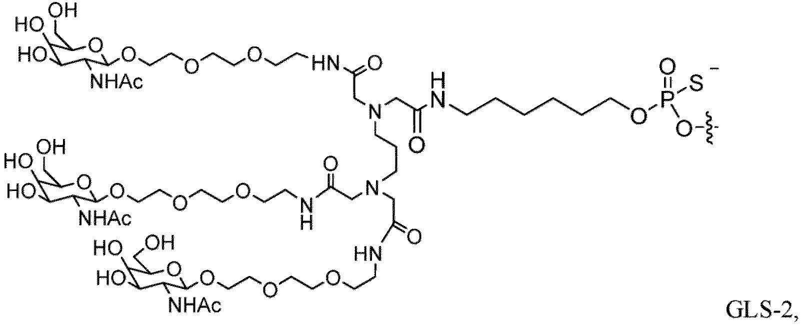 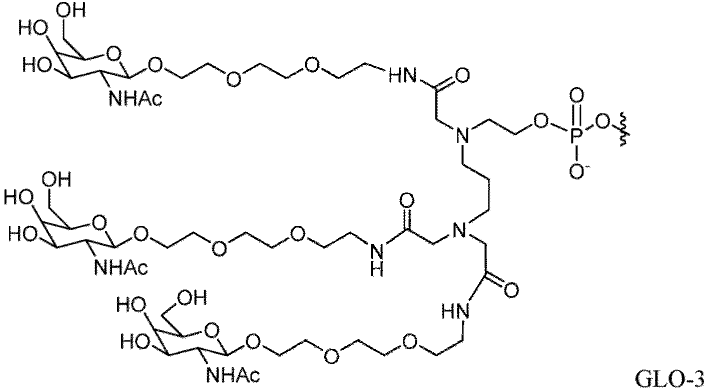 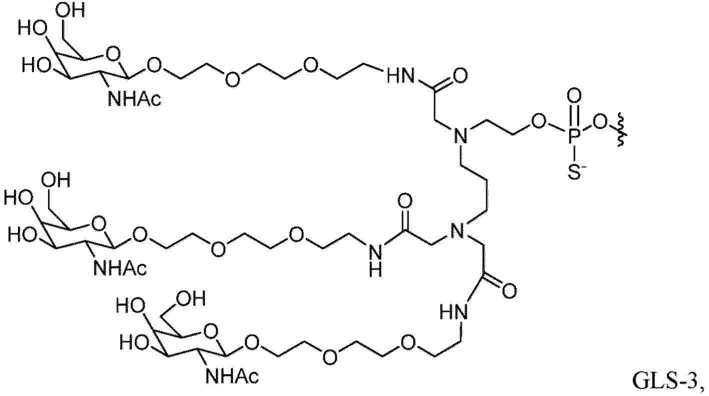 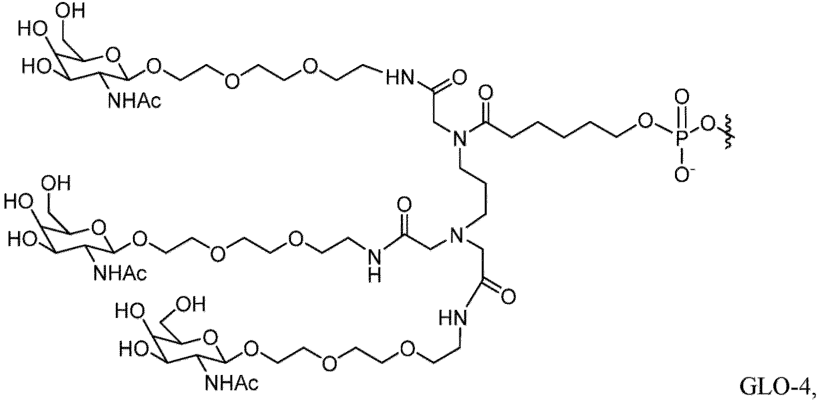 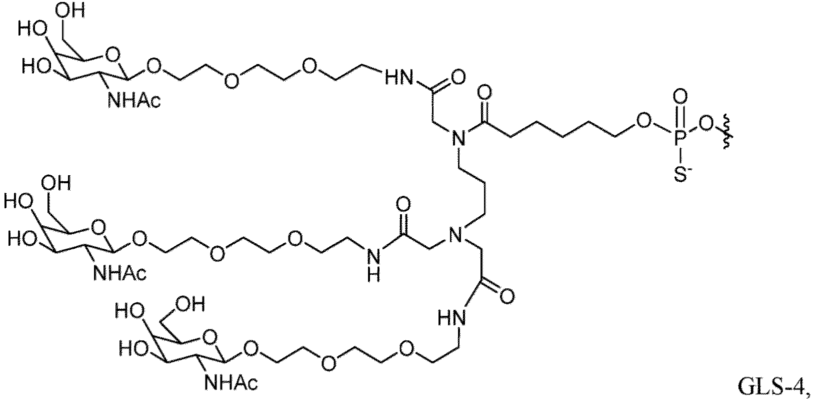 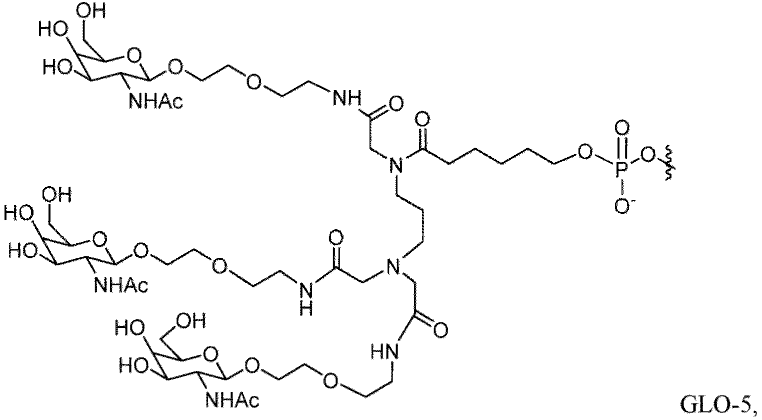 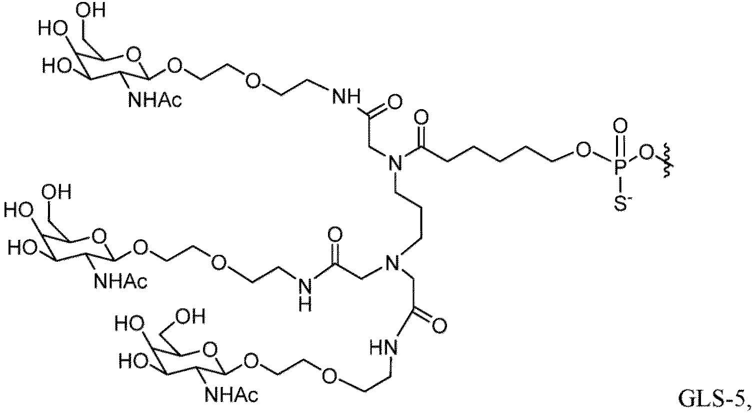 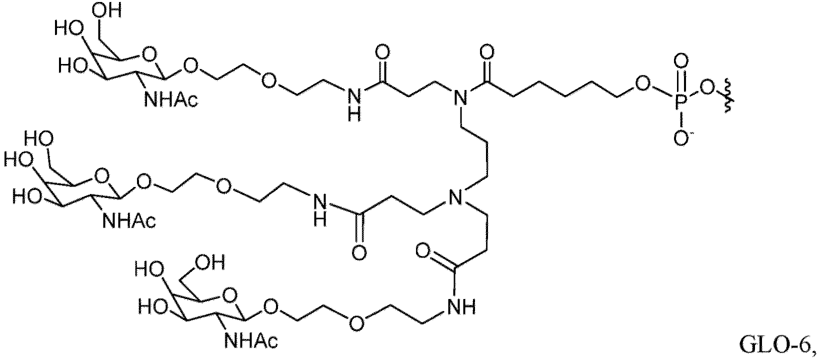 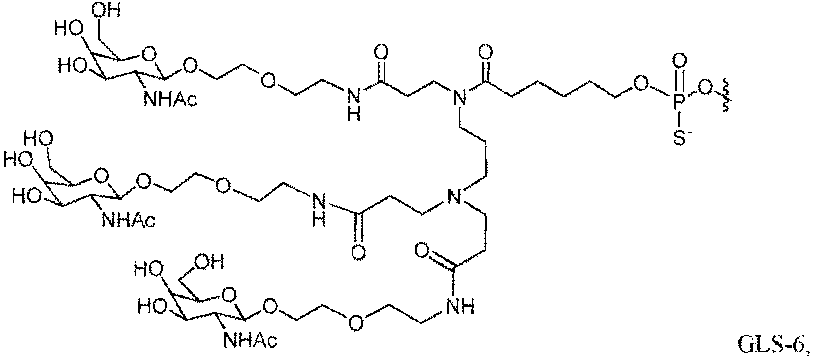 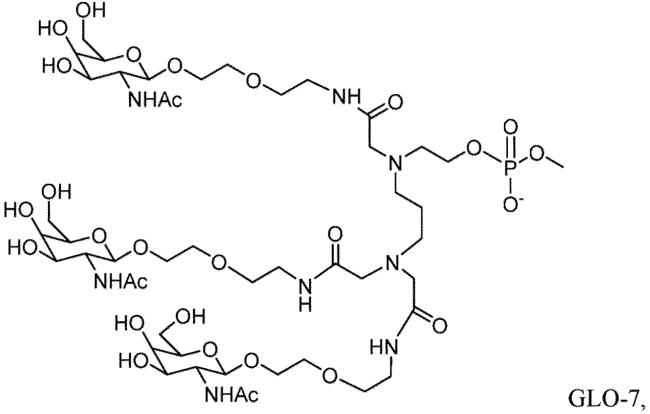 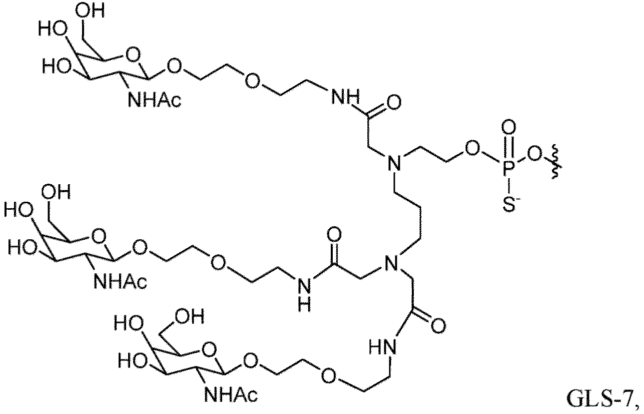 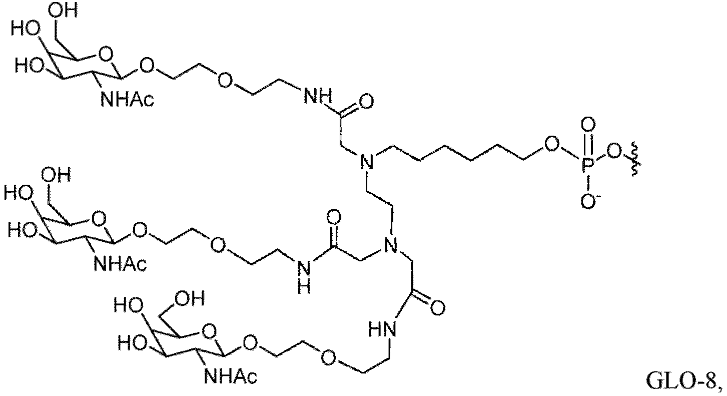 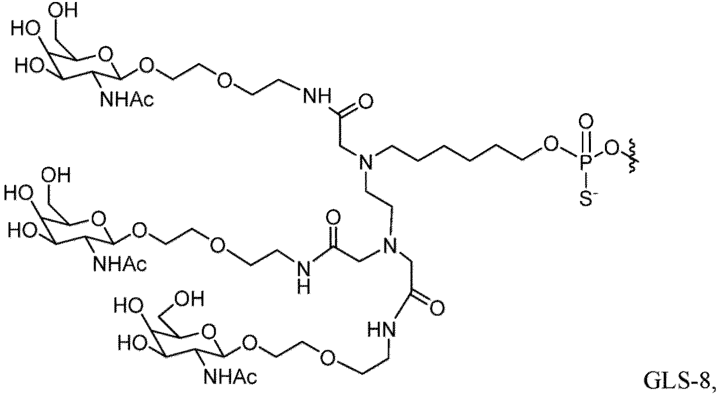 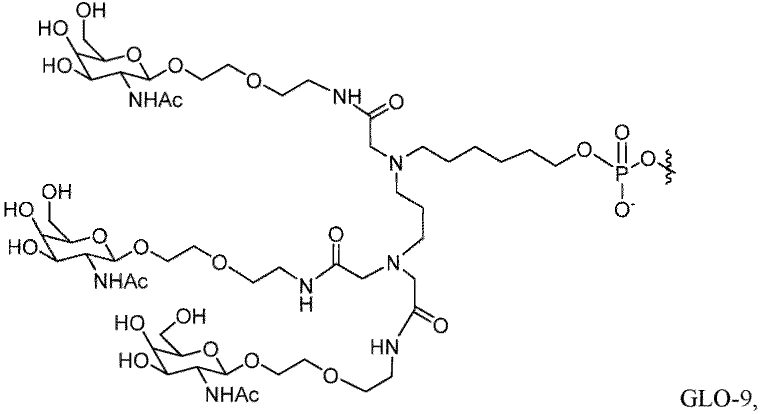 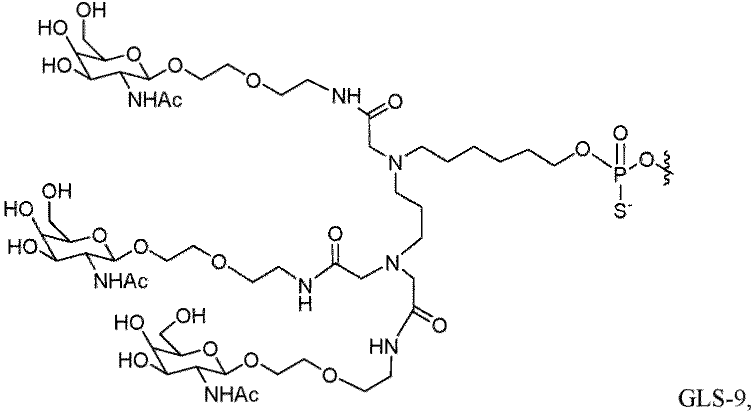 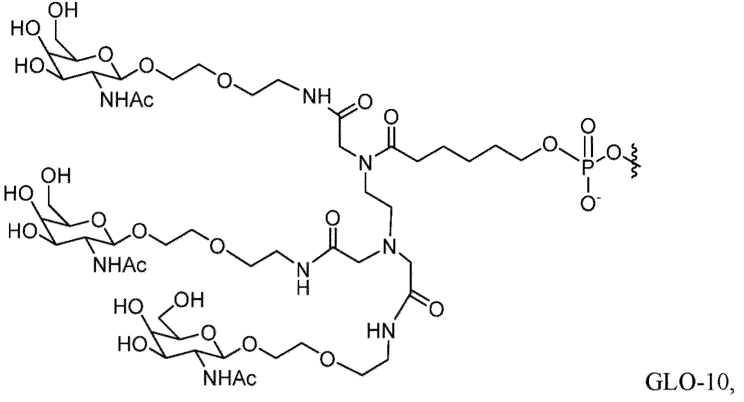 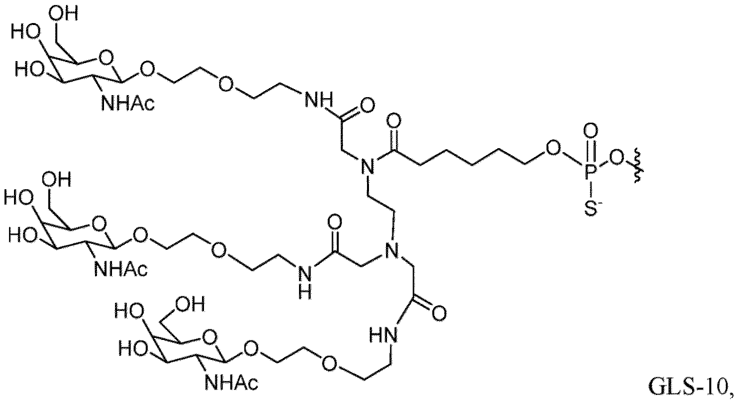 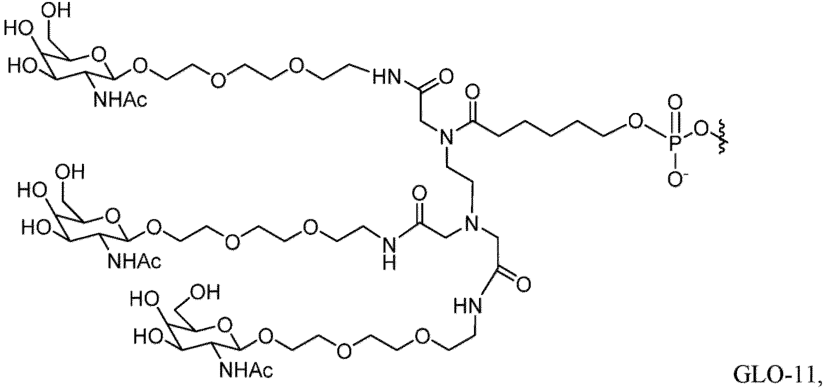 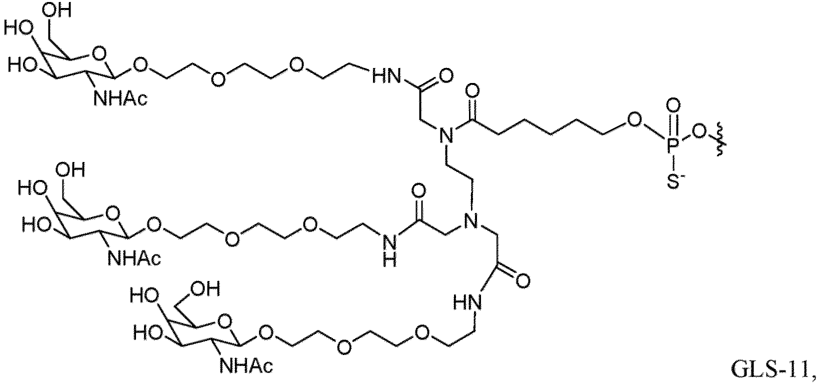 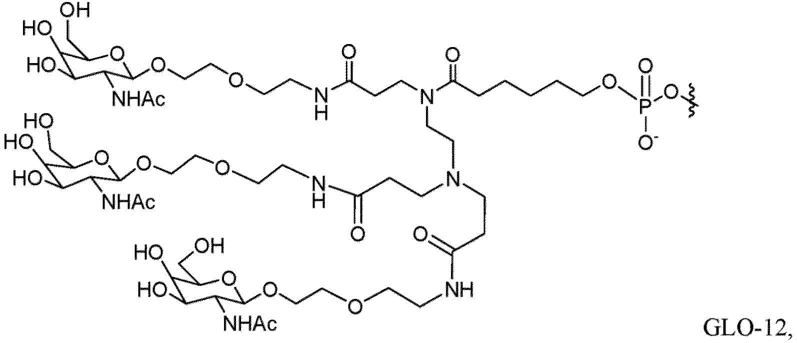 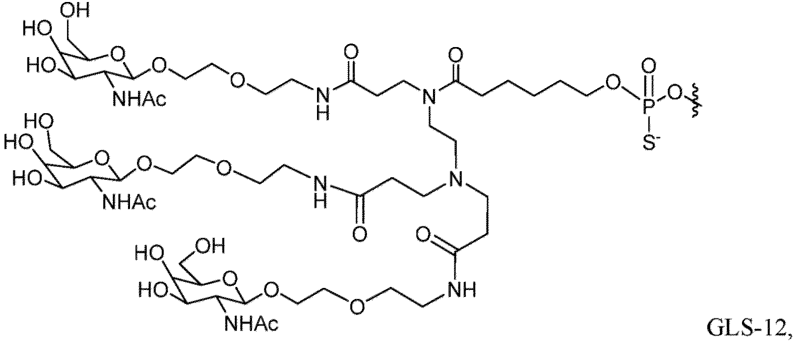 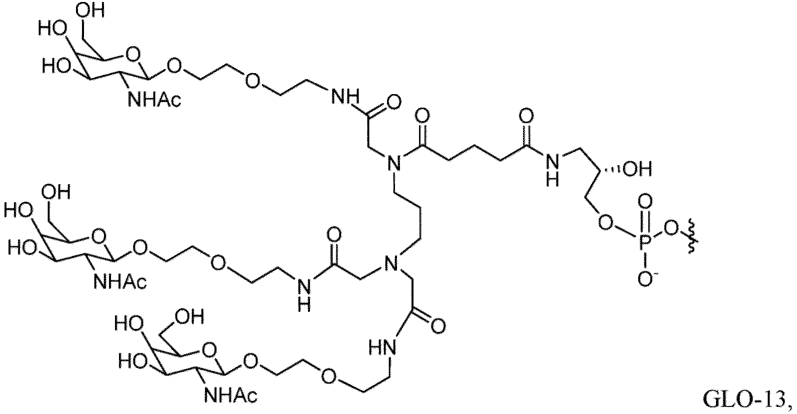 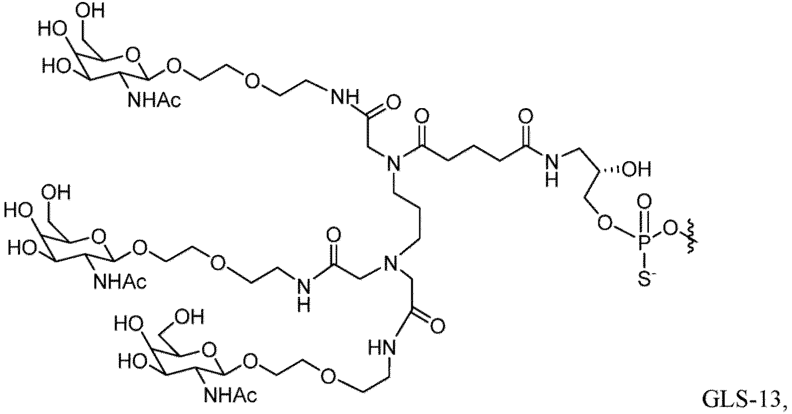 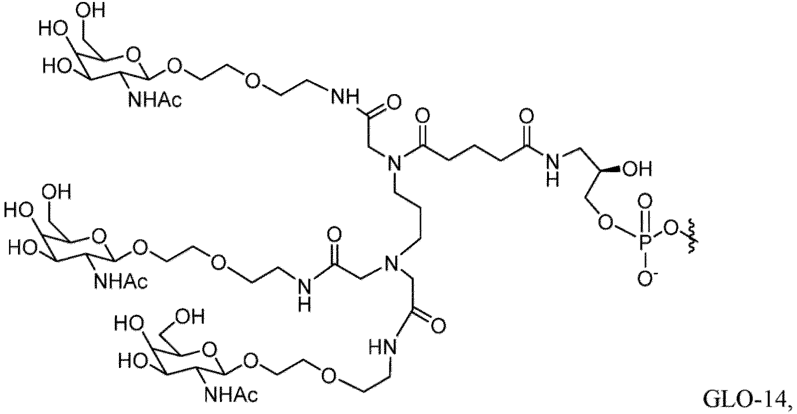 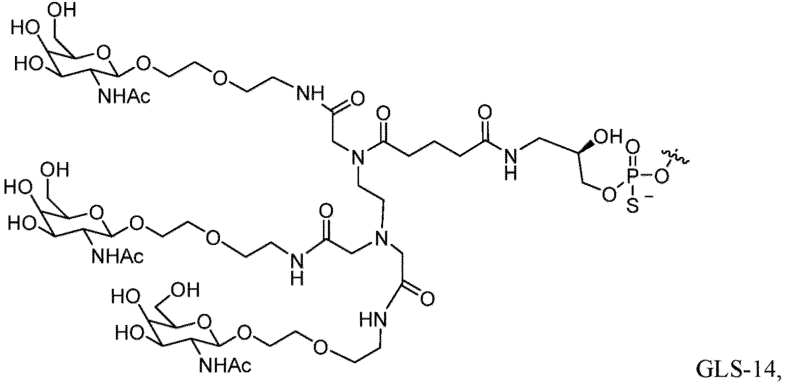 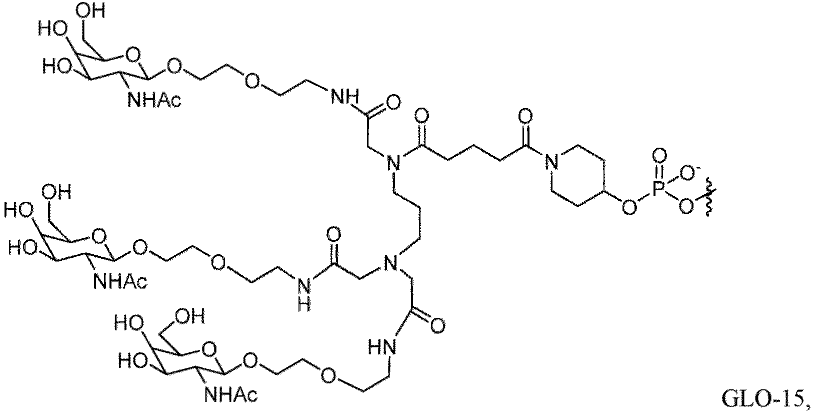 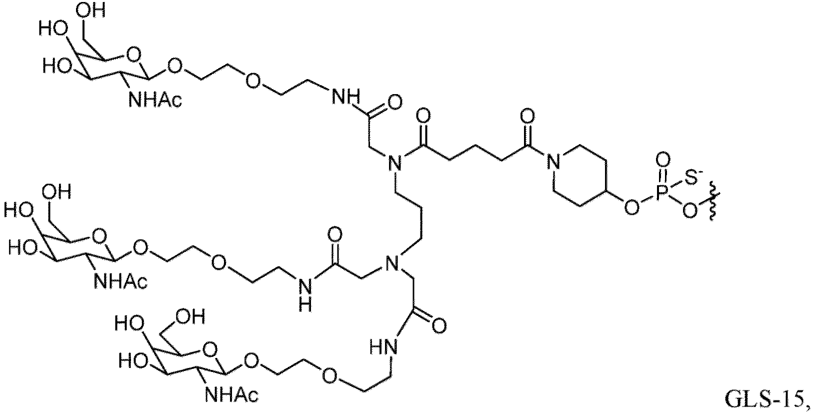 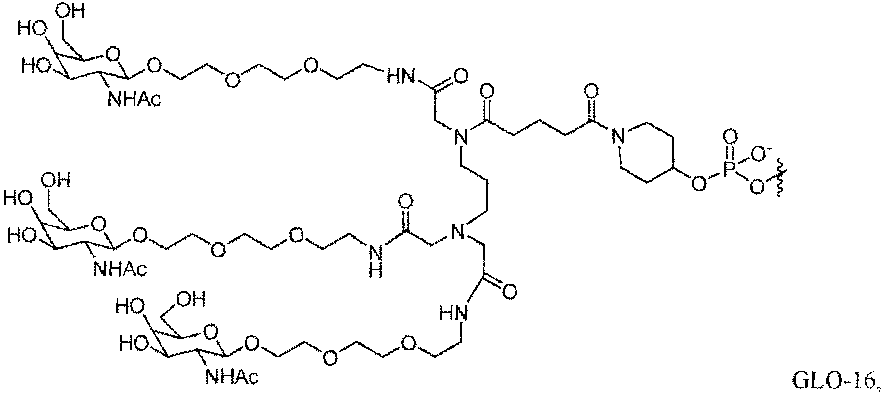 or

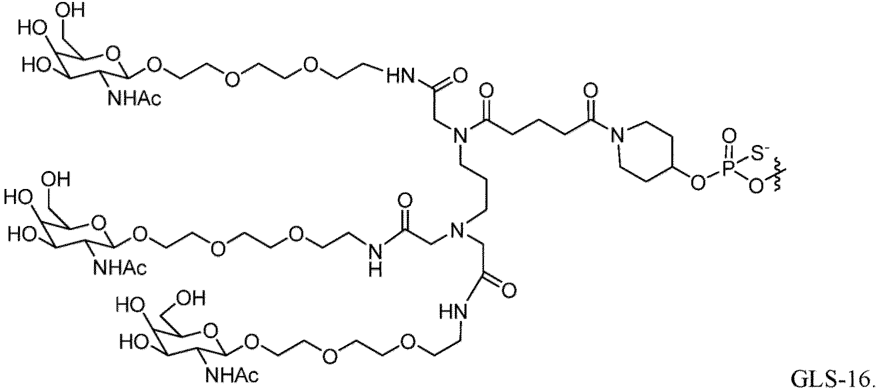

30. The dsRNA agent of claim 1, wherein the dsRNA agent comprises a targeting group that is conjugated to the 5'-terminal end of the sense strand.

31. The dsRNA agent of claim 1, wherein the dsRNA agent comprises a targeting group that is conjugated to the 3'-terminal end of the sense strand.

32. The dsRNA agent of claim 1, wherein the antisense strand comprises one inverted abasic residue at 3'-terminal end.

33. The dsRNA agent of claim 1, wherein the sense strand comprises one or two inverted abasic residues at the 3' and/or the 5' terminal end.

34. The dsRNA agent of claim 1, wherein the dsRNA agent has two blunt ends.

35. The dsRNA agent of claim 1, wherein at least one strand comprises a 3' overhang of at least 1 nucleotide.

36. The dsRNA agent of claim 1, wherein at least one strand comprises a 3' overhang of at least 2 nucleotides.

37. The dsRNA agent of claim 1, wherein the sense strand comprises one of: SEQ ID NO: 281, 290, 295, 300, 304, 306, 307, 331, 557, 567, 569, 571, 572, 573, 560, 563, 607, 628-637 or 918-921, preferably, SEQ ID NO: 557, 607, 631, 918, 919 or 921.

38. The dsRNA agent of claim 1, wherein the antisense strand comprises one of: SEQ ID NO: 419, 428, 433, 438, 442, 444, 445, 469, 582, 592, 594, 596, 597, 598, 585, 588, 642, 663-672 or 922-925, preferably, SEQ ID NO: 582, 642, 666, 922, 923 or 925.

39. The dsRNA agent of claim 1, wherein the dsRNA agent comprises SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; or SEQ ID NO: 637 and SEQ ID NO: 672, SEQ ID NO: 918 and SEQ ID NO: 922, SEQ ID NO: 919 and SEQ ID NO: 923, SEQ ID NO: 920 and SEQ ID NO: 924, or SEQ ID NO: 921 and SEQ ID NO: 925; preferably, SEQ ID NO: 557 and SEQ ID NO: 582; SEQ ID NO: 607 and SEQ ID NO: 642, SEQ ID NO: 631 and SEQ ID NO: 666, SEQ ID NO: 918 and SEQ ID NO: 922, SEQ ID NO: 919 and SEQ ID NO: 923, or SEQ ID NO: 921 and SEQ ID NO: 925.

40. A composition comprising one, two, three, or more dsRNA agents of any one of claims 1-39.

41. The composition of claim 40, further comprising a pharmaceutically acceptable carrier.

42. The composition of claim 41, further comprising one or more additional therapeutic agents.

43. The composition of claim 42, wherein the composition is packaged in a kit, container, pack, dispenser, pre-filled syringe, or vial.

44. The composition of claim 40, wherein the composition is formulated for subcutaneous administration or is formulated for intravenous (IV) administration.

45. The composition of claim 40, wherein the sense strand of at least one of the dsRNA agents comprises one of: SEQ ID NO: 281, 290, 295, 300, 304, 306, 307, 331, 557, 567, 569, 571, 572, 573, 560, 563, 607, 628-637 or 918-921, preferably, SEQ ID NO: 557, 607, 631, 918, 919 or 921.

46. The composition of claim 40, wherein the antisense strand of at least one of the dsRNA agents comprises one of: SEQ ID NO: 419, 428, 433, 438, 442, 444, 445, 469, 582, 592, 594, 596, 597, 598, 585, 588, 642, 663-672, or 922-925, preferably, SEQ ID NO: 582, 642, 666, 922, 923 or 925.

47. The composition of claim 40, wherein at least one of the dsRNA agents comprises: SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; SEQ ID NO: 637 and SEQ ID NO: 672, SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925; preferably, SEQ ID NO: 557 and SEQ ID NO: 582, SEQ ID NO: 607 and SEQ ID NO: 642, SEQ ID NO: 631 and SEQ ID NO: 666, SEQ ID NO: 918 and SEQ ID NO: 922, SEQ ID NO: 919 and SEQ ID NO: 923, or SEQ ID NO: 921 and SEQ ID NO: 925.

48. A cell comprising a dsRNA agent of any one of claims 1-39.

49. The cell of claim 48, wherein the cell is a mammalian cell, optionally a human cell.

50. A method of inhibiting the expression of a hepatitis B virus (HBV) gene in a cell, the method comprising:
(i) preparing a cell comprising an effective amount of one or more double-stranded ribonucleic acid (dsRNA) agents of any one of claims 1-39 or a composition of any one of claims 40-47.

51. The method of claim 50, further comprising:
(ii) maintaining the cell prepared in claim 50(i) for a time sufficient to obtain degradation of the mRNA transcript of an HBV gene, thereby inhibiting expression of the HBV gene in the cell; inhibiting replication of the HBV in the cell; and reducing a level of the HBV antigens in the cell.

52. The method of claim 51, wherein the one or more HBV antigens are HBcAg, HBsAg, and HBeAg.

53. The method claim 50 or 51, wherein the cell is in a subject and the dsRNA agent is administered to the subject subcutaneously.

54. The method of claim 50 or 51, wherein the cell is in a subject and the dsRNA agent is administered to the subject by IV administration.

55. The method of any one of claims 50-54, wherein 2, 3, 4, or more dsRNA agents are administered to the subject.

56. The method of any one of claims 50-55, further comprising assessing inhibition of the HBV gene, following the administration of the dsRNA agent(s) to the subject, wherein a means for the assessing comprises:
(i) determining one or more physiological characteristics of an HBV-associated disease or condition in the subject and
(ii) comparing the determined physiological characteristic(s) to a baseline pre-treatment physiological characteristic of the HBV-associated disease or condition and/or to a control physiological characteristic of the HBV-associated disease or condition, wherein the comparison indicates one or more of a presence or absence of inhibition of expression of the HBV gene in the subject.

57. The method of claim 56, wherein the control physiological characteristic is the physiological characteristic in a subject infected with HBV and not administered the dsRNA agent(s).

58. The method of claim 57, wherein the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subj ect.

59. The method of claim 56 wherein the physiological characteristic is determined in a biological sample obtained from the subject.

60. The method of claim 59, wherein the biological sample comprises one or more of: a serum sample, a tissue sample, a cell sample, and a liver sample.

61. The method of claim 56, wherein the determined physiological characteristic in the subject is abnormal compared to a control level of the physiological characteristic.

62. The method of claim 61, wherein a level of one or more of ALT; AST; HBV viral load, HBV covalently closed circular DNA (cccDNA); one or more HBV antigens; one or more of: HBcAg, HBsAg and HBeAg; and one or more anti-Hepatitis B virus antibodies in the subject determined to be statistically significantly higher compared to a control physiological characteristic of the HBV-associated disease or condition and/or the baseline pre-treatment physiological characteristic of the HBV-associated disease or condition indicates a status of HBV gene expression in the subject.

63. The method of claim 62, wherein the control physiological characteristic is the physiological characteristic in a subject infected with HBV and not administered the dsRNA agent(s).

64. The method of claim 56, wherein a reduction in one or more of the ALT; AST; HBV viral load, HBV covalently closed circular DNA (cccDNA); one or more HBV antigens; HBcAg; HBsAg; and HBeAg in the subject indicates a reduction of HBV gene expression in the subject.

65. The method of claim 56, wherein an increase in anti-Hepatitis B virus antibodies in the subject compared to a control physiological characteristic of the HBV-associated disease or condition and/or the baseline pre-treatment physiological characteristic of the HBV-associated disease or condition, indicates a reduction in HBV gene expression in the subject.

66. The method of claim 56, wherein the HBV-associated disease or condition is one or more of: wherein the disease or condition is one or more of: hepatitis B, chronic hepatitis B, hepatitis D virus infection (delta hepatitis or HDV), liver injury, cirrhosis, acute hepatitis B, acute fulminant hepatitis, and liver fibrosis, liver inflammation, and hepatocellular carcinoma.

67. A method of inhibiting expression of an HBV gene in a subject, the method comprising administering to the subject an effective amount of one or more double-stranded ribonucleic acid (dsRNA) agents of any one of claims 1-39 or a composition of any one of claims 40-47.

68. The method of claim 67, wherein two, three, four, or more of the dsRNA agents are administered to the subject.

69. The method of claim 67 or 68, wherein the dsRNA agent is administered to the subject subcutaneously.

70. The method of claim 67 or 68, wherein the dsRNA agent is administered to the subject by IV administration.

71. The method of any one of claims 67-70, further comprising assessing inhibition of the HBV gene, following the administration of the one or more dsRNA agents, wherein a means for the assessing comprises:
(i) determining one or more physiological characteristics of an HBV-associated disease or condition in the subject and
(ii) comparing the determined physiological characteristic(s) to a baseline pre-treatment physiological characteristic of the HBV-associated disease or condition and/or to a control physiological characteristic of the HBV-associated disease or condition,
wherein the comparison indicates one or more of a presence or absence of inhibition of expression of the HBV gene in the subject.

72. The method of claim 71, wherein the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subj ect.

73. The method of claim 71 wherein the physiological characteristic is determined in a biological sample obtained from the subject.

74. The method of claim 73, wherein the biological sample comprises one or more of: a serum sample, a cell sample, a tissue sample, and a liver sample.

75. The method of claim 71, wherein the determined physiological characteristic in the subject is more abnormal compared to a baseline pre-treatment physiological characteristic of the HBV-associated disease or condition and/or to a control physiological characteristic of the HBV-associated disease or condition.

76. The method of claim 73, wherein a level of one or more of ALT; AST; HBV viral load, HBV covalently closed circular DNA (cccDNA); one or more HBV antigens; one or more of: HBcAg, HBsAg and HBeAg; and one or more anti-Hepatitis B virus antibodies in the subject determined to be statistically significantly higher, lower, or unchanged compared to a baseline pre-treatment physiological characteristic of the HBV-associated disease or condition and/or to a control physiological characteristic of the HBV-associated disease or condition indicates a status of HBV gene expression in the subject.

77. The method of claim 71, wherein a reduction in one or more of the ALT; AST; HBV viral load, HBV covalently closed circular DNA (cccDNA); one or more HBV antigens; HBcAg; HBsAg; and HBeAg in the subject indicates a reduction of HBV gene expression in the subject.

78. The method of claim 76, wherein an increase in anti-Hepatitis B virus antibodies in the subject compared to a baseline pre-treatment physiological characteristic of the HBV-associated disease or condition and/or to a control physiological characteristic of the HBV-associated disease or condition, indicates a reduction in HBV gene expression in the subject.

79. The method of claim 71, wherein the HBV-associated disease or condition is one or more of: hepatitis B, chronic hepatitis B, acute hepatitis B, hepatocellular carcinoma, liver injury, cirrhosis, acute fulminant hepatitis, end-stage liver disease, liver fibrosis, liver inflammation, and a hepatitis D virus infection (delta hepatitis or HDV).

80. A method of treating a disease or condition associated with the presence of a hepatitis B virus (HBV) protein, the method comprising administering to a subject an effective amount of one or more double-stranded ribonucleic acid (dsRNA) agents of any one of claims 1-39, or a composition of any one of claims 40-47, to inhibit expression of the HBV gene encoding the HBV protein.

81. The method of claim 80, wherein the method comprises administering two, three, four, or more dsRNA agents to the subject.

82. The method of claim 80 or 81, wherein the disease or condition is one or more of: hepatitis B, chronic hepatitis B, hepatitis D virus infection (delta hepatitis or HDV), liver injury, cirrhosis, acute hepatitis B, acute fulminant hepatitis, liver inflammation, liver fibrosis, and hepatocellular carcinoma.

83. The method of claim 80 or 81, further comprising administering an additional therapeutic regimen to the subject.

84. The method of claim 81, wherein the additional therapeutic regimen comprises: administering to the subject one or more: HBV antisense polynucleotides, additional HBV dsRNA therapeutic agent, a non-HBV dsRNA therapeutic agent, a HBV non-dsRNA therapeutic agent, and a behavioral modification.

85. The method of claim 84, wherein the non-HBV dsRNA therapeutic agent is one of more of: an antiviral agent, an antiviral nucleoside or nucleotide analogue (NUC), a viral polymerase inhibitor, a reverse transcriptase inhibitor, an immune stimulator, a therapeutic vaccine, a viral entry inhibitor, an oligonucleotide that inhibits the secretion or release of HBsAg, a capsid inhibitor, a covalently closed circular (ccc) HBV DNA inhibitor.

86. The method of claim 85, wherein the NUC is: Tenofovir disoproxil fumarate (TDF), Tenofovir alafenamide, Lamivudine, Adefovir dipivoxil, Entecavir (ETV), or Telbivudine.

87. The method of claim 85, wherein the immune stimulator is pegylated interferon alfa 2a (PEG-IFN-a2a), Interferon alfa-2b, a recombinant human interleukin-7, a Toll-like receptor 7 (TLR7) agonist, or a checkpoint inhibitor (e.g., PD1 inhibitor).

88. The method of claim 80, wherein the one or more dsRNA agent(s) is administered to the subject subcutaneously.

89. The method of claim 80, wherein the one or more dsRNA agent(s) is administered to the subject by IV administration.

90. The method of any one of claims 80-89, further comprising determining an efficacy of the administered one or more double-stranded ribonucleic acid (dsRNA) agent(s) in the subj ect.

91. The method of claim 90, wherein a means of determining an efficacy of the treatment in the subject comprises:
(i) determining one or more physiological characteristics of the HBV-associated disease or condition in the subject and
(ii) comparing the determined physiological characteristic(s) to a baseline pre-treatment physiological characteristic of the HBV-associated disease or condition,
wherein the comparison indicates one or more of a presence, absence, and level of efficacy of the administration of the double-stranded ribonucleic acid (dsRNA) agent to the subject.

92. The method of claim 91, wherein the determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subj ect.

93. The method of claim 91 wherein the physiological characteristic is determined in a biological sample obtained from the subject.

94. The method of claim 93, wherein the biological sample comprises one or more of: a serum sample, a cell sample, a tissue sample, and a liver sample.

95. The method of claim 91, wherein the determined physiological characteristic in the subject is different compared to the baseline pre-treatment physiological characteristic of the HBV-associated disease or condition or to a control level of the physiological characteristic.

96. The method of claim 95, wherein a level of one or more of ALT; AST; HBV viral load, HBV covalently closed circular DNA (cccDNA); one or more HBV antigens; one or more of: HBcAg, HBsAg and HBeAgin the subject determined to be statistically significantly lower compared to the baseline pre-treatment physiological characteristic of the HBV-associated disease or condition indicates a status of reduced HBV gene expression in the subject.

97. The method of claim 95, wherein an increase in anti-Hepatitis B virus antibodies in the subject compared to the baseline pre-treatment anti-Hepatitis B virus antibodies or a control level of anti-Hepatitis B virus antibodies indicates a reduction in HBV gene expression in the subj ect.

98. The method of any one of claims 91-97, wherein the HBV-associated disease or condition is one or more of: hepatitis B, chronic hepatitis B, acute hepatitis B, hepatocellular carcinoma, liver injury, cirrhosis, end-stage liver disease, acute fulminant hepatitis, liver inflammation, liver fibrosis, and hepatitis D virus infection (delta hepatitis or HDV).

99. A method of decreasing a level of HBV protein in a subject compared to a baseline pre-treatment level of HBV protein in the subject, the method comprising administering to the subject an effective amount of one or more double-stranded ribonucleic acid (dsRNA) agent(s) of any one of claims 1-39, or a composition of any one of claims 40-47, to decrease the level of HBV gene expression.

100. The method of claim 99, wherein the dsRNA agent is administered to the subject subcutaneously or is administered to the subject by IV administration.

101. A method of altering a physiological characteristic of a hepatitis B virus (HBV)-associated disease or condition in a subject compared to a baseline pre-treatment physiological characteristic of the HBV-associated disease or condition in the subject, the method comprising administering to the subject an effective amount of one or more double-stranded ribonucleic acid (dsRNA) agent of any one of claims 1-39, or a composition of any one of claims 40-47, to alter the physiological characteristic of the HBV-associated disease or condition in the subject.

102. The method of claim 101, wherein the one or more dsRNA agent(s) is administered to the subject subcutaneously or is administered to the subject by IV administration.

103. The method of claim 101, wherein the physiological characteristic is one or more of determined physiological characteristic in the subject is one or more of: a level of alanine aminotransferase (ALT) in the subject, a level of aspartate aminotransferase (AST) in the subject; an HBV viral load in the subject; HBV covalently closed circular DNA (cccDNA) level in the subject; a level of one or more HBV antigens in the subject; a level of one or more of: HBcAg, HBsAg and HBeAg in the subject; presence, absence, and/or a level of one or more anti-Hepatitis B virus antibodies in the subject.

104. The method of any one of claims 50, 67, 80, 99, and 101, wherein the sense strand of the dsRNA agent comprises one of: SEQ ID NO: 281, 290, 295, 300, 304, 306, 307, 331, 557, 567, 569, 571, 572, 573, 560, 563, 607 , 628-637 or 918-921, preferably, SEQ ID NO: 557, 607, 631, 918, 919 or 921.

105. The method of any one of claims 50, 67, 80, 99, and 101, wherein the antisense strand of the dsRNA agent comprises one of: SEQ ID NO: 419, 428, 433, 438, 442, 444, 445, 469, 582, 592, 594, 596, 597, 598, 585, 588, 642 , 663-672 or 922-925, preferably, SEQ ID NO: 582, 642, 666, 922, 923 or 925.

106. The method of any one of claims 50, 67, 80, 99, and 101, wherein the dsRNA agent comprises SEQ ID NO: 281 and SEQ ID NO: 419; SEQ ID NO: 290 and SEQ ID NO: 428; SEQ ID NO: 295 and SEQ ID NO: 433; SEQ ID NO: 300 and SEQ ID NO: 438; SEQ ID NO: 304 and SEQ ID NO: 442; SEQ ID NO: 306 and SEQ ID NO: 444; SEQ ID NO: 307 and SEQ ID NO: 445; SEQ ID NO: 331 and SEQ ID NO: 469; SEQ ID NO: 557 and SEQ ID NO: 582; SEQ ID NO: 567 and SEQ ID NO: 592; SEQ ID NO: 569 and SEQ ID NO: 594; SEQ ID NO: 571 and SEQ ID NO: 596; SEQ ID NO: 572 and SEQ ID NO: 597; SEQ ID NO: 573 and SEQ ID NO: 598; SEQ ID NO: 560 and SEQ ID NO: 585; SEQ ID NO: 563 and SEQ ID NO: 588; SEQ ID NO: 607 and SEQ ID NO: 642; SEQ ID NO: 628 and SEQ ID NO: 663; SEQ ID NO: 629 and SEQ ID NO: 664; SEQ ID NO: 630 and SEQ ID NO: 665; SEQ ID NO: 631 and SEQ ID NO: 666; SEQ ID NO: 632 and SEQ ID NO: 667; SEQ ID NO: 633 and SEQ ID NO: 668; SEQ ID NO: 634 and SEQ ID NO: 669; SEQ ID NO: 635 and SEQ ID NO: 670; SEQ ID NO: 636 and SEQ ID NO: 671; or SEQ ID NO: 637 and SEQ ID NO: 672; SEQ ID NO: 918 and SEQ ID NO: 922; SEQ ID NO: 919 and SEQ ID NO: 923; SEQ ID NO: 920 and SEQ ID NO: 924; or SEQ ID NO: 921 and SEQ ID NO: 925; preferably, SEQ ID NO: 557 and SEQ ID NO: 582; SEQ ID NO: 607 and SEQ ID NO: 642; SEQ ID NO: 631 and SEQ ID NO: 666; SEQ ID NO: 918 and SEQ ID NO: 922, SEQ ID NO: 919 and SEQ ID NO: 923, or SEQ ID NO: 921 and SEQ ID NO: 925.
